# FASCICULE DE BREVET EUROPEEN

(11) **EP 2 225 240 B1**
(45) Date de publication et mention de la délivrance du brevet: **22.01.2014**
(21) Numéro de dépôt: 08872775.5
(22) Date de dépôt: 31.12.2008
(51) Int. Cl.: C07D 471/04, A61K 31/437, C07F 5/04, C07F 7/08, A61P 29/00, A61P 19/10, A61P 35/00, A61P 9/00, C07D 233/70, C07D 213/24, C07D 213/61, C07D 213/50, C07D 213/53, C07D 213/48, C07D 213/30, C07D 213/84, C07D 233/72, C07F 5/02, C07F 7/22

(54) **Dérivés 6-hétérocyclique-imidazo[1,2-a]pyridine-2-carboxamides, leur préparation et leur application en thérapeutique**
6-Heterozyklische Imidazo[1,2-a]pyridin-2-carboxamid-derivate, ihre Herstellung und ihre therapeutische Anwendung
Derivatives of 6-heterocyclic-imidazo[1,2-a]pyridine-2-carboxamides, preparation thereof and therapeutic application thereof

(30) Priorité: 02.01.2008 FR 0800005
(43) Date de publication de la demande: 08.09.2010
(73) Titulaire: SANOFI, 75008 Paris (FR)
(72) Inventeur: PEYRONEL, Jean-François, F-75013 Paris (FR)
(74) Mandataire: Le Coupanec, Pascale A.M.P.
(86) Numéro de dépôt international: PCT/FR2008/001836
(87) Numéro de publication internationale: WO 2009/106750

(56) Documents cités:
- EP-A- 0 436 831
- WO-A-2004/050659
- FR-A- 2 638 161
- FR-A- 2 903 107
- ENGUEHARD-GUEIFFIER, CECILE ET AL: "Convenient synthesis of alkenyl-, alkynyl-, and allenyl-substituted imidazo[1,2-a]pyridines via palladium-catalyzed cross-coupling reactions" HELVETICA CHIMICA ACTA , 90(12), 2349-2367 CODEN: HCACAV; ISSN: 0018-019X, vol. 90, 2007, XP002496753
- DATABASE CA [Online] CHEMICAL ABSTRACTS SERVICE, COLUMBUS, OHIO, US; IVASHCHENKO, ALEXANDER VASILIEVICH ET AL: "A preparation of combinatorial library of 2-substituted piperidine derivatives" XP002496762 extrait de STN Database accession no. 2004:1037092 & WO 2004/103991 A1 (CHEMICAL DIVERSITY RESEARCH INSTITUTE, LTD., RUSSIA) 2 décembre 2004 (2004-12-02)

## Description

La présente invention se rapporte à des dérivés d'imidazo[1,2-α]pyridine-2-carboxamides, à leur préparation et à leur application en thérapeutique dans le traitement ou la prévention de maladies impliquant les récepteurs nucléaires Nurr-1, aussi appelés NR4A2, NOT, TINUR, RNR-1, et HZF3.

La présente invention a pour objet les composés de formule (I) : 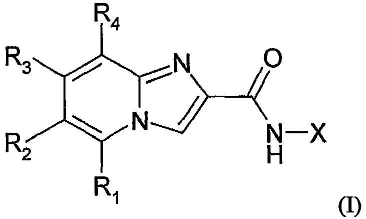 dans laquelle :
X représente un groupe phényle éventuellement substitué par un ou plusieurs groupes choisis indépendamment les uns des autres parmi les atomes ou groupes suivants : halogène, (C₁-C₆)alcoxy, (C₁-C₆)alkyle, NRaRb, cyano, (C₁-C₆)alcoxycarbonyl, les groupes (C₁-C₆)alkyle et (C₁-C₆)alcoxy étant éventuellement substitués par un ou plusieurs atomes d'halogène;
R₁ représente un atome d'hydrogène, un halogène, un groupe (C₁-C₆)alcoxy, un groupe (C₁-C₆)alkyle, un groupe NRaRb, les groupes alkyles et alcoxy pouvant éventuellement être substitués par un ou plusieurs halogène, hydroxy, amino, ou groupe (C₁-C₆)alcoxy ;
R₂ représente un groupe hétérocyclique aromatique, insaturé, partiellement saturé ou totalement saturé, éventuellement substitué par un ou plusieurs groupes choisis indépendamment les uns des autres parmi les atomes ou groupes suivants : hydroxy, (C₁-C₆)alcoxy, halogène, cyano, NRaRb, -CO-R₅, -CO-NR₆R₇, -CO-O-R₈, NR₉-CO-R₁₀, un groupe (C₁-C₆)alkyle, lui-même éventuellement substitué par un ou plusieurs hydroxy ou NRaRb, un groupe oxido ;
R₃ représente un atome d'hydrogène, un groupe (C₁-C₆)alkyle, un groupe (C₁-C₆)alcoxy ou un atome d'halogène ;
R₄ représente un atome d'hydrogène, un groupe (C₁-C₄)alkyle, un groupe (C₁-C₄)alcoxy ou un atome de fluor ;
R₅ représente un atome d'hydrogène, un groupe phényle ou un groupe (C₁-C₆)alkyle ;
R₆ et R₇, identiques ou différents, représentent un atome d'hydrogène ou un groupe (C₁-C₆)alkyle ou forment avec l'atome d'azote un cycle de 4 à 7 chaînons incluant éventuellement un autre hétéroatome choisi parmi N, O ou S ;
R₈ représente un groupe (C₁-C₆)alkyle ;
R₉ et R₁₀, identiques ou différents, représentent un atome d'hydrogène ou un groupe (C₁-C₆)alkyle ;
Ra et Rb sont indépendamment l'un de l'autre, hydrogène, (C₁-C₆)alkyle ou forment avec l'atome d'azote qui les porte un cycle de 4 à 7 chaînons, incluant éventuellement un autre hétéroatome choisi parmi N, O ou S ;
à l'exception du *N*-(4-bromophényl)-6-(1-méthyl-2-pipéridinyl)imidazo[1,2-a]pyridine-2-carboxamide ;
à l'état de base ou de sel d'addition à un acide.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purifcation ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Le *N*-(4-bromophényl)-6-(1-méthyl-2-pipéridinyl)imidazo[1,2-a]pyridine-2-carboxamide, spécifiquement exclu de la formule (I) selon l'invention, est cité dans les librairies chimiques sous le numéro RN = 797785-86-5.

On connaît du document FR 2 638 161 des composés dérivés de benzoyl-2-imidazo[1,2-a]pyridine, utiles en tant que médicament. On connaît également du document WO 2004/050659 des composés dérivés d'imidazo[1,2-a]pyridinyles utiles dans le traitement des cancers. EP 0 436 831 décrit en outre des composés imidazo[1,2-a]pyridinylalkyl utiles dans le traitement des maladies neurodégénératives.

Dans le cadre de la présente invention, on entend par :
- un atome d'halogène : un fluor, un chlore, un brome ou un iode ;
- un groupe alkyle : un groupe aliphatique saturé linéaire, ramifé ou cyclique, éventuellement substitué par un groupe alkyle saturé linéaire, ramifié ou cyclique. A titre d'exemples, on peut citer les groupes méthyle, éthyle, propyle, isopropyle, butyle, isobutyle, tertbutyle, cyclopropyle, cyclobutyle, cyclopentyle, cyclohexyle, méthylcyclopropyle ;

- un groupe alcényle : un groupe aliphatique mono- ou poly-insaturé, linéaire ou ramifié, comprenant par exemple une ou deux insaturations éthyléniques ;
- un groupe alcoxy un radical -O-alkyle où le groupe alkyle est tel que précédemment défini ;
- un groupe hétérocyclique: un groupe monocyclique aromatique, insaturé, partiellement saturé ou totalement saturé comportant de 5 à 10 atomes dont 1 à 4 hétéroatomes choisis parmi N, O et S. A titre d'exemples de groupes hétérocycliques, on peut citer : pyrrole, furane, thiophène, pyrazole, imidazole, triazole, tetrazole, oxazole, isoxazole, oxadiazole, thiazole, isothiazole, thiadiazole, pyridine, pyrimidine, pyrazine, pyridazine, triazine ; ces groupes pouvant être partiellement insaturés ou saturés.

A titre d'exemples de groupes hétérocycliques, on peut également citer les groupes dioxolane, indazoline.

Ce groupe hétérocyclique en R₂ n'est pas relié à l'imidazo[1,2-α]pyridine par un azote si c'est un groupe hétérocyclique monocyclique saturé azoté.

Différents sous-ensembles de composés sont définis ci-après, faisant également partie de l'invention.

Parmi les composés objets de l'invention, un premier groupe de composés est constitué par les composés de formule (I) telle que définie précédemment, dans laquelle :
X représente un groupe phényle éventuellement substitué par un ou plusieurs atomes d'halogène ou groupes cyano ;
les autres groupes étant tels que définis précédemment.

Parmi les composés objets de l'invention, un deuxième groupe de composés est constitué par les composés de formule (I) telle que définie précédemment, dans laquelle :
X représente un groupe phényle éventuellement substitué par un ou plusieurs atomes de fluor, de chlore ou groupes cyano ;
les autres groupes étant tels que définis précédemment.
   Parmi les composés objets de l'invention, un troisième groupe de composés est constitué par les composés de formule (I) telle que définie précédemment, dans laquelle R₂ représente un groupe hétérocyclique mono ou bicyclique aromatique, insaturé, partiellement saturé ou totalement saturé, comportant de 5 à 10 atomes dont 1 à 4 hétéroatomes choisis parmi N, O et S, à l'exclusion d'un groupe pipéridine, ledit hétérocycle étant éventuellement substitué par un ou plusieurs groupes choisis indépendamment les uns des autres parmi les atomes ou groupes suivants : hydroxy, (C₁-C₆)alcoxy, halogène, cyano, NRaRb, -CO-R₅, -CO-NR₆R₇, -CO-O-R₈, -NR₉-CO-R₁₀, un groupe oxido, un groupe (C₁-C₆)alkyle, lui-même éventuellement substitué par un ou plusieurs hydroxy ou groupe NRaRb;
les autres groupes étant tels que définis précédemment.

Parmi les composés objets de l'invention, un quatrième groupe de composés est constitué par les composés de formule (I) telle que définie précédemment, dans laquelle :
R₂ représente un groupe pyrrole, furane, thiophène, pyrazole, imidazole, triazole, tetrazole, oxazole, isoxazole, oxadiazole, thiazole, isothiazole, thiadiazole, pyridine, pyrimidine, pyrazine, pyridazine, triazine ; éventuellement substitué par un ou plusieurs groupes choisis parmi les atomes ou groupes suivants : hydroxy, (C₁-C₆)alkoxy, oxido, halogène -NRaRb, (C₁-C₆)alkyle lui-même éventuellement substitué par un groupe hydroxy,
Ra et Rb sont indépendamment l'un de l'autre, hydrogène, (C₁-C₆)alkyle;
Parmi les composés objets de l'invention, un quatrième groupe de composés est constitué par les composés de formule (I) telle que définie précédemment, dans laquelle :
R₂ représente un groupe dioxolane, pyridine, imidazole, pyrazole, triazole, pyrrole, furane, oxazole, imidazoline, thiophène, pyrazine, pyrimidine, thiazole ; éventuellement substitué par un ou plusieurs groupes choisis parmi les atomes ou groupes suivants : hydroxy, (C₁-C₆)alkoxy, oxido, halogène -NRaRb, (C₁-C₆)alkyle lui-même éventuellement substitué par un groupe hydroxy,
Ra et Rb sont indépendamment l'un de l'autre, hydrogène, (C₁-C₆)alkyle,
les autres groupes étant tels que définis précédemment.

Parmi les composés objets de l'invention, un quatrième groupe de composés est constitué par les composés de formule (I) telle que définie précédemment, dans laquelle :
R₂ représente un groupe indole, isoindole, benzimidazole, indazole, indolizine, benzofuran, isobenzofuran, benzothiophene, benzo[c]thiophen, pyrrolopyridine, imidazopyridine, pyrazolopyridine, triazolopyridine, tétrazolopyridine, pyrrolopyrimidine, imidazopyrimidine, pyrazolopyrimidine, triazolopyrimidine, tetrazolopyrimidine, pyrrolopyrazine, imidazopyrazine, pyrazolopyrazine, triazolopyrazine, tetrazolopyrazine, pyrrolopyridazine, imidazopyridazine, pyrazolopyridazine, triazolopyridazine, tetrazolopyridazine, pyrrolotriazine, imidazotriazine, pyrazolotriazine, triazolotriazine, tetrazolotriazine, furopyridine, furopyrimidine, furopyrazine, furopyridazine, furotriazine, oxazolopyridine, oxazolopyrimidine, oxazolopyrazine, oxazolopyridazine, oxazolotriaziie, isoxazolopyridine, isoxazolopyrimidine, isoxazolopyrazine, isoxazolopyridazine, isoxazolotriazine, oxadiazolopyridine, oxadiazolopyrimidine, oxadiazolopyrazine, oxadiazolopyridazine, oxadiazolotriazine, benzoxazole, benzisoxazole, benzoxadiazole, thiénopyridine, thiénopyrimidine, thiénopyrazine, thiénopyridazine, thiénotriazine, thiazolopyridine, thiazolopyrimidine, thiazolopyrazine, thiazolopyridazine, thiazolotriazine, isothiazolopyridine, isothiazolopyrimidine, isothiazolopyrazine, isothiazolopyridazine, isothiazolotriazine, thiadiazolopyridine, thiadiazolopyrimidine, thiadiazolopyrazine, thiadiazolopyridazine, thiadiazolotriazine, benzothiazole, benzoisothiazole, benzothiadiazole ; éventuellement substitué par un ou plusieurs groupes choisis parmi les atomes ou groupes suivants : hydroxy, (C₁-C₆)alkoxy, oxido, halogène -NRaRb, (C₁-C₆)alkyle lui-même éventuellement substitué par un groupe hydroxy,
Ra et Rb sont indépendamment l'un de l'autre, hydrogène, (C₁-C₆)alkyle ;
les autres groupes étant tels que définis précédemment.

Parmi les composés objets de l'invention, un septième groupe de composés est constitué par les composés de formule (I) telle que définie précédemment, dans laquelle :
R₂ représente un groupe indole éventuellement substitué par un ou plusieurs groupes choisis parmi les atomes ou groupes suivants : hydroxy, (C₁-C₆)alkoxy, oxido, halogène -NRaRb, (C₁-C₆)alkyle lui-même éventuellement substitué par un groupe hydroxy,
Ra et Rb sont indépendamment l'un de l'autre, hydrogène, (C₁-C₆)alkyle ;
les autres groupes étant tels que définis précédemment.

Parmi les composés objets de l'invention, un huitième groupe de composés est constitué par les composés de formule (I) telle que définie précédemment, dans laquelle :
R₂ représente un groupe dioxolane, pyridine, imidazole, pyrazole, triazole, pyrrole, furane, oxazole, indole, imidazoline, thiophène, pyrazine, pyrimidine, thiazole, éventuellement substitué par un ou plusieurs groupes hydroxy, méthyle, hydroxyméthyle, méthoxy, oxido, halogène, NH₂,
les autres groupes étant tels que définis précédemment.

Parmi les composés objets de l'invention, un neuvième groupe de composés est constitué par les composés de formule (I) telle que définie précédemment, dans laquelle R₁ représente un atome d'hydrogène ou un groupe (C₁-C₆)alkyle ;
les autres groupes étant tels que définis précédemment.

Parmi les composés objets de l'invention, un dixième groupe de composés est constitué par les composés de formule (I) telle que définie précédemment, dans laquelle R₁ représente un atome d'hydrogène ou un groupe méthyle ;
les autres groupes étant tels que définis précédemment.

Parmi les composés objets de l'invention, un onzième groupe de composés est constitué par les composés de formule (I) telle que définie précédemment, dans laquelle R₃ et R₄ représentent un atome d'hydrogène ;
les autres groupes étant tels que définis précédemment.

Parmi les composés objets de l'invention, un douzième groupe de composés est constitué par les composés de formule (I) telle que définie précédemment, dans laquelle :
X représente un groupe phényle éventuellement substitué par un ou plusieurs atomes de fluor, de chlore ou par un groupe cyano ;
R₂ représente un groupe dioxolane, pyridine, imidazole, pyrazole, triazole, pyrrole, furane, oxazole, indole, imidazoline, thiophène, pyrazine, pyrimidine, thiazole, éventuellement substitué par un ou plusieurs groupes hydroxy, méthyle, hydroxyméthyle, méthoxy, oxido, halogène, NH₂,
R₁ représente un atome d'hydrogène ou un groupe méthyle ;
R₃ et R₄ représentent un atome d'hydrogène ;
à l'état de base ou de sel d'addition à un acide.

Parmi les composés de formule (I) objets de l'invention, un treizième groupe de composés est constitué des composés pour lesquels :
X représente un groupe phényle éventuellement substitué par un ou plusieurs atomes de fluor ;
R₂ représente un groupe pyridine, furane, dioxolane, imidazole, pyrazole, triazole, pyrrole, thiazole, oxazole, pyrazine, pyrimidine, thiophène, indole, imidazoline ces groupes étant éventuellement substitué par un groupe hydroxyméthyle, NH₂, méthyle, méthoxy, hydroxy, oxido, ou un atome de fluor ;
R₁ représente un atome d'hydrogène ou un groupe méthyle ;
R₃ et R₄ représentent un atome d'hydrogène,
à l'état de base ou de sel d'addition à un acide.

Parmi les composés de formule (I) objets de l'invention, on peut notamment citer les composés suivants :
- 6-(1,3-Dioxolan-2-yl)-*N*-phénylimidazo[1,2-*a*]pyridine-2-carboxamide
- *N*-Phényl-6-(pyridin-3-yl)imidazo[1,2-*a*]pyridine-2-carboxarnide
- *N*-Phényl-6-(pyridin-2-yl)imidazo[1,2-*a*]pyridine-2-carboxamide
- 6-[5-(Hydroxyméthyl)pyridin-3-yl]-*N*-phénylimidazo[1,2-*a*]pyridine-2-carboxamide
- 6-[4-(Hydroxyméthyl)pyridin-2-yl]-*N*-phénylimidazo[1,2-*a*]pyridine-2-carboxamide et son chlorhydrate (1:1)
- 6-(6-Aminopyridin-2-yl)-*N*-phénylimidazo[1,2-*a*]pyridine-2-carboxamide et son chlorhydrate (1:1)
- 6-(1*H*-Imidazol-4-yl)-*N*-phénylimidazo[1,2-*a*]pyridine-2-carboxamide et son chlorhydrate (1 :1)
- *N*-Phényl-6-(1*H*-pyrazol-3-yl)imidazo[1,2-*a*]pyridine-2-carboxamide
- *N*-Phényl-6-(1*H*-1,2,4-triazol-3-yl)imidazo[1,2-*a*]pyridine-2-carboxamide
- *N*-Phényl-6-(1*H*-pyrrol-3-yl)imidazo[1,2-*a*]pyridine-2-carboxamide
- 6-(2-Méthyl-1*H*-imidazol-4-yl)-*N*-phénylimidazo[1,2-*a*]pyridine-2-carboxamide
- 6-(3-Furyl)-*N*-phénylimidazo[1,2-*a*]pyridine-2-carboxamide
- 6-(1*H*-Imidazol-1-yl)-*N*-phénylimidazo[1,2-*a*]pyridine-2-carboxamide et son trifluoroacetate (1:1)
- 6-(Oxazol-5-yl)-*N*-phénylimidazo[1,2-*a*]pyridine-2-carboxamide
- 6-(2-Amino-thiazol-4-yl)-*N*-phénylimidazo[1,2-*a*]pyridine-2-carboxamide
- 6-(2-Méthyl-1,3-dioxolan-2-yl)-*N*-phénylimidazo[1,2-*a*]pyridine-2-carboxamide
- 6-(4,5-Dihydro-1*H*-imidazol-2-yl)-*N*-phénylimidazo[1,2-*a*]pyridine-2-carboxamide et son chlorhydrate (1:1)
- 6-(6-Méthoxypyridin-2-yl)-*N*-phénylimidazo[1,2-*a*]pyridine-2-carboxamide
- 5-Méthyl-*N*-phényl-6-(pyridin-2-yl)imidazo[1,2-*a*]pyridine-2-carboxamide
- 6-(2-Amino-1*H*-imidazol-4-yl)-*N*-phénylimidazo[1,2-*a*]pyridine-2-carboxamide et son trifluoroacétate (1:1)
- 6-(2-Amino-thiazol-5-yl)-*N*-phénylimidazo[1,2-*a*]pyridine-2-carboxamide
- 6-(6-Hydroxypyridin-2-yl)-*N-*phénylimidazo[1,2-*a*]pyridine-2-carboxamide
- *N*-Phényl-6-(1*H*-1,2,3-triazol-4-yl)imidazo[1,2-*a*]pyridine-2-carboxamide
- *N*-(3,5-Difluorophényl)-6-(furan-3-yl)imidazo[1,2-*a*]pyridine-2-carboxamide
- *N*-(3-Fluorophényl)-6-(furan-3-yl)imidazo[1,2-*a*]pyridine-2-carboxamide
- 6-(2-Furyl)-*N*-phénylimidazo[1,2-*a*]pyridine-2-carboxamide
- 6-[6-(Hydroxyméthyl)pyridin-2-yl]-*N*-phénylimidazo[1,2-*a*]pyridine-2-carboxamide et son chlorhydrate (1:1)
- 6-(1-Oxidopyridin-3-yl)-*N*-phénylimidazo[1,2-*a*]pyridine-2-carboxamide et son chlorhydrate (1:1)
- *N*-Phényl-6-(1*H*-pyrazol-4-yl)imidazo[1,2-*a*]pyridine-2-carboxamide
- 6-(1*H*-Imidazol-2-yl)-*N*-phénylimidazo[1,2-*a*]pyridine-2-carboxamide, chlorhydrate (1:1)
- 6-(1-Méthyl-1*H*-imidazol-4-yl)-*N*-phénylimidazo[1,2-*a*]pyridine-2-carboxamide
- 6-(Oxazol-2-yl)-*N*-phénylimidazo[1,2-a]pyridine-2-carboxamide
- *N*-(3,5-Difluorophényl)-6-(pyridin-2-yl)imidazo[1,2-*a*]pyridine-2-carboxamide
- *N*-Phényl-6-(pyridin-4-yl)imidazo[1,2-a]pyridine-2-carboxamide
- 6-(1*H*-Indol-3-yl)-*N*-phénylimidazo[1,2-*a*]pyridine-2-carboxamide
- *N*-Phényl-6-(thiophén-2-yl)imidazo[1,2-*a*]pyridine-2-carboxamide
- *N*-Phényl-6-(pyrazin-2-yl)imidazo[1,2-*a*]pyridine-2-carboxamide
- 6-(1-Oxidopyridin-2-yl)-*N*-phénylimidazo[1,2-*a*]pyridine-2-carboxamide
- *N*-Phényl-6-(pyrimidin-5-yl)imidazo[1,2-*a*]pyridine-2-carboxamide
- *N*-Phényl-6-(thién-3-yl)imidazo[1,2-*a*]pyridine-2-carboxamide
- 6-(5-Fluoro-2-furyl)-*N*-phénylimidazo[1,2-*a*]pyridine-2-carboxamide
- *N*-(3-Fluorophényl)-6-(pyridin-2-yl)imidazo[1,2-*a*]pyridine-2-carboxamide
- *N*-(2-Fluorophényl)-6-(pyridin-2-yl)imidazo[1,2-*a*]pyridine-2-carboxamide
- N-(2,5-Difluorophényl)-6-(pyridin-2-yl)imidazo[1,2-*a*]pyridine-2-carhoxamide
- *N*-(2,3-Difluorophényl)-6-(pyridin-2-yl)imidazo[1,2-*a*]pyridine-2-carboxamide
- 6-(6-Aminopyridin-2-yl)-*N*-(3-fluorophényl)imidazo[1,2-*a*]pyridine-2-carboxamide
- *N*-(3-Fluorophényl)-6-(1*H*-pyrrol-3-yl)imidazo[1,2-*a*]pyridine-2-carboxamide
- *N*-(3-Fluorophényl)-6-(1*H*-pyrazol-3-yl)imidazo[1,2-*a*]pyridine-2-carboxamide
- *N*-(3-Fluorophényl)-6-(furan-2-yl)imidazo[1,2-*a*]pyridine-2-carboxamide
- *N*-(3-Fluorophényl)-6-(oxazol-2-yl)imidazo[1,2-*a*]pyridine-2-carboxamide
- *N*-Phényl-6-(pyrimidin-2-yl)imidazo[1,2-*a*]pyridine-2-carboxamide
- *N*-(3-Fluorophényl)-6-(1*H*-imidazol-4-yl)imidazo[1,2-*a*]pyridine-2-carboxamide
- *N*-(3-Fluorophényl)-6-(1*H*-1,2,4-triazol-3-yl)imidazo[1,2-*a*]pyridine-2-carboxamide
- 6-(2-Amino-thiazol-4-yl)-*N*-(3-fluorophényl)imidazo[1,2-*a*]pyridine-2-carboxamide
- *N*-(3-Fluorophényl)-6-(1*H*-1,2,3-triazol-4-yl)imidazo[1,2-*a*]pyridine-2-carboxamide
- *N*-(3-Fluorophényl)-6-(1*H*-pyrazol-4-yl)imidazo[1,2-*a*]pyridine-2-carboxamide
- *N*-(3-Fluorophényl)-6-[5-(hydroxyméthyl)furan-2-yl]imidazo[1,2-*a*]pyridine-2-carboxamide
- 6-[2-(Hydroxyméthyl)-1*H*-imidazol-4-yl]-*N*-phénylimidazo[1,2-*a*]pyridine-2-carboxamide
- *N*-(3,5-Difluorophényl)-6-(6-méthylpyridin-2-yl)imidazo[1,2-*a*]pyridine-2-carboxamide
- *N*-(2-Chlorophényl)-6-(pyridin-2-yl)imidazo[1,2-*a*]pyridine-2-carboxamide
- *N*-(3,5-Difluorophényl)-6-(1*H*-pyrrol-3-yl)imidazo[1,2-*a*]pyridine-2-carboxamide
- *N*-(2-Fluorophényl)-6-(1*H*-pyrrol-3-yl)imidazo[1,2-*a*]pyridine-2-carboxamide
- *N*-(2,5-Difluorophényl)-6-(1*H* pyrrol-3-yl)imidazo]1,2-*a*]pyridine-2-carboxamide
- *N*-(2,3-Difluorophényl)-6-(1*H*-pyrrol-3-yl)imidazo[1,2-*a*]pyridine-2-carboxamide
- *N*-(3,5-Difluorophényl)-6-(1*H*-pyrazol-3-yl)imidazo[1,2-*a*]pyridine-2-carboxamide
- *N*-(2-Fluorophényl)-6-(furan-2-yl)imidazo[1,2-*a*]pyridine-2-carboxamide
- *N*-(2,5-Difluorophényl)-6-(furan-2-yl)imidazo[1,2-*a*]pyridine-2-carboxamide
- *N*-(2,3-Difluorophényl)-6-(furan-2-yl)imidazo[1,2-*a*]pyridine-2-carboxamide
- *N*-(2-Chlorophényl)-6-(furan-2-yl)imidazo[1,2-*a*]pyridine-2-carboxamide
- *N*-(2,5-Difluorophényl)-6-(oxazol-2-yl)imidazo[1,2-*a*]pyridine-2-carboxamide
- *N*-(2-Chlorophényl)-6-(oxazol-2-yl)imidazo[1,2-*a*]pyridine-2-carboxamide
- *N*-(2-Fluorophényl)-6-(furan-3-yl)imidazo[1,2-*a*]pyridine-2-carboxamide
- *N*-(2,5-Difluorophényl)-6-(furan-3-yl)imidazo[1,2-*a*]pyridine-2-carboxamide
- *N*-(2,3-Difluorophényl)-6-(furan-3-yl)imidazo[1,2-*a*]pyridine-2-carboxamide
- *N*-(2-Chlorophényl)-6-(furan-3-yl)imidazo[1,2-*a*]pyridine-2-carboxamide
- *N*-(2,3-Difluorophényl)-6-[5-(hydroxyméthyl)furan-2-yl]imidazo[1,2-*a*]pyridine-2-carboxamide
- *N*-(2-Fluorophényl)-6-(1*H*-1,2,4-triazol-3-yl)imidazo[1,2-*a*]pyridine-2-carboxamide
- *N*-(2-Chlorophényl)-6-(1*H*-1,2,4-triazol-3-yl)imidazo[1,2-*a*]pyridine-2-carboxamide
- *N*-(2-Cyanophényl)-6-(pyridin-2-yl)imidazo[1,2-*a*]pyridine-2-carboxamide
- 6-(6-Aminopyridin-2-yl)-*N*-(3,5-difluorophényl)imidazo[1,2-*a*]pyridine-2-carboxamide et son dichlorhydrate
- 6-(6-Aminopyridin-2-yl)-*N*-(2-fluorophényl)imidazo[1,2-*a*]pyridine-2-carboxamide et son dichlorhydrate
- 6-(6-Aminopyridin-2-yl)-*N*-(2,5-difluorophényl)imidazo[1,2-*a*]pyridine-2-carboxamide et son dichlorhydrate
- 6-(6-Aminopyridin-2-yl)-*N*-(2,3-difluorophényl)imidazo[1,2-*a*]pyridine-2-carboxamide et son dichlorhydrate
- 6-(6-Aminopyridin-2-yl)-*N*-(2-chlorophényl)imidazo[1,2-*a*]pyridine-2-carboxamide et son dichlorhydrate
- *N*-(2-Chlorophényl)-6-(1*H*-pyrrol-3-yl)imidazo[1,2-*a*]pyridine-2-carboxamide
- *N*-(2-Fluorophényl)-6-(1*H*-pyrazol-3-yl)imidazo[1,2-*a*]pyridine-2-carboxamide
- *N*-(2-Chlorophényl)-6-(1*H*-pyrazol-3-yl)imidazo[1,2-*a*]pyridine-2-carboxamide
- *N*-(3,5-Difluorophényl)-6-(furan-2-yl)imidazo[1,2-*a*]pyridine-2-carboxamide
- *N*-(3,5-Difluorophényl)-6-(oxazol-2-yl)imidazo[1,2-*a*]pyridine-2-carboxamide
- *N*-(2-Fluorophényl)-6-(oxazol-2-yl)imidazo[1,2-*a*]pyridine-2-carboxamide
- *N*-(2,3-Difluorophényl)-6-(oxazol-2-yl)imidazo[1,2-*a*]pyridine-2-carboxamide
- *N*-(3,5-Difluorophényl)-6-[5-(hydroxyméthyl)furan-2-yl]imidazo[1,2-*a*]pyridine-2-carboxamide
- *N*-(2,5-Difluorophényl)-6-[5-(hydroxyméthyl)furan-2-yl]imidazo[1,2-*a*]pyridine-2-carboxamide
- *N*-(2,3-Difluorophényl)-6-(1*H*-imidazol-4-yl)imidazo[1,2-*a*]pyridine-2-carboxamide
- *N*-(2,5-Difluorophényl)-6-(1*H*-1,2,4-triazol-3-yl)imidazo[1,2-*a*]pyridine-2-carboxamide
- *N*-(2,3-Difluorophényl)-6-(1*H*-1,2,4-triazol-3-yl)imidazo[1,2-*a*]pyridine-2-carboxamide
- *N*-(3,5-Difluorophényl)-6-(1*H*-1,2,3-triazol-4-yl)imidazo[1,2-*a*]pyridine-2-carboxamide
- *N*-(2-Fluorophényl)-6-(1*H*-1,2,3-triazol-4-yl)imidazo[1,2-*a*]pyridine-2-carboxamide
- *N*-(2,5-Difluorophényl)-6-(1*H*-1,2,3-triazol-4-yl)imidazo[1,2-*a*]pyridine-2-carboxamide
- *N*-(2-Chlorophényl)-*6*-(1*H*-1,2,3-triazol-4-yl)imidazo[1,2-*a*]pyridine-2-carboxamide ;
et leurs sels d'addition à un acide.

Conformément à l'invention, on peut préparer les composés de formule générale (I) selon le procédé décrit dans le schéma 1.

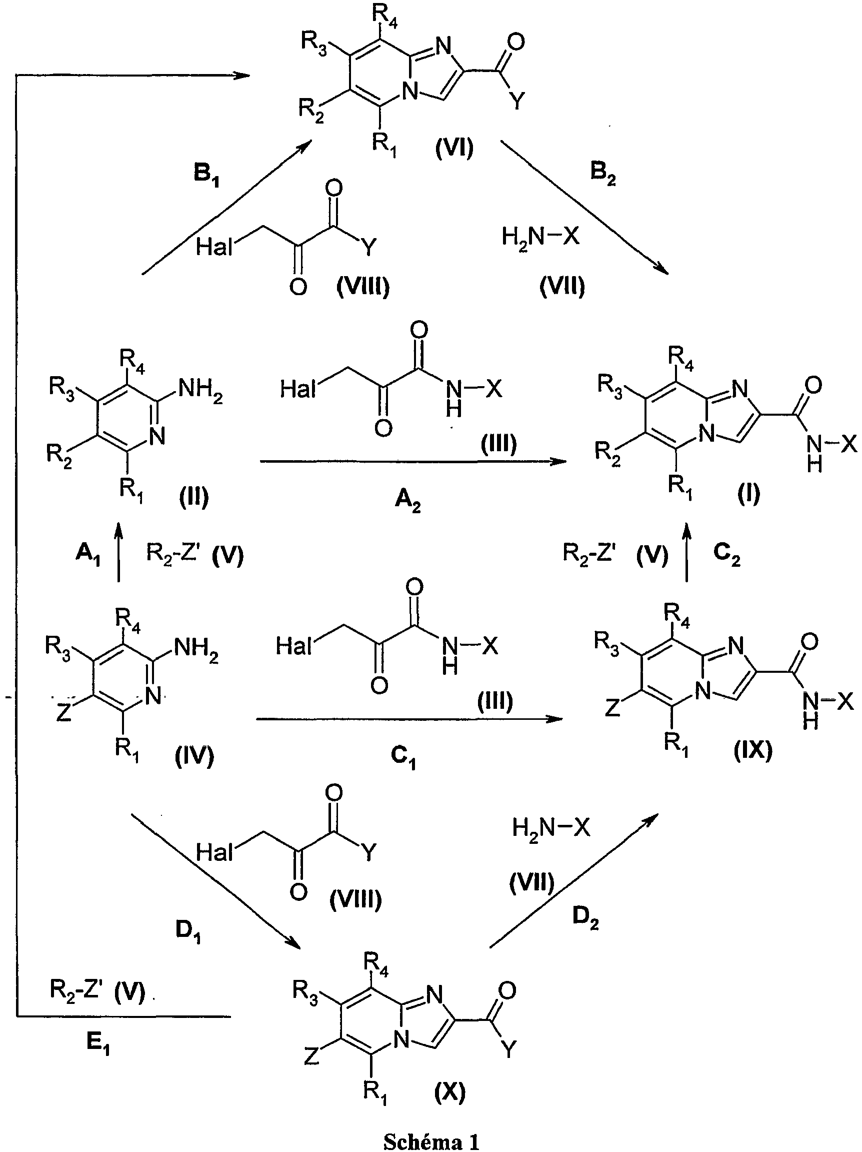

La première voie de synthèse (transformation A₂) consiste à préparer, selon les méthodes connues de l'homme du métier, une 2-amino-pyridine de formule (II), dans laquelle R₁, R₂, R₃ et R₄ sont définis comme précédemment, puis à former le cycle imidazo[1,2-*a*]pyridine par condensation d'un dérivé halogéné de 2-oxo-*N*-aryl-propionamide (III) dans lequel Hal représente un atome de chlore, de brome ou d'iode et X est défini comme précédemment par analogie avec les méthodes décrites par J-J. Bourguignon et coll. dans Aust. J. Chem., 50, 719 (1997) et par J.G. Lombardino dans J. Org. Chem., 30, 2403 (1965) par exemple.

Les dérivés halogènés de 2-oxo-*N*-aryl-propionamide (III) peuvent être obtenus par exemple selon la méthode décrite par R. Kluger et coll. dans J. Am. Chem. Soc., 106, 4017 (1984).

Les 2-amino-pyridines de formule (II), dans laquelle R₁, R₂, R₃ et R₄ sont définis comme précédemment peuvent être préparées par exemple par la transformation A₁, c'est à dire:
- par réaction de couplage d'un dérivé de 2-aminopyridine de formule (IV) dans laquelle R₁, R₃ et R₄ sont définis comme précédemment et Z représente un groupe boryle, stannyle ou silyle et un dérivé R₂-Z' (V) dans lequel R₂ est défini comme précédemment et Z' représente un atome d'halogène tel que brome ou iode ou un groupe sulfonyloxy,
- par réaction de couplage d'un dérivé de 2-aminopyridine de formule (IV), dans laquelle R₁, R₃ et R₄ sont définis comme précédemment et Z représente un atome d'halogène tel que brome ou iode avec un dérivé R₂-Z' (V) dans lequel R₂ est défini comme précédemment et Z' représente un groupe réactif tel qu'un groupe boryle, stannyle ou silyle ou un atome d'hydrogène, ou par toute autre méthode connue de l'homme du métier.

La seconde voie de synthèse (transformation B₂) consiste à coupler un acide imidazopyridine-2-carboxylique ou l'un de ses dérivés de formule (VI) dans laquelle R₁, R₂, R₃ et R₄ sont définis comme précédemment, Y représente un groupe hydroxy, un atome d'halogène ou un groupe (C₁-C₆)alcoxy, avec une arylamine X-NH₂ de formule (VII), dans laquelle X est défini comme précédemment, selon des méthodes connues de l'homme du métier. Ainsi l'acide peut être préalablement converti en l'un de ses dérivés réactifs tel que halogènure d'acide, anhydride, anhydride mixte ou ester activé puis mis en réaction avec l'amine (VII) en présence d'une base telle que la diisopropyléthylamine, la triéthylamine ou la pyridine, dans un solvant inerte tel que le THF, le DMF ou le dichlorométhane. Le couplage peut également être réalisé en présence d'un agent de couplage tel que CDI, EDCI, HATU ou HBTU dans les mêmes conditions sans isoler d'intermédiaire réactif. Alternativement, on peut faire réagir l'amine (VII) avec un ester de l'acide de formule (VI) en présence d'un catalyseur tel que le triméthylaluminium selon la méthode de Weinreb, S. et coll (Tet. Lett. (1977), 18,4171), ou le terbutylate de zirconium.

Les dérivés des acides imidazopyridine-2-carboxyliques de formule (VI) dans laquelle R1, R₂, R₃ et R₄ sont définis comme précédemment et Y est (C₁-C₆)alcoxy, hydroxy ou halogène, sont préparés par condensation d'une 2-aminopyridine de formule (II), dans laquelle R₁, R₂, R₃ et R₄ sont définis comme précédemment, sur un ester d'acide 3-halogèno 2-oxo-propionique de formule (VIII) dans laquelle Hal représente un halogène et Y est (C₁-C₆)alcoxy, dans les conditions similaires à celles utilisées pour la condensation d'un dérivé de formule (II) sur un dérivé de formule (III), suivie le cas échéant de la conversion de l'ester en acide puis en chlorure d'acide ou autre dérivé réactif (transformation B₁).

La troisième voie de synthèse (transformation C₂) consiste à coupler un dérivé de formule générale (IX) dans laquelle R₁, R₃, R₄ et X sont définis comme précédemment et Z représente un atome d'halogène tel que brome ou iode, un groupe sulfonyloxy ou un groupe réactif tel que boryle, stannyle ou silyle sur un dérivé de formule R₂-Z' (V) dans laquelle R₂ est défini comme précédemment et
- Z' représente un groupe réactif tel qu'un groupe boryle, stannyle ou silyle ou un atome d'hydrogène lorsque Z représente un atome d'halogène ou un groupe sulfonyloxy, ou
- Z' représente un atome d'halogène tel que brome ou iode lorsque Z représente un groupe réactif tel qu'un groupe boryle, stannyle ou silyle ou un atome d'hydrogène.

Les dérivés de formule générale (IX) dans laquelle R₁, R₃, R₄, X et Z sont définis comme précédemment peuvent être préparés :
- par condensation d'une 2-amino-pyridine de formule (IV) dans laquelle R₁, R₃, R₄ et Z sont définis comme précédemment, sur un dérivé de 2-oxo-N-aryl-propionamide (III) dans lequel Hal représente un atome de chlore, de brome ou d'iode et X est défini comme précédemment (transformation C₁) selon des méthodes citées pour la conversion d'un composé de formule (II) en composé de formule (I) ou
- par amidification d'un acide imidazopyridine-2-carboxylique ou l'un de ses dérivés de formule (X) dans laquelle R₁, R₂, R₃ et R₄ sont définis comme précédemment, Y un groupe hydroxy, un atome d' halogène ou un groupe (C₁-C₆)alcoxy avec une arylamine X-NH₂ de formule (VII), dans laquelle X est défini comme précédemment (transformation D₂), selon des méthodes citées pour la conversion d'un composé de formule (VI) en composé de formule (I).

Les acides imidazopyridine-2-carboxyliques ou leurs dérivés de formule (X), dans laquelle R₁, R₃ et R₄ sont définis comme précédemment, Y est (C₁-C₆)alcoxy, hydroxy ou halogène et Z représente un groupe boryle, stannyle ou silyle ou un atome d'halogène peuvent être préparés (transformation D₁) par condensation d'une 2-aminopyridine de formule (IV), dans laquelle R₁, R₃ et R₄ sont définis comme précédemment et Z représente un groupe boryle, stannyle ou silyle ou un atome d'halogène, avec un ester d'acide 3-halogèno 2-oxo-propionique de formule (VIII), dans laquelle Hal représente un halogène et Y est (C₁-C₆)alcoxy, dans les conditions similaires à celles citées précédemment pour la condensation des 2-aminopyridines de formule (II), sur un dérivé de formule (VIII) pour obtenir les acides imidazopyridine-2-carboxyliques ou leurs dérivés de formule (VI), selon la transformation B₁, suivie le cas échéant de la conversion de l'ester en acide puis en chlorure d'acide ou autre dérivé réactif.

Les acides imidazopyridine-2-carboxyliques ou leurs dérivés de formule (VI), dans laquelle R₁, R₂, R₃ et R₄ sont définis comme précédemment, Y est (C₁-C₆)alcoxy, hydroxy ou halogène peuvent également être préparés (transformation E₁) par couplage d'un dérivé de formule générale (X), dans laquelle R₁, R₃, et R₄ sont définis comme précédemment, Y est (C₁-C₆)alcoxy et Z représente un atome d'halogène tel que brome ou iode, un groupe sulfonyloxy ou un groupe réactif tel que boryle, stannyle ou silyle sur un dérivé de formule R₂-Z' (V), dans laquelle R₂ est défini comme précédemment et
- Z' représente un groupe réactif tel qu'un groupe boryle, stannyle ou silyle ou un atome d'hydrogène lorsque Z représente un atome d'halogène ou un groupe sulfonyloxy, ou
- Z' représente un atome d'halogène tel que brome ou iode lorsque Z représente un groupe réactif tel qu'un groupe boryle, stannyle ou silyle ou un atome d'hydrogène,
suivi le cas échéant de la conversion de l'ester en acide puis en chlorure d'acide ou autre dérivé réactif.

Les couplages des dérivés de formule (IV), (IX) ou (X) avec les produits de formule (V) peuvent être effectués par toute méthode connue de l'homme du métier, en particulier en opérant en présence de catalyseurs à base de cuivre ou de palladium, de ligands tels que des phosphines, selon ou par analogie avec les méthodes décrites par exemple dans les références suivantes et références citées :
- pour les réactions de type Suzuki : N. Miyaura, A. Suzuki, Chem. Rev., 95, 2457, (1995),
- pour les réactions de type Stille : V. Farina et coll., Org. React., 50, 1 (1997),
- pour les réactions de type Hiyama : T. Hiyama et coll., Top. Curr. Chem., 2002, 219, 61 (2002),
- pour les réactions de type Negishi : E. Negishi et coll., Chem. Rev., 103, 1979 (2003),
- pour les réactions de type Bellina : M. Miura et coll., Chem. Lett., 200 (2007).

Il est également possible, pour effectuer le couplage, de former intermédiairement, mais sans les isoler, des dérivés organométalliques tels que des dérivés zinciques.

Conformément à l'invention, on peut également préparer les composés de formule générale (I), (VI) et (II) selon les procédés décrits dans le schéma 2.

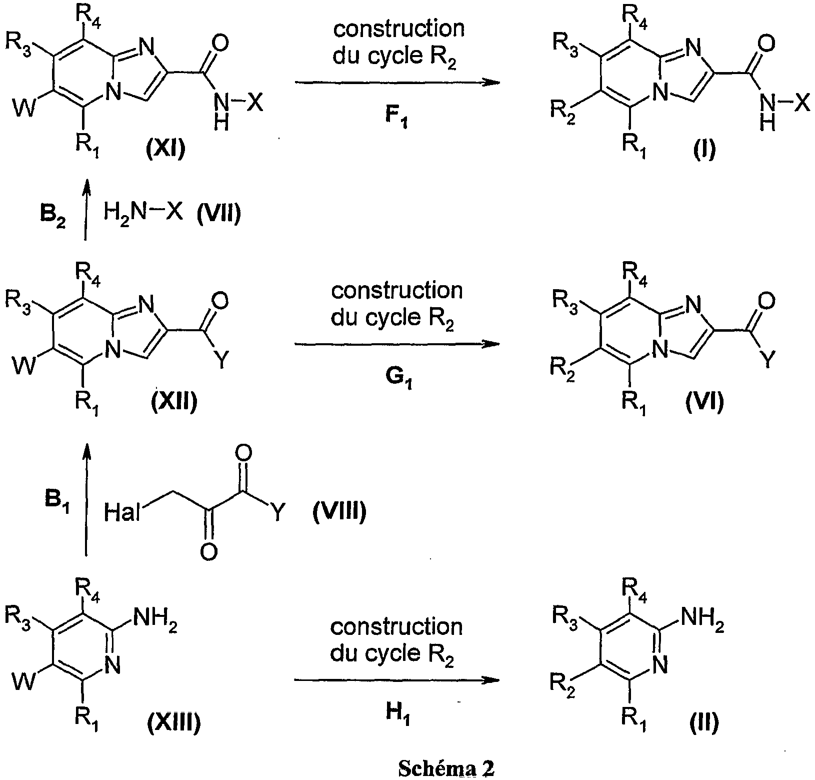

Cette voie de synthèse consiste en la conversion d'un composé de formules générales (XI), (XII) ou (XIII), dans lesquelles R₁, R₃, R₄, X et Y sont définis comme précédemment et W représente un groupement précurseur permettant la construction de l'hétérocycle de formule R₂, selon les méthodes connues de l'homme du métier.

A titre d'exemple W peut représenter :
- un groupement 2-halogéno-acyle tel que bromoacétyle, ou 1-halo-2-oxo-alkyle tel que 1-bromo-2-oxo-éthyle qui peut être converti, par exemple, en groupe thiazolyle, imidazolyle, oxazolyle par traitement par des dérivés de thiourée, de thioamide, de guanidine, d'urée ou d'amide,
- un groupement alkynyl, tel qu'éthynyl qui peut être converti en groupe 1,2,3-triazol-4-yl,
- un groupement acyle tel que formyl qui peut être converti, par exemple, en groupe 1,3-dioxolanyl-2 ou oxazolyl,
- un groupement cyano qui peut être converti, par exemple, en groupe dihydroimidazolyl(2) ou 1,3,4-triazol-2-yl.

Les composés de formule générale (XI) peuvent être obtenus à partir des composés de formule (XII) dans les conditions décrites pour la préparation des composés (I), à partir des dérivés d'acide imidazopyridine-2-carboxyliques de formule (VI) par les transformations B₂.

Les dérivés d'acide imidazopyridine-2-carboxyliques de formule générale (XII) peuvent être obtenus à partir des amino-pyridines de formule (XIII), dans les conditions décrites pour la conversion des amino-pyridines de formule (II) en composés de formule générale (I), par la transformation A₂.

Les produits de formule (I) et leurs précurseurs de formules (II), (IV), (VI), (IX) ou (X), peuvent être soumis, si désiré et si nécessaire, pour obtenir des produits de formule (I) ou être tranformés en d'autres produits de formule (I) à l'une ou plusieurs des réactions de transformations suivantes, dans un ordre quelconque :
a) une réaction d'estérification ou d'amidification de fonction acide,
b) une réaction d'amidification de fonction amine
c) une réaction d'hydrolyse de fonction ester en fonction acide,
d) une réaction de transformation de fonction hydroxyle en fonction alcoxy,
e) une réaction d'oxydation de fonction alcool en fonction aldéhyde ou cétone,
f) une réaction de transformation des fonctions aldéhyde ou cétone en fonction alcool, par réduction ou action d'un organométallique tel qu'un organomagnésien,
g) une réaction de transformation de radical nitrile en fonction aldéhyde,
h) une réaction de transformation de radical nitrile en fonction cétone,
i) une réaction d'oxydation de groupe alcènyle en fonction aldéhyde ou cétone,
j) une réaction de couplage catalytique d'un dérivé organométallique tel qu'un dérivé du bore, de l'étain ou du silicium avec un dérivé halogéné pour introduire un substituant alkyle, alcènyle, alcynyles, aryle ou hétéroaryle,
k) une réaction de conversion d'un groupe amino primaire ou secondaire en un groupe amino secondaire ou tertiaire par amination réductrice ou alkylation
l) une réaction de conversion d'un dérivé halogéné en un groupe amino secondaire ou tertiaire par substitution - éventuellement catalytique - par une amine primaire ou secondaire
m) une réaction de protection des fonctions réactives,
n) une réaction d'élimination des groupements protecteurs que peuvent porter les fonctions réactives protégées,
o) une réaction de salification par un acide minéral ou organique ou par une base pour obtenir le sel correspondant,
p) une réaction de dédoublement des formes racémiques en énantiomères,
lesdits produits de formule (I) ainsi obtenus étant le cas échéant sous toutes les formes isomères possibles : racémiques, énantiomères et diastéréoisomères.

Dans les schémas 1 et 2, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier.

Les exemples suivants décrivent la préparation de certains composés conformes à l'invention. Les numéros des composés exemplifiés renvoient à ceux donnés dans le tableau ci-après, qui illustre les structures chimiques et les propriétés physiques de quelques composés selon l'invention.

### Exemple 1 : 6-(1,3-Dioxolan-2-yl)-N-phénylimidazo[1,2-a]pyridine-2-carboxamide

A une solution de 137 mg de 6-formyl-*N*-phénylimidazo[1,2-*a*]pyridine-2-carboxamide (Intermédiaire 8) dans 5 mL de toluène, on ajoute 45 µL d'éthylèneglycol, 169 mg d'acide paratoluène sulfonique et du tamis moléculaire. Le mélange est chauffé au reflux pendant 24 heures dans un ballon muni d'un Dean-Stark, puis refroidi, filtré et dilué dans 100 mL de dichlorométhane et lavé par de la soude 2N et à l'eau. Les phases organiques sont séchées et concentrées à sec pour donner un mélange du produit de départ et du produit attendu que l'on redissout dans 5 mL de méthanol contenant 410 µL d'éthylèneglycol, 130 mg d'acide paratoluène sulfonique et du tamis moléculaire. Le mélange est chauffé 16 heures au reflux puis refroidi, filtré et concentré à sec. Le résidu est chromatographié sur une cartouche de silice en éluant par un mélange de dichlorométhane et d'acétate d'éthyle 70/30 pour donner 53 mg de 6-(1,3-dioxolan-2-yl)-*N*-phénylimidazo[1,2-*a*]pyridine-2-carboxamide sous la forme d'un solide blanc

### Exemple 2 : N-phényl-6-(pyridin-3-yl)imidazo[1,2-a]pyridine-2-carboxamide

Dans un tube à microondes on charge 150 mg de 6-bromo-*N*-phénylimidazo[1,2-*a*]pyridine-2-carboxamide (Intermédiaire 1), 0,237 g d'acide pyridine-3-boronique, 45 mg de tétrakis(triphénylphosphine)palladium, 2 mL de solution aqueuse 2M de carbonate de sodium, 4 mL d'acétonitrile et 4 mL de toluène. Le mélange est chauffé 20 minutes dans l'appareil à microondes réglé sur 150 °C, puis refroidi et filtré en lavant l'insoluble par un mélange de méthanol et de dichlorométhane. Les filtrats réunis sont concentrés à sec sous pression réduite. Le résidu est concrété par du méthanol aqueux pour donner 73 mg de *N*-phényl-6-(pyridin-3-yl)imidazo[1,2-a]pyridine-2-carboxamide sous la forme d'un solide écru.

### Exemple 3: N-phényl-6-(pyridin-2-yl)imidazo[1,2-a]pyridine-2-carboxamide

Dans un tube à microondes on charge 230 mg de 6-iodo-*N*-phénylimidazo[1,2-*a*]pyridine-2-carboxamide (Intermédiaire 2), 690 mg de 2-tributylstannyi-pyridine, 120 mg de tétrakis(triphénylphosphine)palladium et 4 mL de *N,N*-diméthylformamide. Le mélange réactionnel est chauffé 5 minutes dans l'appareil à microondes réglé sur 100 °C, puis 5 minutes à 150 °C et concentré à sec. Le résidu est chromatographié sur une cartouche de silice en éluant par un mélange de dichlorométhane et d'acétate d'éthyle. Les fractions contenant le produit attendu sont réunies et évaporées à sec sous pression réduite pour donner 56 mg de *N*-phényl-6-(pyridin-2-yl)imidazo[1,2-*a*]pyridine-2-carboxamide sous la forme d'un solide blanc.

### Exemple 4 : 6-[5-(Hydroxyyméthyl)pyridin-3-yl]-N-phénylimidazo[1,2-a] pyridine-2-carboxamide

Dans un tube à microondes on charge 163 mg de 6-triméthylstannyl-*N-*phénylimidazo[1,2-*a*]pyridine-2-carboxamide (Intermédiaire 4), 310 mg de (5-bromo-pyridin-3-yl)-méthanol, 66 mg de tétrakis(triphénylphosphine)palladium et 4 mL de *N,N-*diméthylformamide. Le mélange réactionnel est chauffé 20 minutes dans l'appareil à microondes réglé sur 150 °C et concentré à sec. Le résidu est chromatographié sur une cartouche de silice en éluant par du dichlorométhane. Les fractions contenant le produit attendu sont réunies et évaporées à sec sous pression réduite pour donner 61 mg de 6-[5-(hydroxyméthyl)pyridin-3-yl]-*N-*phénylimidazo[1,2-*a*]pyridine-2-carboxamide sous la forme d'un solide blanc.

### Exemple 5 : Chlorhydrate (1:1) de 6-[4-(hydroxyméthyl)pyridin-2-yl]-N-phénylimidazo[1,2-a]pyridine-2-carboxamide

### 5.1 6-[4-(hydroxyméthyl)pyridin-2-yl]-N-phénylimidazo[1,2-a]pyridine-2-carboxamide

Ce produit est obtenu de manière semblable à l'exemple 4 en remplaçant le (5-bromo-pyridin-3-yl)-méthanol par le (2-bromo-pyridin-4-yl)-méthanol.

### 5.2 Chlorhydrate (1:1) de 6-[4-(hydroxyméthyl)pyridin-2-yl]-N-phénylimidazo[1,2-a]pyridine-2-carboxamide.

Une suspension de 117 mg de 6-[4-(hydroxyméthyl)pyridin-2-yl]-*N-*phénylimidazo[1,2-*a*]pyridine-2-carboxamide dans 2 mL de méthanol est traitée par 0,68 mL de solution aqueuse 0,5 N d'acide chlorhydrique. Le mélange est agité 16 heures à température ambiante puis évaporé à sec sous pression réduite. Le solide obtenu est trituré dans du méthanol, filtré et séché pour donner 91 mg de chlorhydrate (1:1) de 6-[4-(hydroxyméthyl)pyridin-2-yl]-*N-*phénylimidazo[1,2-*a*]pyridine-2-carboxamide.

### Exemple 6 : Chlorhydrate (1:1) de 6-(6-aminopyridin-2-yl)-N-phénylimidazo[1,2-a]pyridine-2-carboxamide

Dans un tube à microondes on charge 236 mg de 6-triméthylstannyl-*N-*phénylimidazo[1,2-*a*]pyridine-2-carboxamide (Intermédiaire 4), 413 mg de 6-bromo-pyridin-2-ylamine, 95 mg de tétrakis(triphénylphosphine)palladium et 4 mL de *N,N-*diméthylformamide. Le mélange réactionnel est chauffé 45 minutes dans l'appareil à microondes réglé sur 150 °C et concentré à sec. Le résidu est chromatographié sur une cartouche de silice en éluant par un mélange de dichlorométhane et d'acétate d'éthyle. Les fractions contenant le produit attendu sont réunies et évaporées à sec sous pression réduite pour donner 147 mg de 6-(6-aminopyridin-2-yl)-*N*-phénylimidazo[1,2-*a*]pyridine-2-carboxamide sous la forme d'un solide jaune que l'on reprend par un mélange de dioxanne et méthanol et traite par 112 µL de solution 4 M d'acide chlorhydrique dans le dioxanne. Après 1 heure d'agitation à température ambiante, le précipité est essoré lavé au dioxanne et séché pour donner 156 mg de chlorhydrate (1:1) de 6-(6-aminopyridin-2-yl)-*N*-phénylimidazo[1,2-*a*]pyridine-2-carboxamide sous la forme d'un solide jaune pâle.

### Exemple 7 : Chlorhydrate (1:1) de 6-(1H-imidazol-4-yl)-N-phénylimidazo[1,2-a]pyridine-2-carboxamide

### 7.1 : 6-(1-Triphénylméthyl-1H-imidazol-4-yl)-N-phénylunidazo[1,2-a]pyridine-2-carboxamide

Dans un tube à microondes on charge 540 mg de *N*-phényl-6-(4,4,5,5-tétraméthyl-1,3,2-dioxaborolan-2-yl)imidazo[1,2-*a*]pyridine-2-carboxamide (Intermédiaire 5), 13 mL de dioxanne, 6,8 mL de solution 2M de carbonate de sodium, 843 mg de 4-iodo-1-triphénylméthylimidazole et 86 mg de tétrakis(triphénylphosphine)palladium. Le mélange est chauffé 10 minutes dans l'appareil à microondes réglé sur 120 °C, puis refroidi et concentré sous pression réduite. Le résidu est repris dans 100 mL de dichlorométhane. Après lavage à l'eau, la phase organique est séchée sur sulfate de magnésium et concentrée à sec sous pression réduite. Le résidu est chromatographié sur une cartouche de silice en éluant par un gradient de cyclohexane et d'acétate d'éthyle (de 100/0 à 60/40). Les fractions contenant le produit attendu sont réunies et concentrées à sec sous pression réduite. Le solide est cristallisé dans l'acétonitrile. Les cristaux sont lavés par de l'acétonitrile puis de l'éther éthylique puis séchés sous vide pour donner 342 mg de 6-(1-triphénylméthyl-1*H*-imidazol-4-yl)-*N*-phénylimidazo[1,2-*a*]pyridine-2-carboxamide sous la forme d'un solide blanc.

Spectre RMN ¹H (DMSO-d6, δ en ppm) : 7,09 (t, J = 7,5 Hz, 1H), 7,19 (d, J = 8,0 Hz, 6H), 7,33 (t, J = 7,5 Hz, 2H), de 7,38 à 7,49 (m, 9H), 7,53 (d, J = 1,5 Hz, 1H), 7,57 (d, J = 1,5 Hz, 1H), 7,59 (d, J = 9,5 Hz, 1H), 7,79 (dd, J = 1,5 et 9,5 Hz, 1H), 7,89 (d, J = 7,5 Hz, 2H), 8,50 (s, 1H), 9,03 (s large, 1H), 10,2 (s, lez.

Spectre de masse (LC-MS-DAD-ELSD) : m/z 546 [M+H]⁺.

### 7.2 : Chlorhydrate (1:1) de 6-(1H-imidazol-4-yl)-N-phénylimidazo[1,2-a]pyridine-2-carboxamide

Une suspension de 270 mg de 6-(1-triphénylméthyl-1*H*-imidazol-4-yl)-*N-*phénylimidazo[1,2-*a*]pyridine-2-carboxamide dans 4 mL d'acide chlorhydrique 2N est chauffée 30 minutes à 70 °C puis diluée par 2 mL de méthanol et chauffée 45 minutes au reflux et diluée encore par addition de 2 mL de dichlorométhane et chauffée encore 2 heures au reflux. Le mélange réactionnel est agité 60 heures à température ambiante. Le précipité est essoré lavé par du méthanol, de l'eau puis du pentane et séché sous pression réduite pour donner 96 mg de chlorhydrate (1:1) de 6-(1*H*-imidazol-4-yl)-*N*-phénylimidazo[1,2-*a*]pyridine-2-carboxamide sous la forme d'un solide blanc.

### Exemple 8 : N-phényl-6-(1H-pyrazol-3-yl)imidazo[1,2-a]pyridine-2-carboxamide

Dans un tube à microondes on charge 300 mg de 6-iodo-*N*-phénylimidazo[1,2-*a*]pyridine-2-carboxamide (Intermédiaire 2), 4 mL de dioxanne, 4 mL d'eau, 185 mg d'acide 1*H-*pyrazole-3- boronique, 45 mg de [1,1'-bis(diphenylphosphino)ferrocène]dichloropalladium, et 1,077 g de carbonate de césium. Le mélange est chauffé 20 minutes dans l'appareil à microondes réglé sur 160 °C, puis refroidi, dilué par 20 mL d'eau et extrait deux fois par 20 mL de dichlorométhane. Les phases organiques réunies sont séchées sur sulfate de magnésium, filtrées et concentrées à sec sous pression réduite. Le résidu est chromatographié sur une cartouche de silice en éluant par un gradient de dichlorométhane et de méthanol (de 0 à 10 %). Les fractions contenant le produit attendu sont réunies et concentrées à sec sous pression réduite pour donner 40 mg de *N*-phényl-6-(1Hpyrazol-3-yl)imidazo[1,2-*a*]pyridine-2-carboxamide sous la forme d'un solide blanc.

### Exemple 9 : N-phényl-6-(1H-1,2,4-triazol-3-yl)imidazo[1,2-a]pyridine-2-carboxamide

Dans un tube à microondes on charge 250 mg de 6-iodo-*N*-phénylimidazo[1,2-*a*]pyridine-2-carboxamide (Intermédiaire 2), 8 mL de *N,N*-diméthylformamide, 7,7 mg d'acétate de palladium, 197 mg d'iodure cuivreux et 71,3 mg de 1,2,4-triazole. Le mélange réactionnel est chauffé 2,5 heures dans l'appareil à microondes réglé sur 200 °C. Le mélange réactionnel refroidi est filtré, l'insoluble rincé par du *N,N*-diméthylformamide, du dichlorométhane et du méthanol et les filtrats réunis sont concentrés à sec sous pression réduite. Le résidu est chromatographié sur une cartouche de silice en éluant par un mélange de dichlorométhane.et de méthanol 90/10. Les fractions contenant le produit attendu pur sont réunies et évaporées à sec sous pression réduite pour donner 40 mg de *N*-phényl-6-(1I-1,2,4-triazol-3-yl)imidazo[1,2-*a*]pyridine-2-carboxamide sous la forme d'un solide blanc.

### Exemple 10: N-phényl-6-(1H-pyrrol-3-yl)imidazo[1,2-a]pyridine-2-carboxamide

### 10.1 : N-phényl-6-(1-triisopropylsilyl-1H-pyrrol-3-yl)-imidazo[1,2-a]pyridine-2-carboxamide

Ce produit est obtenu de manière semblable à l'exemple 8 en remplaçant l'acide 1*H-*pyrazole-3- boronique par l'acide 1-triisopropylsilyl-1*H*-pyrrol-3-boronique.

Spectre RMN ¹H (DMSO-d6, δ en ppm) : 1,09 (d, J = 7,5 Hz, 18H), 1,56 (m, 3H), 6,66 (s large, 1H), 6,95 (t, J = 2,0 Hz, 1H), 7,09 (t, J = 7,5 Hz, 1H), 7,33 (t, J = 7,5 Hz, 2H), 7,39 (s large, 1H), 7,61 (d, J = 9,5 Hz, 1H), 7,73 (dd, J = 1,5 et 9,5 Hz, 1H), 7,89 (d, J = 7,5 Hz, 2H), 8,39 (s, 1H), 8,82 (s large, 1H), 10,15 (s, 1H).

Spectre de masse (LC-MS-DAD-ELSD) : m/z 459 [M+H]⁺

### 10.2 : N-phényl-6-(1H-pyrrol-3-yl)imidazo[1,2-a]pyridine-2-carboxamide

Une solution de 120 mg de *N*-phényl-6-(1-triisopropylsilyl-1*H*-pyrrol-3-yl)-imidazo[1,2-*a*]pyridine-2-carboxamide dans 5 mL de tétrahydrofuranne est traitée par 262 µL d'une solution molaire de fluorure tétrabutylammonium dans le tétrahydrofuranne et agitée 10 minutes à température ambiante. Puis diluée par 15 mL de dichlorométhane et 20 mL d'eau. La phase organique est séchée et concentrée à sec sous pression réduite. Le résidu est repris dans 2 mL de dichlorométhane, le solide filtré est lavé deux fois par 1 mL de dichlorométhane puis deux fois par 1 mL d'éther diisopropylique, trituré 2 fois avec 2 mL d'eau, essoré et relavé par 2 mL de dichlorométhane et 2 mL d'éther diisopropylique puis séché pour donner 40 mg de *N*-phényl-6-(1*H*-pyrrol-3-yl)imidazo[1,2-*a*]pyridine-2-carboxamide sous la forme d'un solide blanc

### Exemple 11 : 6-(2-Méthyl-1H-imidazol-4-yl)-N-phénylimidazo[1,2-a]pyridine-2-carboxamide

Dans un tube à microondes on charge 400 mg de 6-triméthylstannyl-*N-*phénylimidazo[1,2-*a*]pyridine-2-carboxamide (Intermédiaire 4), 229 mg de 2-méthyl-4-iodo-1*H-*imidazole, 8 mL de tétrahydrofuranne, 9,1 mg de bis(dibenzylidèneacétone)palladium et 4,6 mg de tris(2-furylphosphine). Le mélange réactionnel est chauffé 50 minutes dans l'appareil à microondes réglé sur 130 °C. puis concentré à sec. Le résidu est repris dans 3,5 mL de *N,N-*diméthylformamide et on rajoute 150 mg de 2-méthyl-4-iodo-1*H*-imidazole, 10 mg de bis(dibenzylidèneacétone)palladium et 5 mg de tris(2-furylphosphine) avant de chauffer à nouveau 40 minutes dans l'appareil à microondes réglé sur 120 °C. Le mélange réactionnel est filtré, l'insoluble rincé par du méthanol et du dichlorométhane et les filtrats réunis concentrés sous pression réduite. Le résidu est chromatographié sur une cartouche de silice en éluant par du dichlorométhane puis par un mélange de dichlorométhane et de méthanol 90/10. Les fractions contenant le produit attendu pur sont réunies et évaporées à sec sous pression réduite pour donner 28 mg de 6-(2-méthyl-1*H*-imidazol-4-yl)-*N*-phénylimidazo[1,2-*a*]pyridine-2-carboxamide sous la forme d'un solide beige.

### Exemple 12 : 6-(3-Furyl)-N-phénylimidazo[1,2-a]pyridine-2-carboxamide

Dans un tube à microondes on charge 200 mg de 6-bromo-*N*-phénylimidazo[1,2-*a*]pyridine-2-carboxamide (Intermédiaire 1), 10 mL de dioxanne, 400 µL de 3-(triéthoxysilyl)furanne, 42 mg d'acétate de palladium, 42 mg de 1,4-diazabicyclo(2.2.2)octane (DABCO), 1,5 mL d'une solution molaire de fluorure tétrabutylammonium dans le tétrahydrofuranne. Le mélange réactionnel est chauffé 3 heures dans l'appareil à microondes réglé sur 120 °C, puis additionné de 200 µL de 3-(triéthoxysilyl)furanne, 20 mg d'acétate de palladium et 20 mg de DABCO et chauffé encore 1,5 heures dans l'appareil à microondes réglé sur 150 °C puis refroidi et filtré. Le filtrat est concentré à sec sous pression réduite et le résidu est chromatographié sur une cartouche de silice en éluant par du dichlorométhane. Les fractions contenant le produit attendu pur sont réunies et évaporées à sec sous pression réduite. Le solide est dissous à chaud dans un mélange de dichlorométhane et de méthanol. La solution chaude est filtrée et traitée par de l'éther diisopropylique. Le précipité formé est essoré et séché pour donner 25 mg de 6-(3-furyl)-*N*-phénylimidazo[1,2-*a*]pyridine-2-carboxamide sous la forme d'un solide jaune.

### Exemple 13 : Trifluoroacetate (1:1) de 6-(1H-imidazol-1-yl)-N-phénylimidazo[1,2-a]pyridine-2-carboxamide

Un mélange de 0,6 g de 6-iodo-*N*-phénylimidazo[1,2-a]pyridine-2-carboxamide (Intermédiaire 2), 20 mL de *N,N*-diméthylformamide, 1,08 g de carbonate de césium, 113 mg d'imidazole, 60 mg de 1,10-phénanthroline et 31,5 mg d'iodure cuivreux est chauffé pendant 21 heures à 130 °C et, après addition de 70 mg d'imidazole, encore 3 heures à la même température et 64 heures à température ambiante. Le mélange réactionnel est filtré, l'insoluble rincé par du dichlorométhane et les filtrats réunis concentrés sous pression réduite. Le résidu est purifié par CLHP préparative sur une colonne Waters Sunfire 30x100, 5 µm, en éluant par un gradient d'acétonitrile contenant de 0 à 60 % d'eau et 0,07 % d'acide trifluoroacétique en 15 minutes et avec un débit de 30 mL/min. Les fractions contenant le produit attendu pur sont réunies et évaporées à sec sous pression réduite. Le solide est battu dans 2 mL de méthanol, filtré et lavé par 1 mL de méthanol et séché pour donner 110 mg de trifluoroacetate (1:1) de 6-(1*H*-imidazol-1-yl)-*N*-phénylimidazo[1,2-*a*]pyridine-2-carboxamide sous la forme d'un solide beige.

### Exemple 14: 6-(Oxazol-5-yl)-N-phénylimidazo[1,2-a]pyridine-2-carboxamide

A une suspension de 200 mg de 6-formyl-*N*-phénylimidazo[1,2-*a*]pyridine-2-carboxamide (Intermédiaire 8) dans 10 mL de méthanol, on ajoute 104 mg de carbonate de potassium et 147 mg de paratoluènesulfonylméthylisonitrile (TOSMIC). Le mélange réactionnel est chauffé au reflux pendant 2 heures puis évaporé à sec sous pression réduite, repris dans 300 mL de dichlorométhane et lavé à l'eau. La phase organique est séchée et concentrée à sec sur de la silice pour être chromatographiée sur une cartouche de silice en éluant par un gradient de 0 à 20 % de méthanol dans le dichlorométhane. Les fractions contenant le produit attendu sont concentrées à sec et le solide obtenu trituré avec un peu de dichlorométhane, filtré et séché pour donner 80 mg de 6-(oxazol-5-yl)-*N*-phénylimidazo[1,2-*a*]pyridine-2-carboxamide sous la forme d'un solide blanc.

### Exemple 15: 6-(2-Amino-thiazol-4-yl)-N-phénylimidazo[1,2-a]pyridine-2-carboxamide

A une suspension de 60 mg de 6-(2-bromo-acétyl)-*N*-phényl-imidazo[1,2-*a*]pyridine-2-carboxamide brut dans 10 mL de méthanol, on ajoute 14 mg de thiourée. Le mélange réactionnel est chauffé pendant 45 minutes au reflux puis concentré à sec sous pression réduite. Le résidu est chromatographié sur une cartouche de silice en éluant par un mélange de dichlorométhane et de méthanol (gradient de 100/0 à 90/10). Les fractions contenant le produit attendu sont réunies et évaporées à sec sous pression réduite pour donner 20 mg de 6-(2-amino-thiazol-4-yl)-*N*-phénylimidazo[1,2-*a*]pyridine-2-carboxamide sous la forme d'un solide blanc.

### Exemple 16 : 6-(2-Méthyl-1,3-dioxolan-2-yl)-N-phénylimidazo[1,2-a]pyridine-2-carboxamide

Une mélange de 80 mg de 6-(1-éthoxyvinyl)-*N*-phénylimidazo[1,2-*a*]pyridine-2-carboxamide, 2,5 mL de toluène, 280 µL d'éthylèneglycol, 170 mg d'acide paratoluène sulfonique et du tamis moléculaire. Le mélange est chauffé au reflux pendant 2 heures, puis refroidi, filtré et dilué dans 20 mL de dichlorométhane et 20 mL d'eau et neutralisé par une solution de soude 2N. La phase aqueuse et lavée par du dichlorométhane et les phases organiques réunies sont séchées et concentrées à sec. Le résidu est chromatographié sur une cartouche de silice en éluant par un gradient de dichlorométhane et d'acétate d'éthyle de 0/30 à 70/30 pour donner 17 mg de 6-(2-méthyl-1,3-dioxolan-2-yl)-*N*-phénylimidazo[1,2-*a*]pyridine-2-carboxamide sous la forme d'un solide blanc.

### Exemple 17 : Chlorhydrates (1:1) et (2:1) de 6-(4,5-dihydro-1H-imidazol-2-yl)-N-phénylimidazo[1,2-a]pyridine-2-carboxamide

### 17.1 Chlorhydrate (1:1) de 2-phénylcarbamoyl-imidazo[1,2-a]pyridine-6-carboximidate d'éthyle

Une suspension de 300 mg de *N*-phényl-6-cyano-imidazo[1,2-*a*]pyridine-2-carboxamide (Intermédiaire 2) dans 25 mL d'éthanol additionnée de 0,5 mL de DMF est refroidie à 0 °C puis traitée pendant 1 heure 40 minutes par du gaz chlorhydrique. Le mélange réactionnel est agité à température ambiante pendant 16 heures puis concentré sous pression réduite à petit volume. Le précipité est essoré et lavé par de l'éthanol et de l'éther éthylique pour donner 296 mg de chlorhydrate (1:1) de 2-phénylcarbamoyl-imidazo[1,2-*a*]pyridine-6-carboximidate d'éthyle sous la forme d'un solide blanc qui est utilisé tel quel pour la suite de la synthèse.

### 17.2 Chlorhydrates (1:1) et (2:1) de 6-(4,5-dihydro-1H-imidazol-2-yl)-N-phénylimidazo[1,2-a]pyridine-2-carboxamide

Une suspension de 296 mg de chlorhydrate (1:1) de 2-phénylcarbamoyl-imidazo[1,2-*a*]pyridine-6-carboximidate d'éthyle dans 10 mL d'éthanol est refroidie à 0 °C avant addition de 144 µL d'éthylène diamine. Le mélange réactionnel est chauffé au reflux pendant 16 heures puis agité à température ambiante pendant 60 heures. Le précipité est essoré et lavé par de l'éthanol puis recristallisé dans du méthanol pour donner 64 mg de chlorhydrate (2:1) de 6-(4,5-dihydro-1*H*-imidazol-2-yl)-*N*-phénylimidazo[1,2-*a*]pyridine-2-carboxamide sous la forme d'un solide blanc. Le filtrat est concentré à sec et repris dans de l'eau. L'insoluble est essoré et lavé par du méthanol puis séché pour donner 99 mg de chlorhydrate (1:1) de 6-(4,5-dihydro-1*H*-imidazol-2-yl)-*N*-phénylimidazo[1,2-*a*]pyridine-2-carboxamide sous la forme d'un solide blanc.

### Exemple 18 : 6-(6-Méthoxypyridin-2-yl)-N-phénylimidazo[1,2-a]pyridine-2-carboxamide

A une solution de 624 mg de carbonate de césium, 90 mg de 2-bromo-6-méthoxypyridine, 17,5 mg de [1,1'-bis(diphenylphosphino)ferrocène]dichloropalladium dans 10 mL de dioxanne et 4 mL d'eau on ajoute 160 mg d'acide 2-phénylcarbamoyl-imidazo[1,2-*a*]pyridine-6-boronique. Le mélange est chauffé une heure au reflux, puis refroidi et concentré à sec sous pression réduite. Le résidu est repris dans 150 mL de dichlorométhane et lavé par 100 mL d'eau. La phase organique est séchée sur sulfate de magnésium, filtrée et concentrée à sec sous pression réduite sur de la silice pour être chromatographié sur une cartouche de silice en éluant par un gradient de 0 à 35 % d'acétate d'éthyle dans le cyclohexane. Les fractions contenant le produit attendu pur sont réunies et concentrées à sec sous pression réduite pour donner 36 mg de 6-(6-méthoxypyridin-2-yl)-*N*-phénylimidazo[1,2-*a*]pyridine-2-carboxamide sous la forme d'un solide blanc cassé.

### Exemple 19 : 5-Méthyl-N-phényl-6-(pyridin-2-yl)imidazo[1,2-a]pyridine-2-carboxamide

### 19.1 : 6-Iodo-5-méthyl-(pyridin-2-yl)imidazo[1,2-a]pyridine-2-carboxylate d'éthyle

A une suspension de 2 g de 5-iodo-6-méthyl-pyridine-2-amine dans 15 mL de diméthoxyéthane on ajoute 1,3 ml de bromopyruvate d'éthyle. Le mélange réactionnel est agité à 20 °C pendant 16 heures puis concentré à sec, repris dans 15 mL d'éthanol, chauffé au reflux pendant 2,5 heures et enfin concentré sous pression réduite. Le résidu est repris dans un mélange de dichlorométhane et d'une solution saturée de bicarbonate de sodium. La phase organique est séchée sur sulfate de magnésium et concentré à sec sous pression réduite pour donner 2,77 g de 6-iodo-5-méthyl-(pyridin-2-yl)imidazo[1,2-*a*]pyridine-2-carboxylate d'éthyle sous la forme d'un solide beige.

Spectre RMN 1H (DMSO-d6, δ en ppm) : 1,33 (t, J=7,1 Hz, 3H), 2,84 (s, 3H), 4,33 (q, J=7,1 Hz, 2H), 7,34 (d, J=9,3 Hz, 1H), 7,66 (d, J=9,3 Hz, 1H), 8,48 (s, 1H).

Spectre de masse (LC-MS-DAD-ELSD) : m/z 331 [M+H]+.

### 19.2 : 6-Iodo-5-méthyl-N-phényl-(pyridin-2-yl)imidazo[1,2-a]pyridine-2-carboxamide

A une solution de 852 µL d'aniline dans 104 mL de toluène refroidie à 0°, on ajoute goutte à goutte 6,2 mL d'une solution de triméthylaluminium 2M dans le toluène puis à 20 °C, 1,5 g de 6-iodo-5-méthyl-(pyridin-2-yl)imidazo[1,2-*a*]pyridine-2-carboxylate d'éthyle. Le mélange réactionnel est agité 2 heures à 20 °C. On refroidit à 4°C puis on ajoute 120 mL d'une solution saturée de chlorure d'ammonium. Après concentration sous pression réduite, le résidu est repris dans de l'acétate d'éthyle, la phase organique est lavée à l'eau, séchée sur sulfate de magnésium, filtrée sur célite, évaporée à sec sous pression réduite. Le résidu est trituré dans du méthanol, filtré et séché pour donner 1,15 g de 6-iodo-5-méthyl-*N-*phényl-(pyridin-2-yl)imidazo[1,2-*a*]pyridine-2-carboxamide sous la forme d'un solide jaune

Spectre RMN 1H (DMSO-d6, δ en ppm) : 2,89 (s, 3H), 7,10 (tt, J = 1,5 et 7,5 Hz, 1H), 7,33 (t, J = 7,5 Hz, 2H), 7,39 (d, J = 9,5 Hz, 1H), 7,70 (d, J = 9,5 Hz, 1H), 7,90 (d, J = 7,5 Hz, 2H), 8,49 (s, 1H), 10,3 (s, 1H).

Spectre de masse (LC-MS-DAD-ELSD) : m/z 378 [M+H]⁺.

### 19.3 : 5-Méthyl-N-phényl-6-(pyridin-2-yl)imidazo[1,2-a]pyridine-2-carboxamide

Ce produit est obtenu de manière analogue au produit de l'exemple 3 en remplaçant le 6-iodo-*N-*phénylimidazo[1,2-*a*]pyridine-2-carboxamide par le 6-iodo-5-méthyl-*N*-phényl-(pyridin-2-yl)imidazo[1,2-*a*]pyridine-2-carboxamide.

### Exemple 20 : Trifluoroacétate (1:1) de 6-(2-Amino-1H-imidazol-4-yl)-N-phénylimidazo[1,2-a]-pyridine-2-carboxamide

A une solution de 135 mg de 6-(2-bromo-acétyl)-*N*-phényl-imidazo[1,2-*a*]pyridine-2-carboxamide (Intermédiaire 9) dans 10 mL de *N,N*-diméthylformamide, on ajoute 190 mg de 1-*tert-*butyloxycarbonyl-guanidine. Le mélange réactionnel est agité pendant 16 heures à 20 °C puis concentré à sec à 60 °C sous pression réduite. Le résidu est repris dans 5 mL de dichlorométhane et 3 mL de méthanol et la solution évaporée sur silice pour être chromatographiée sur une cartouche de silice en éluant par un mélange de dichlorométhane et de méthanol (gradient de 100/0 à 90/10). Les fractions contenant le produit attendu sont réunies et évaporées à sec sous pression réduite pour donner 50 mg d'un mélange de 6-(2-*tert-*butyloxycarbonyl-amino-thiazol-4-yl)-*N*-phénylimidazo[1,2-*a*]pyridine-2-carboxamide et 6-(2-amino-1-*tert*-butyloxycarbonyl-thiazol-4-yl)-*N*-phényl-imidazo[1,2-*a*]pyridine-2-carboxamide sous la forme d'un solide beige. Ce produit est dissout dans 5 mL de dioxanne et traité par 320 µL d'une solution 4 N d'acide chlorhydrique dans le dioxanne. Le mélange est chauffé à 60 °C pendant 4 heures après ajout de 200 µL d'une solution 4 N d'acide chlorhydrique dans le dioxanne puis concentré à sec sous pression réduite. Le résidu est purifié par LC/MS préparative pour donner 18 mg de trifluoroacétate (1:1) de 6-(2-amino-1*H*-imidazol-4-yl)-*N*-phénylimidazo[1,2-*a*]pyridine-2-carboxamide sous la forme d'un solide beige.

### Exemple 21 : 6-(2-Amino-thiazol-5-yl)-N-phénylimidazo[1,2-a]pyridine-2-carboxamide

### 21.1 : 6-(2-Ethoxyvinyl)-N-phénylimidazo[1,2-a]pyridine-2-carboxamide

Ce produit est obtenu dans des conditions similaires à celles décrites dans la première étape de la préparation de l'intermédiaire 9, en remplaçant le tributyl(1-éthoxyvinyl)étain par du tributyl(2-éthoxyvinyl)étain.

Spectre RMN ¹H (DMSO-d6, δ en ppm) : 1,33 (t, J=7,2 Hz, 3H), 4,06 (q, J=7,2 Hz, 2H), 5,26 (d, J=6,8 Hz, 1H), 6,52 (d, J=6,8 Hz, 1H), 7,08 (t, J=7,8 Hz, 1H), 7,34 (t, J=7,8 Hz, 2H), 7,54 - 7,66 (m, 2H), 7,88 (d, J=7,8 Hz, 2H), 8,51 (s, 1H), 8,75 (s, 1H), 10.16 (s, 1H).

### 21.2 : 6-(2-Amino-thiazol-5-yl)-N-phénylimidazo[1,2-a]pyridine-2-carboxamide

A une solution de 168 mg de 6-(2-éthoxyvinyl)-*N*-phénylimidazo[1,2-*a*]pyridine-2-carboxamide dans 5,1 mL de tétrahydrofuranne on ajoute 1,4 mL d'eau puis, après refroidissement à 0 °C, une solution de 97 mg de *N*-bromo-succinimide dans 0,7 mL de tétrahydrofuranne. Le mélange réactionnel est agité pendant 3 heures à température ambiante. Le 6-(1-bromo-2-oxo-éthyl)-*N*-phényl-imidazo[1,2-*a*]pyridine-2-carboxamide formé n'est pas isolé du mélange réactionnel qui est traité par 42 mg de thiourée et agité encore 16 heures après avoir laissé la températureremonter à 20 °C. Le solide formé est essoré puis lavé par de l'eau puis par du méthanol et séché pour donner 51 mg de 6-(2-amino-thiazol-5-yl)-*N-*phénylimidazo[1,2-*a*]pyridine-2-carboxamide sous la forme d'un solide blanc.

### Exemple 22 : 6-(6-Hydroxypyridin-2-yl)-N-phénylimidazo[1,2-a]pyridine-2-carboxamide

### 22.1 : 6-(6-Benzyloxypyridin-2-yl)-N-phénylimidazo[1,2-a]pyridine-2-carboxamide

A une solution de 250 mg de 2-benzyloxy-6-bromopyridine, dans 12 mL de dioxanne, on ajoute une solution de 1,234 g de carbonate de césium dans 3 mL d'eau, 34,6 mg de [1,1'-bis(diphenylphosphino)ferrocène]dichloropalladium puis 546 mg de bromhydrate (1:1) de *N-*phényl-6-(4,4,5,5-tétraméthyl-1,3,2-dioxaborolan-2-yl)-imidazo[1,2-*a*]pyridine-2-carboxamide. Le mélange est chauffé à 110 °C pendant 2 h 45 min., puis refroidi et filtré. Le solide est lavé par un peu de méthanol puis du dichlorométhane puis repris dans 250 mL de méthanol bouillant additionné de 5 mL d'acide trifluoroacétique. L'insoluble est filtré et lavé par du méthanol puis du dichlorométhane et le filtrat concentré à sec sous pression réduite. Le résidu est chromatographié sur une cartouche de silice en éluant par un gradient de 0 à 5 % de méthanol dans le dichlorométhane. Les fractions contenant le produit attendu pur sont réunies et concentrées à sec sous pression réduite pour donner 0,3 g de 6-(6-benzyloxypyridin-2-yl)-*N*-phénylimidazo[1,2-*a*]pyridine-2-carboxamide sous la forme d'un solide blanc.

Spectre RMN 1H (DMSO-d6, δ en ppm) : 5,54 (s, 2H), 6,91 (d, J=8,3 Hz, 1H), 7,11 (t large, J=7,7 Hz, 1H), 7,31 - 7,45 (m, 5H), 7,54 (m, 2H), 7,63 (d, J=7,8 Hz, 1H), 7,77 (d, J=9,7 Hz, 1H), 7,85 - 7,93 (m, 3H), 8,12 (dd, J=9,7, 2,0 Hz, 1H), 8,64 (s, 1H), 9,44 (s large, 1H), 10,30 (s large, 1H).

Spectre de masse (LC-MS-DAD-ELSD) : m/z 421 [M+H]⁺.

### 22.2 : 6-(6-Hydroxypyridin-2-yl)-N-phénylimidazo[1,2-a]pyridine-2-carboxamide

Une solution de 300 mg de 6-(6-benzyloxypyridin-2-yl)-*N*-phénylimidazo[1,2-*a*]pyridine-2-carboxamide dans 3 mL d'acide trifluoroacétique est agitée à 25 °C pendant 48 heures puis évaporée à sec sous pression réduite à 45 °C. Le résidu est trituré avec de l'éther filtré et séché, puis trituré à nouveau avec 2 mL d'une solution saturée de bicarbonate de sodium, lavé 2 fois par 2 mL d'eau et 2 fois par 2 mL d'éther éthylique et séché sous pression réduite pour donner 168 mg de 6-(6-hydroxypyridin-2-yl)-*N*-phénylimidazo[1,2-*a*]pyridine-2-carboxamide sous la forme d'un solide beige.

### Exemple 23 : N-phényl-6-(1H-1,2,3-triazol-4-yl)imidazo[1,2-a]pyridine-2-carboxamide

### 23.1 6-Ethynyl-N-phénylimidazo[1,2-a]pyridine-2-carboxamide

Dans un tube à microondes de 20 mL on charge 0,2 g de 6-iodo-*N-*phénylimidazo[1,2-*a*]pyridine-2-carboxamide (Intermédiaire 2), 156µL de triméthylsilylacétylène, 20 mg de dichloro bis(triphénylphosphine)palladium, et 2 mL de pipéridine. Le mélange est chauffé 15 minutes dans l'appareil à microondes réglé sur 130 °C. Après refroidissement, le mélange est versé dans 50 mL de solution aqueuse saturée de chorure d'ammonium. On extrait 2 fois par 70 mL d'éther éthylique. Les phases organiques réunies sont décantées, séchées et concentrées à sec sous pression réduite. Le résidu est repris dans 4 mL d'une solution 1M de fluorure de tétrabutylammonium dans le THF et agité 16 heures à 25 °C. Après évaporation à sec du milieu réactionnel, le résidu est chromatographié sur silice en éluant par un mélange de cyclohexane et d'acétate d'éthyle (gradient de 0 à 35 %) pour donner 30 mg de 6-éthynyl-*N-*phénylimidazo[1,2-*a*]pyridine-2-carboxamide sous la forme d'un solide beige.

Spectre RMN ¹H (DMSO-d6, δ en ppm) : 4,37 (s, 1H), 7,09 (t, J = 8,0 Hz, 1H), 7,34 (t large, J = 8,0 Hz, 2H), 7,39 (d large, J = 9,5 Hz, 1H), 7,67 (d, J = 9,5 Hz, 1H), 7,89 (d large, J = 8,0 Hz, 2H), 8,48 (s, 1H), 8,92 (s large, 1H), 10,3 (s, 1H).

Spectre de masse (IE) : m/z 261 [M]⁺ (pic de base), m/z=221 [M-NHPh]⁺.

### 23.2 : N-phényl-6-(1H-1,2,3-triazol-4-yl)imidazo[1,2-a]pyridine-2-carboxamide

Dans un tube à microondes on charge 123 mg de 6-éthynyl-*N*-phénylimidazo[1,2-*a*]pyridine-2-carboxamide, 46 mg d'azoture de sodium, 38 mg de chlorure d'ammonium et 5 mL de *N,N*-diméthylformamide. Le mélange réactionnel est chauffé 20 minutes dans l'appareil à microondes réglé sur 170 °C puis à nouveau 30 minutes dans les mêmes conditions après ajout de 46 mg d'azoture de sodium et 38 mg de chlorure d'ammonium et enfin concentré à 50° sous pression réduite. Le résidu est repris dans 20 mL d'acétate d'éthyle et 20 mL d'eau. La phase aquese est extraite 2 fois par 20 mL d'acétate d'éthyle et les phases organiques réunies sont lavées par 30 mL de saumure saturée puis séchées sur sulfatede magnésium et concentrées à sec sous pression réduite. Le résidu est chromatographié sur une cartouche de silice en éluant par gradient de dichlorométhane et méthanol (de 100/0 à 90/10). Les fraction contenant le produit attendu sont réunies et et concentrées à sec sous pression réduite pour donner 36 mg de *N*-phényl-6-(1*H*-1,2,3-triazol-4-yl)imidazo[1,2-*a*]pyridine-2-carboxamide sous la forme d'un solide blanc cassé.

### Exemple 24 : N-(3,5-difluorophényl)-6-(furan-3-yl)imidazo[1,2-a]pyridine-2-carboxamide

Dans un tube à microondes on charge 83 mg de carbonate de potassium et 4 mL de diméthoxy-1,2-éthane puis après dégazage à l'argon, 120 mg de *N*-(3,5-difluorophényl)-6-iodoimidazo[1,2-*a*]pyridine-2-carboxamide (Intermédiaire 10), 40 mg d'acide 3-furanne-boronique et 21 mg de dichloro bis(triphenylphosphine)palladium(II). Le mélange réactionnel est chauffé 20 minutes dans l'appareil à microondes réglé sur 120 °C puis versé sur un mélange de 15 mL d'acétate d'éthyle et 15 mL d'eau. La phase aqueuse est extraite 2 fois par 15 mL d'acétate d'éthyle et les phases organiques réunies sont lavées par 15 mL de saumure saturée puis séchées sur sulfatede magnésium et concentrées à sec sous pression réduite. Le résidu est trituré 2 fois avec 10 mL de mélange méthanol-éther éthylique (1/1) puis lavé par de l'isopropanol et du pentane et séché pour donner 22 mg de *N*-(3,5-difluorophényl)-6-(furan-3-yl)imidazo[1,2-*a*]pyridine-2-carboxamide sous la forme d'un solide blanc.

### Exemple 25 : N-(3-fluorophényl)-6-(furan-3-yl)imidazo[1,2-a]pyridine-2-carboxamide

A une suspension de 65 mg d'acide 6-(furan-3-yl)imidazo[1,2-*a*]pyridine-2-carboxylique (Intermédiaire 19) et 104 mg de chlorhydrate de 1-(3-diméthylaminopropyl)-3-éthylcarbodiimide dans 2 mL de pyridine anhydre, placée sous argon, on ajoute 68 mg de 3-fluoroaniline. Le mélange réactionnel est agité pendant 16 heures à 80 °C puis concentré à sec sous pression réduite. Le résidu est repris par du dichlorométhane et lavé à l'eau. La phase organique est séchée sur sulfate de magnésium et concentrée à sec sous pression réduite pour donner 46 mg de *N*-(3-fluorophényl)-6-(furan-3-yl)imidazo[1,2-*a*]pyridine-2-carboxamide sous la forme d'un solide blanc.

Les intermédiaires décrits ci-dessous sont utiles à la préparation des composés de la présente invention.

### Intermédiaire 1 : 6-Bromo-N-phénylimidazo[1,2-a]pyridine-2-carboxamide

A une solution de 3 g d'aniline dans 366 mL de toluène refroidie à 0°, on ajoute goutte à goutte 22,5 mL d'une solution de triméthylaluminium 2M dans le toluène puis à 20 °C, 5,6 g de 6-bromo-imidazo[1,2-*a*]pyridine-2-carboxylate d'éthyle. Le mélange réactionnel est agité 2 heures à température ambiante. On refroidit à 4 °C puis ajoute 150 mL d'une solution saturée de chlorure d'ammonium. Le mélange réactionnel est concentré à sec puis repris dans 400 mL d'eau et 400 mL de dichlorométhane. La phase organique est séchée sur sulfate de magnésium, filtrée sur célite, évaporée à sec sous pression réduite pour donner 4,6 g de 6-bromo-*N-*phénylimidazo[1,2-*a*]pyridine-2-carboxamide sous la forme d'une poudre écru.

Spectre RMN ¹H (DMSO-d6, δ en ppm) : 7,09 (t, J = 7,5 Hz, 1H), 7,34 (t, J = 7,5 Hz, 2H), 7,51 (d, J = 9,5 Hz, 1H), 7,57 (dd, J = 1,5 et 9,5 Hz, 1H), 7,88 (d, J = 8,0 Hz, 2H), 8,42 (s, 1H), 9,02 (s large, 1H), 10,25 (s, 1H).

Spectre de masse (IE): m/z=363 [M]⁺, m/z=271 [M-C₆H₆N]⁺, m/z=144 [m/z=271-I]⁺.

### Intermédiaire 2 : 6-Iodo-N-phénylimidazo[1,2-a]pyridine-2-carboxamide

En opérant de la même manière que dans la préparation de l'intermédiaire 1, en remplaçant le 6-bromo-imidazo[1,2-*a*]pyridine-2-carboxylate d'éthyle par le 6-iodo-imidazo[1,2-*a*]pyridine-2-carboxylate d'éthyle, on obtient le 6-iodo-*N*-phénylimidazo[1,2-*a*]pyridine-2-carboxamide sous la forme d'un solide beige.

Spectre RMN ¹H (DMSO-d6, δ en ppm) : 7,09 (t, J = 7,5 Hz, 1H), 7,34 (t, J = 7,5 Hz, 2H), 7,51 (d, J = 9,5 Hz, 1H), 7,57 (dd, J = 1,5 et 9,5 Hz, 1H), 7,88 (d, J = 8,0 Hz, 2H), 8,42 (s, 1H), 9,02 (s large, 1H), 10,25 (s, 1H).

Spectre de masse (IE): m/z=363 [M]⁺, m/z=-271 [M-C₆H₆N]⁺, m/z=144 [m/z=271-I]⁺.

### Intermédiaire 3 : N-Phényl-6-cyano-imidazo[1,2-a]pyridine-2-carboxamide

En opérant de la même manière que dans la préparation de l'intermédiaire 1, en remplaçant le 6-bromo-imidazo[1,2-*a*]pyridine-2-carboxylate d'éthyle par le 6-cyano-imidazo[1,2-*a*]pyridine-2-carboxylate d'éthyle (J. Med. Chem. (1998), 41(22), 4317), on obtient le *N*-phényl-6-cyano-imidazo[1,2-*a*]pyridine-2-carboxamide sous la forme d'un solide jaune

Spectre RMN ¹H (DMSO-d6, δ en ppm) : 7,11 (t, J = 7,5 Hz, 1H), 7,35 (t, J = 7,5 Hz, 2H), 7,65 (dd, J = 2,0 et 9,5 Hz, 1H), 7,81 (d, J = 9,5 Hz, 1H), 7,89 (d, J = 8,0 Hz, 2H), 8,58 (s, 1H), 9,41 (s large, 1H), 10,4 (s large, 1H)

Spectre IR (KBr) : 3364; 2234; 1671; 1599; 1560; 1527; 1504; 1433 & 748 cm⁻¹ Spectre de masse (IE): m/z=262 [M]⁺ (pic de base), m/z=170 [M - C₆H₆N]⁺, m/z=143 [m/z=170-HCN]^{+.}

### Intermédiaire 4 : 6-Triméthylstannyl-N-phénylimidazo[1,2-a]pyridine-2-carboxamide

A une suspension de 160 mg de 6-bromo-*N*-phénylùnidazo[1,2-*a*]pyridine-2-carboxamide dans 10 mL de tolène on ajoute 260 µL d'hexaméthyldistannane et 30 mg de tétrakis-(triphénylphosphine)palladium(0). Le mélange réactionnel est chauffé 2 heures au reflux puis agité 16 h à température ambiante et filtré sur un culot de célite. Le filtrat est concentré sous pression réduite et le résidu est chromatographié sur une cartouche de silice en éluant par du dichlorométhane. Les fractions contenant le produit attendu sont réunies et évaporées à sec sous pression réduite pour donner 163 mg de 6-triméthylstannyl-*N*-phénylimidazo[1,2-*a*]pyridine-2-carboxamide sous la forme d'un solide jaune.

Spectre RMN ¹H (DMSO-d6, δ en ppm) : 0,85 (m, 9H), 7,09 (t, J = 7,5 Hz, 1H), 7,33 (t, J = 7,5 Hz, 2H), 7,40 (d large, J = 9,5 Hz, 1H), 7,61 (d large, J = 9,5 Hz, 1H), 7,89 (d, J = 7,5 Hz, 2H), 8,43 (s, 1H), 8,53 (m, 1H), 10,2 (s, 1H).

Spectre de masse (LC-MS-DAD-ELSD) : m/z 402, [M+H]⁺

### Intermédiaire 5 : N-phényl-6-(4,4,5,5-tétraméthyl-1,3,2-dioxaborolan-2-yl)-imidazo[1,2-a]pyridine-2-carboxamide et son bromhydrate (1:1)

A une solution de 1 g de 3-bromo-2-oxo-*N*-phényl-propionamide dans 50 mL de 1,2-diméthoxyéthane on ajoute 1,09 g de 2-amino-5-(4,4,5,5-tétraméthyl-1,3,2-dioxaborolan-2-yl)pyridine. Le mélange réactionnel est agité 40 heures à température ambiante puis concentré à sec sous pression réduite. Le résidu est repris dans 30 mL d'éthanol et chauffé au reflux pendant 90 minutes. Après concentration à sec sous pression réduite, le solide est trituré dans un peu d'éthanol, filtré et lavé par de l'éthanol puis par de l'éther éthylique pour donner 0,6 g de bromhydrate (1:1) de *N*-phényl-6-(4,4,5,5-tétrarnéthyl-1,3,2-dioxaborolan-2-yl)-imidazo[1,2-*a*]pyridine-2-carboxamide sous la forme d'un solide blanc.

Ce bromhydrate est repris dans 200 mL d'acétate d'éthyle et lavé par une solution saturée de bicarbonate de sodium. La phase organique est séchée et concentrée à sec sous pression réduite pour donner 0,53 g de *N*-phényl-6-(4,4,5,5-tétraméthyl-1,3,2-dioxaborolan-2-yl)imidazo[1,2-*a*]pyridine-2-carboxamide sous la forme d'un solide blanc.

Spectre RMN ¹H (DMSO-d6, δ en ppm) : 1,33 (s, 12H), 7,10 (t, J = 7,5 Hz, 1H), 7,34 (t, J = 7,5 Hz, 2H), 7,46 (dd, J = 1,5 et 9,5 Hz, 1H), 7,63 (d, J = 9,5 Hz, 1H), 7,89 (d, J = 7,5 Hz, 2H), 8,58 (s, 1H), 8,96 (s large, 1H), 10,25 (s, 1H).

Spectre de masse (IE) : m/z 363 [M]⁺ ; m/z 271= [M-NHPh]⁺ ;m/z 171 = [271-C6H12O]⁻.

### Intermédiaire 6 : Chlorhydrate (1:1) de l'acide 2-phénylcarbamoyl-imidazo[1,2-a]pyridine-6-boronique

1°) A une solution de 0,8 g de 3-bromo-2-oxo-*N*-phényl-propionamide dans 30 mL de 1,2-diméthoxyéthane on ajoute 0,87 g de 2-amino-5-(4,4,5,5-tétraméthyl-1,3,2-dioxaborolan-2-yl)pyridine. Le mélange réactionnel est agité 16 heures à température ambiante puis concentré à sec sous pression réduite. Le résidu est repris dans 15 mL d'éthanol et chauffé au reflux pendant 2 heures. Après concentration à sec sous pression réduite, le résidu est cristallisé dans l'éthanol, essoré et lavé par de l'éthanol puis par de l'éther éthylique pour donner 0,75 g de bromhydrate (1:1) de *N*-phényl-6-(4,4,5,5-tétraméthyl-1,3,2-dioxaborolan-2-yl)imidazo[1,2-*a*]pyridine-2-carboxamide sous la forme d'un solide blanc.
   Spectre RMN ¹H (DMSO-d6, δ en ppm) : 1,34 (s, 12 H), 7,11 (t, J=7,8 Hz, 1 H), 7,35 (t, J=7,8 Hz, 2 H), 7,54 (d large, J=9,3 Hz, 1 H), 7,66 (d, J=9,3 Hz, 1 H), 7,87 (d, J=7,8 Hz, 2 H), 8,63 (s, 1 H), 9,02 (s, 1 H), 10,37 (s large, 1 H).
   Spectre de masse (IE) : m/z 363 [M]⁺.
2°) Une solution de 0,19 g de bromhydrate (1:1) de *N*-phényl-6-(4,4,5,5-tétraméthyl-1,3,2-dioxaborolan-2-yl)imidazo[1,2-*a*]pyridine-2-carboxamide dans 9 mL d'acétonitrile est traitée par 0,5 mL d'acide chlorhydrique et par 1 g d'acide benzèneboronique supporté sur polymère (Alfa-Aesar L19459, - 3 mMol/g). Le mélange réactionnel est agité 16 h à 25 °C puis chauffé au reflux pendant 1 heure. La résine est filtrée, lavée à l'acétonitrilepuis au méthanol et les filtrats réunis sont évaporés à sec sous pression réduite pour donner 160 mg du chlorhydrate (1:1) de l'acide 2-phénylcarbamoyl-imidazo[1,2-*a*]pyridine-6-boronique sous la forme d'un solide orangé.
   Spectre RMN ¹H (DMSO-d6, δ en ppm) : 7,14 (t, J=7,8 Hz, 1 H), 7,38 (t, J=7,8 Hz, 2 H), 7,72 (m large, 1 H), 7,85 (d, J=7,8 Hz, 2 H), 7,93 (m large, 1 H), 7,94 - 8,63 (m très étalé, 2 H), 8,81 (m large, 1 H), 9,04 (m large, 1 H), 10,52 (m large, 1 H).

### Intermédiaire 7 : N-phényl-6-vinylimidazo[1,2-a]pyridine-2-carboxamide

Un mélange de 0,73 g de 6-iodo-*N*-phénylimidazo[1,2-*a*]pyridine-2-carboxamide, 209 mg de tétrakis(triphénylphosphine)palladium(0), 587 µL de tributylvinylétain et 17 mL de DMF est chauffé 10 minutes à 130 °C dans un appareil à microondes puis concentré à sec. Le résidu est repris dans 100 mL d'eau et extrait par deux fois 70 mL d'acétate d'éthyle. Les phases organiques réunies sont lavées par une solution saturée de chlorure de sodium, séchées sur sulfate de magnésium et évaporées à sec sous pression réduite. Le solide est trituré dans de l'acétate d'éthyle essoré ét lavé par de l'acétate d'éthyle puis par de l'éther isopropylique, repris dans un mélange de méthanol et de dichlorométhane. L'insoluble est filtre et lavé par du méthanol. Le filtrat est concentré à sec sous pression réduite pour donner 0,29 g de *N*-phényl-6-vinylimidazo[1,2-*a*]pyridine-2-carboxamide sous la forme d'un solide blanc.

Spectre RMN ¹H (DMSO-d6, δ en ppm) : 5,39 (d, J = 11,0 Hz, 1H), 5,92 (d, J = 17,5 Hz, 1H), 6,77 (dd, J = 11,0 et 17,5 Hz, 1H), 7,09 (t large, J = 7,5 Hz, 1H), 7,34 (t large, J = 7,5 Hz, 2H), 7,64 (d, J = 9,5 Hz, 1H), 7,70 (dd, J = 2,0 et 9,5 Hz, 1H), 7,89 (d large, J = 8,0 Hz, 2H), 8,47 (s, 1H), 8,66 (s large, 1H), 10,2 (s, 1H).

Spectre de masse (IE) : m/z 263 [M^{+.}]

### Intermédiaire 8 : 6-Formyl-N-phénylimidazo[1,2-a]pyridine-2-carboxamide

Une suspension de 150 mg de *N*-phényl-6-vinylimidazo[1,2-*a*]pyridine-2-carboxamide, 232 µL de tétroxyde d'osmium et 167,5 mg de périodate de sodium dans un mélange de 6 mL de THF, 3 mL de t-butanol et 3 mL d'eau, est agitée pendant 20 heures à 20 °C puis encore 48 heures en rajoutant à 4 reprises 100 µL de tétroxyde d'osmium et 80 mg de périodate de sodium. Le mélange réactionnel est versé dans 50 mL d'eau et que l'on extrait deux fois par 50 mL d'acétate d'éthyle. Les phases organiques réunies sont lavées par une solution saturée aqueuse de chlorure de sodium, décantées, séchées et concentrées à sec sous pression réduite. Le résidu est chromatographié sur silice en éluant par un mélange de cyclohexane et d'acétate d'éthyle (gradient de 0 à 50 %) pour donner 100 mg de 6-formyl-*N*-phénylimidazo[1,2-*a*]pyridine-2-carboxamide sous la forme d'un solide blanc

Spectre RMN ¹H (DMSO-d6, δ en ppm) : 7,11 (t, J = 8,0 Hz, 1H), 7,36 (t large, J = 8,0 Hz, 2H), 7,71 (dd, J = 1,5 et 9,5 Hz, 1H), 7,77 (d large, J = 9,5 Hz, 1H), 7,90 (d large, J = 8,0 Hz, 2H), 8,73 (s, 1H), 9,39 (s large, 1H), 10,0 (s, 1H), 10,35 (s large, 1H).

Spectre de masse (LC/MS) : m/z 266, [M+H⁺]

### Intermédiaire 9 : 6-(2-Bromo-acétyl)-N-phényl-imidazo[1,2-a]pyridine-2-carboxamide

### 9.1 : 6-(1-Ethoxyvinyl)-N-phénylimidazo[1,2-a]pyridine-2-carboxamide

A une suspension de 1 g de 6-iodo-*N*-phénylimidazo[1,2-*a*]pyridine-2-carboxamide dans 50 mL de toluène on ajoute 159 mg de tétrakis(triphénylphosphine)palladium et 1,09 g de tributyl(1-éthoxyvinyl)étain. Le mélange réactionnel est chauffé 9 heures à 150 °C, puis 16 heures à 130 °C et concentré à sec. Le résidu est repris dans du dichlorométhane et lavé par une solution aqueuse à 10 % de fluorure de potassium. La phase organique est séchée et concentrée à sec et le résidu est chromatographié sur une cartouche de silice en éluant par un mélange de cyclohexane et d'acétate d'éthyle. Les fractions contenant le produit attendu sont réunies et évaporées à sec sous pression réduite pour donner 0,52 g de 6-(1-éthoxyvinyl)-*N*-phénylimidazo[1,2-*a*]pyridine-2-carboxamide sous la forme d'un solide beige.

Spectre RMN ¹H (DMSO-d6, δ en ppm) : 1,40 (t, J = 7,0 Hz, 3H), 3,97 (q, J = 7,0 Hz, 2H), 4,46 (d, J = 3,0 Hz, 1H), 4,90 (d, J = 3,0 Hz, 1H), 7,09 (t, J = 8,0 Hz, 1H), 7,34 (t large, J = 8,0 Hz, 2H), 7,61 (d, J = 9,5 Hz, 1H), 7,66 (dd, J = 2,0 et 9,5 Hz, 1H), 7,89 (d large, J = 8,0 Hz, 2H), 8,57 (s, 1H), 8,83 (s large, 1H), 10,2 (s, 1H).

### 9.2 : 6-(2-Bromo-acétyl)-N-phényl-imidazo[1,2-a]pyridine-2-carboxamide

A une solution de 123 mg de 6-(1-éthoxyvinyl)-*N*-phénylimidazo[1,2-*a*]pyridine-2-carboxamide dans 4 mL de tétrahydrofuranne on ajoute 1 mL d'eau puis, après refroidissement à 0 °C, 71 mg de *N*-bromo-succinimide. Le mélange réactionnel est agité pendant 2,5 heures à température ambiante puis dilué par 80 mL de dichlorométhane. La phase organique est lavée à l'eau puis séchée et concentrée à sec sous pression réduite pour donner 60 mg de 6-(2-bromo-acétyl)-*N*-phényl-imidazo[1,2-*a*]pyridine-2-carboxamide brut sous la forme d'un solide blanc (contenant de petites quantités de dérivé 6-acétyl et de dérivé dibromé) utilisé sans autre purification.

Spectre RMN ¹H (DMSO-d6, δ en ppm) : 4,92 (s, 2H), 7,11 (t, J = 7,5 Hz, 1H), 7,36 (t, J = 7,5 Hz, 2H), 7,75 (d, J = 9,5 Hz, 1H), 7,82 (dd, J =1,5 et 9,5 Hz, 1H), 7,90 (d, J = 7,5 Hz, 2H), 8,61 (s, 1H), 9,57 (s large, 1H), 10,35 (s, 1H).

Spectre de masse (LC-MS-DAD-ELSD) : m/z 358 [M+H]⁺ , m/z 356 [M-H]⁻.

### Intermédiaire 10 : N-(3,5-difluorophényl)-6-iodoimidazo[1,2-a]pyridine-2-carboxamide

A une solution de 980 mg d'aniline dans 100 mL de toluène refroidie à 0°, on ajoute goutte à goutte 5 mL d'une solution de triméthylaluminium 2M dans le toluène puis à 20 °C, 1,5 g de 6-iodoimidazo[1,2-*a*]pyridine-2-carboxylate d'éthyle. Le mélange réactionnel est agité 2 heures à 20 °C. On refroidit à 4°C puis ajoute 100 mL d'une solution saturée de chlorure d'ammonium. Après concentration sous pression réduite, le résidu est repris dans de l'acétate d'éthyle, la phase organique est lavée à l'eau, séchée sur sulfate de magnésium, filtrée sur célite, évaporée à sec sous pression réduite. Le résidu est trituré dans de l'éther éthylique, filtré et séché pour donner 258 mg de *N*-(3,5-difluorophényl)-6-iodoimidazo[1,2-*a*]pyridine-2-carboxamide sous la forme d'un solide jaune.

Spectre RMN 1H (DMSO-d6, δ en ppm) : 6,92 (tt, J = 2,0 et 9,0 Hz, 1H), 7,51 (d, J = 9,5 Hz, 1H), 7,59 (dd, J = 1,5 et 9,5 Hz, 1H), 7,72 (m, 2H), 8,47 (s, 1H), 9,02 (s large, 1H), 10,75 (s, 1H).

Spectre de masse (LC-MS-DAD-ELSD) : m/z 398 [M+H]⁻; m/z 400 [M+H]⁺.

### Intermédiaire 11 : Acide 6-(6-{[(1,1-diméthyléthoxy)carbonyl]amino}pyridin-2-yl)imidazo[1,2-a]pyridine-2-carboxylique

### 11.1 : 6-(6-Aminopyridin-2-yl)imidazo[1,2-a]pyridine-2-carboxylate d'éthyle

350 mg de 2-amino-6-bromopyridine, 750 mg d'acide 2-éthoxycarbonyl-imidazo[1,2-*a*]pyridine-6-boronique et 57 mg de [1,1'-bis(diphenylphosphino)ferrocène]dichloropalladium sont dégazés sous vide puis mis en suspension, sous argon, dans 20 mL de dioxanne dégazé. Après ajout de 2 mL de solution aqueuse 2N de carbonate de sodium, le mélange est dégazé sous vide puis placé sous argon et chauffé 5 heures à 90 °C, puis refroidi, dilué et agité dans un mélange de 50 mL de solution saturée de bicarbonate de sodium et 50 mL de dichlorométhane. La phase organique est séchée sur sulfate de sodium, filtrée et concentrée à sec sous pression réduite. Le résidu est chromatographié sur silice en éluant par un mélange d'acétate d'éthyle et d'hexane. Les fractions contenant le produit attendu sont réunies et concentrées à sec sous pression réduite pour donner 446 mg de 6-(6-aminopyridin-2-yl)imidazo[1,2-*a*]pyridine-2-carboxylate d'éthyle.

Spectre RMN ¹H (DMSO-d6, δ en ppm) : 9,13 (dd, J = 1,0, 1,6 1H), 8,61 (d, J = 0,7, 1H), 7,94 (dd, J = 1,8, 9,6, 1H), 7,65 (d, J = 9,6, 1H), 7,50 (t, J = 8,1, 1H), 7,07 (d, J = 7,0, 1H), 6,48 (dd, J = 0,3, 8,1, 1H), 6,08 (s large, 2H), 4,33 (q, J = 7,1, 2H), 1,33 (t, J = 7,1, 3H).

Spectre de masse (APCI): m/z= 283 [M+H]⁺.

### 11.2 : 6-(6-{[(1,1-Diméthyléthoxy)carbonyl]amino}pyridin-2-yl)imidazo[1,2-a]pyridine-2-carboxylate d'éthyle et 6-(6-{bis[(1,1-diméthyléthoxy)carbonyl]amino}pyridin-2-yl)-imidazo[1,2-a]pyridine-2-carboxylate d'éthyle

A une suspension de 700 mg de 6-(6-aminopyridin-2-yl)imidazo[1,2-*a*]pyridine-2-carboxylate d'éthyle et 25 mg de 4-diméthylaminopyridine dans 5 mL d'acétonitrile on ajoute 1,14 mL de ditertbutyledicarbonate. Le mélange est agité pendant 16 heures à 25 °C puis concentré. Le résidu est chromatographié sur silice en éluant par un gradient d'acétate d'éthyle et d'hexane (de 50/50 à 100/0) pour donner 370 mg de 6-(6-{bis[(1,1-diméthyléthoxy)carbonyl]amino}pyridin-2-yl)imidazo[1,2-*a*]pyridine-2-carboxylate d'éthyle

Spectre RMN ¹H (DMSO-d6, δ en ppm) : 9,23 (s, 1H), 8,65 (s, 1H), 8,06,-7,98 (m, 2H), 7,95 (d, J = 7,7, 1H), 7,76 (d, J = 9,6, 1H), 7,43 (d, J = 7,8, 1H), 4,33 (q, J = 7,0, 2H), 1,43 (s, 18H), 1,34 (t, J = 7,1, 3H).

Spectre de masse (APCI): m/z= 483 [M+H]⁺,
et 163 mg de 6-(6-{[(1,1-diméthyléthoxy)carbonyl]amino}pyridin-2-yl)imidazo[1,2-*a*]pyridine-2-carboxylate d'éthyle

Spectre RMN ¹H (DMSO-d6, δ en ppm) : 9,28 (s, 1H), 8,50 (s, 1H), 8,04-8,00 (m, 2H), 7,95 (d, J = 7,8, 1H), 7,70 (d, J = 9,6, 1H), 7,38 (d, J = 7,9, 1H), 4,31 (q, J = 7,0, 2H), 1,39 (s, 9H), 1,33 (t, J = 7,1, 3H).

Spectre de masse (APCI): m/z= 383 [M+H]⁺.

### 11.3 : Acide 6-(6-{[(1,1-diméthyléthoxy)carbonyl]amino}pyridin-2-yl)imidazo[1,2-a]pyridine-2-carboxylique

0,9 mL d'une solution aqueuse 2 M de lithine sont ajoutés à une solution de 292 mg de 6-(6-{bis[(1,1-diméthyléthoxy)carbonyl]amino}pyridin-2-yl)imidazo[1,2-*a*]pyridine-2-carboxylase d'éthyle dans 4,73 mL de mélange 50:1 de tétrahydrofuranne et de méthanol. Le mélange réactionnel est agité pendant 7 heures à 25 °C, puis traité goutte à goutte à 0 °C par de l'acide chlorhydrique 2 N HCl jusqu'à atteindre un pH de 3. Le précipité formé après 20 minutes est essoré et lavé par de l'eau (20 mL) et de l'éther diéthylique (20 ml) puis séché sous pression réduite pour donner 195 mg d'acide 6-(6-{[(1,1-diméthyléthoxy)carbonyl]amino}pyridin-2-yl)imidazo[1,2-*a*]pyridine-2-carboxylique sous la forme d'un solide beige.

Spectre RMN ¹H (DMSO-d6, δ en ppm) : 13,5-12,0 (br, 1H), 9,80 (s, 1H), 9,24 (s, 1H), 8,51 (s, 1H), 8,03 (dd, J = 1,5, 9,6 1H), 7,88 (app, t, J = 8,0, 7,8, 1H), 7,77 (d, J = 8,2, 1H), 7,73 (d, J = 9,6, 1H), 7,62 (d, J = 7,5, 1H), 1,50 (s, 9H)

### Intermédiaire 12 : Acide 6-(pyridin-2-yl)imidazo[1,2-a]pyridine-2-carboxylique

### 12.1 : (6-Pyridin-2-yl)imidazo[1,2-a]pyridine-2-carboxylate d'éthyle

Un mélange de 3,18 g de carbonate de césium, 25 mL de dioxanne, 9,3 mL d'eau, 500 mg de 2-iodopyridine, 89 mg de [1,1'-bis(diphenylphosphino)ferrocène]dichloropalladium et 848 mg de bromhydrate (1:1) de 6-(4,4,5,5-tétraméthyl-1,3,2-dioxaborolan-2-yl)imidazo[1,2-*a*]pyridine-2-carboxylate d'éthyle est chauffé 2 heures à 110 °C, puis partiellement concentré et dilué avec du dichlorométhane et filtré. La phase organique est lavée à l'eau et séchée sur sulfate de magnésium, filtrée et concentrée à sec sous pression réduite. Le résidu est chromatographié sur une cartouche de silice en éluant par un mélange de dichlorométhane et de cyclohexane (80/20). Les fractions contenant le produit attendu sont réunies et concentrées à sec sous pression réduite pour donner 317 mg de 6-(pyridin-2-yl)imidazo[1,2-*a*]pyridine-2-carboxylate d'éthyle sous la forme d'une huile brune.

Spectre RMN ¹H (DMSO-d6, δ en ppm) : 1,34 (t, J=7,0 Hz, 3H), 4,33 (q, J=7,0 Hz, 2H), 7,42 (ddd, J=7,5, 5,5, 2,0 Hz, 1H), 7,73 (d, J=9,3 Hz, 1H), 7,85 - 8,02 (m, 2H), 8,07 (dd, J=9,3, 2,0 Hz, 1H), 8,64 (s, 1H), 8,70 (d large, J=5,5 Hz, 1H), 9,36 (s large, 1H).

Spectre de masse (LC-MS-DAD-ELSD) : m/z 268 [M+H]⁺.

### 12.2 : Acide 6-(pyridin-2-yl)imidazo[1,2-a]pyridine-2-carboxylique

317 mg de 6-(pyridin-2-yl)imidazo[1,2-*a*]pyridine-2-carboxylate d'éthyle sont saponifiés dans des conditions analogues à celles décrites pour la préparation de l'intermédiaire 11 (étape 11.3) pour donner 280 mg d'acide 6-(pyridin-2-yl)imidazo[1,2-*a*]pyridine-2-carboxylique sous la forme d'un solide rosé pâteux.

Spectre RMN ¹H (DMSO-d6, δ en ppm) : 7,47 (m, 1H), 7,83 (d, J=9,8 Hz, 1H), 7,99 (dt, J=8,5, 2,0 Hz, 1H), 8,06 (d, J=8,5 Hz, 1H), 8,31 (d large, J=9,8 Hz, 1H), 8,73 (m, 2 H), 9,52 (s large, 1H).

Spectre de masse (LC-MS-DAD-ELSD) : m/z 240 [M+H]⁺.

### Intermédiaire 13 : Acide 6-(1-triphénylméthyl-1H-imidazol-4-yl)imidazo[1,2-a]pyridine-2-carboxyliqne

### 13.1 : 6-(1-Triphénylméoxyl-1H-imidazol-4-yl)imidazo[1,2-a]pyridine-2-carboxylate d'éthyle

873 mg de 4-iodo-1-triphénylméthyl-imidazole, 750 mg d'acide 2-éthoxycarbonyl-imidazo[1,2-*a*]pyridine-6-boronique, 23 mg d'acétate de palladium et 70 mg de (2-biphényl)-dicyclohexylphosphine sont dégazés sous vide puis mis en suspension, sous argon, dans un mélange dégazé de 15 mL de toluène, 5 mL d'eau et 5 mL de N-méthylpyrrolidone. Après ajout de 950 mg de phosphate de potassium, le mélange est dégazé sous vide puis placé sous argon et chauffé 15 minutes à 100 °C sous microondes, puis refroidi, dilué et agité dans un mélange de 50 mL de solution saturée de bicarbonate de sodium et 50 mL de dichlorométhane. La phase organique est séchée sur sulfate de sodium, filtrée et concentrée à sec sous pression réduite. Le résidu est chromatographié sur silice en éluant par un mélange d'acétate d'éthyle et d'hexane. Les fractions contenant le produit attendu sont réunies et concentrées à sec sous pression réduite pour donner 508 mg de 6-(1-triphénylméthyl-1*H*-imidazol-4-yl)imidazo[1,2-*a*]pyridine-2-carboxylate d'éthyle.

Spectre RMN ¹H (DMSO-d6, δ en ppm) : 8,97 (s, 1H), 8,54 (s, 1H), 7,76-7,72 (m, 1H), 7,56-7,52 (m, 3H), 7,47-7,37 (m, 9H), 7,20-7,17 (m, 6H), 4,31-4,27 (m, 2H), 1,34-1,20 (m, 3H).

Spectre de masse (APCI): m/z= 499 [M+H]⁺.

### 13.2 : Acide 6-(1-triphénylméthyl-1H-imidazol-4-yl)imidazo[1,2-a]pyridine-2-carboxylique

500 mg de 6-(1-triphénylméthyl-1*H*-imidazol-4-yl)imidazo[1,2-*a*]pyridine-2-carboxylate d'éthyle sont saponifiés dans des conditions analogues à celles décrites pour la préparation de l'intermédiaire 11 (étape 11.3) pour donner 346 mg d'acide 6-(1-triphénylméthyl-1*H*-imidazol-4-yl)imidazo[1,2-*a*]pyridine-2-carboxylique.

Spectre RMN ¹H (DMSO-d6, δ en ppm) : 9,01 (s, 1H), 8,51 (s, 1H), 7,83 (d, J = 9,5, 1H), 7,59-7,56 (m, 3H), 7,47-7,37 (m, 9H), 7,20-7,17 (m, 6H). Un proton échangeable n'est pas observé.

Spectre de masse (APCI): m/z= 471 [M+H]⁺.

### Intermédiaire 14 : Acide 6-(2-{[(1,1-diméthyléthoxy)carbonyl]amino}-thiazol-4-yl)imidazo[1,2-a]pyridine-2-carboxylique

### 14.1 : 6-(2-{[(1,1-Diméthyléthoxy)carbonyl]amino}-thiazol-4-yl)imidazo[1,2-a]pyridine-2-carboxylate d'éthyle

465 mg de 4-iodothiazol-2-ylcarbamate de tert-butyle, 434 mg d'acide 2-éthoxycarbonyl-imidazo[1,2-*a*]pyridine-6-boronique et 104 mg de [1,1'-bis(diphenylphosphino)-ferrocène]dichloropalladium sont dégazés sous vide. Après ajout de 10 mL de tétrahydrofuranne dégazé et 0,66 mL de solution aqueuse 2N de carbonate de sodium, le mélange réactionnel est chauffé 2 heures à 100 °C, puis refroidi, dilué dans du dichlorométhane et lavé par une solution aqueuse demi-saturée de bicarbonate de. La phase organique est séchée sur sulfate de magnésium, filtrée et concentrée à sec sous pression réduite. Le résidu est chromatographié sur silice en éluant par un mélange de dichlorométhane:méthanol (99:1 à 99:2). Les fractions contenant le produit attendu sont réunies et concentrées à sec sous pression réduite. Le solide obtenu est lavé par 5 mL d'éther diéthylique pour donner 125 mg de 6-(2-{[(1,1-diméthyléthoxy)carbonyl]amino}-thiazol-4-yl)imidazo[1,2-*a*]pyridine-2-carboxylate d'éthyle sous la forme d'un solide blanc cassé.

Spectre RMN ¹H (DMSO-d6, δ en ppm) : 11,65 (s, 1H), 8,84 (s, 1H), 8,47 (s, 1H), 7,84 (s, 1H), 7,68-7,71 (m, 2H), 4,32 (q, J = 7,1, 2H), 1,51 (s, 9H), 1,33 (t, J = 7,1, 3H).

Spectre de masse (APCI): m/z= 389 [M+H]⁺.

### 14.2 : Acide 6-(2-{[(1,1-diméthyléthoxy)carbonyl]amino}-thiazol-4-yl)imidazo[1,2-a]pyridine-2-carboxylique

125 mg de 6-(2-{[(1,1-diméthyléthoxy)carbonyl]amino}-thiazol-4-yl)imidazo[1,2-*a*]pyridine-2-carboxylate d'éthyle sont saponifiés dans des conditions analogues à celles décrites pour la préparation de l'intermédiaire 11 (étape 11.3) pour donner 90 mg d'acide 6-(2-{[(1,1-dimétliyléthoxy)carbonyl]amino}-thiazol-4-yl)imidazo[1,2-*a*]pyridine-2-carboxylique sous la forme d'un solide brun.

Spectre RMN ¹H (DMSO-d6, δ en ppm) : 11,66 (s, 1H), 8,86 (s, 1H), 8,42 (s, 1H), 7,84 (s, 1H), 7,67-7,69 (m, 2H), 1,51 (s, 9H).

Spectre de masse (APCI): m/z= 361 [M+H]⁺.

### Intermédiaire 15 : Acide 6-(1H-pyrrol-3-yl)imidazo[1,2-a]pyridine-2-carboxylique

### 15.1 6-[1-(Triisopropylsilyl)-1H-pyrrol-3-yl]imidazo[1,2-a]pyridine-2-carboxylate d'éthyle

100 mg de 6-iodo-imidazo[1,2-*a*]pyridine-2-carboxylate d'éthyle, 135 mg d'acide 1-(triisopropylsilyl)-pyrrole-3-boronique et 18 mg de tétrakis(triphénylphosphine)palladium(0) sont dégazés sous vide puis mis en suspension, sous argon, dans un mélange dégazé de 1,5 mL de 1,2-diméthoxyéthane, 1,5 mL d'éthanol et 316 µL de solution aqueuse 2N de carbonate de sodium. Le mélange réactionnel est chauffé au reflux pendant 4 heures, puis refroidi, dilué et agité avec un mélange de 5 mL de solution aqueuse demi-saturée de bicarbonate de sodium et 5 mL de dichlorométhane. La phase organique est séchée sur sulfate de sodium, filtrée et concentrée à sec sous pression réduite. Le résidu est chromatographié sur silice en éluant par un mélange d'acétate d'éthyle et d'hexane (50/50). Les fractions contenant le produit attendu sont réunies et concentrées à sec sous pression réduite pour donner 121 mg de 6-[1-(triisopropylsilyl)-1*H*-pyrrol-3-yl]imidazo[1,2-*a*]pyridine-2-carboxylate d'éthyle.

Spectre RMN ¹H (DMSO-d6, δ en ppm) : 8,76 (s, 1H), 8,42 (s, 1H), 7,70 (dd, J = 1,9, 9,7 1H), 7,59 (d, J = 9,7 1H), 7,37 (s large, 1H), 6,94 (m, 1H), 6,63 (m, 1H), 4,33 (q, J = 6,9, 2H), 1,61-1,50 (m, 3H), 1,33 (t, J = 6,9, 3H), 1,10-1,03 (m, 18H).

Spectre de masse (APCI): m/z= 412 [M+H]⁺.

### 15.2 : Chlorhydrate (1:1) de l'acide 6-(1H-pyrrol-3-yl)imidazo[1,2-a]pyridine-2-carboxylique

292 mg de 6-[1-(triisopropylsilyl)-1*H*-pyrrol-3-yl]imidazo[1,2-*a*]pyridine-2-carboxylate d'éthyle sont saponifiés dans des conditions analogues à celles décrites pour la préparation de l'intermédiaire 11 (étape 11.3) pour donner 140 mg de chlorhydrate (1:1) de l'acide 6-(1*H*-pyrrol-3-yl)imidazo[1,2-*a*]pyridine-2-carboxylique sous la forme d'un solide blanc.

Spectre RMN ¹H (DMSO-d6, δ en ppm) :11,07 (s large, 1H), 8,73 (s, 1H), 8,39 (s, 1H), 7,69 (dd, J = 1,3, 9,5, 1H), 7,59 (d, J = 9,5, 1H), 7,31 (s, 1H), 6,86 (s, 1H), 6,46 (s, 1H).

Spectre de masse (APCI): m/z= 228 [M+H]⁺.

### Intermédiaire 16 : Acide 6-(1H-pyrazol-3-yl)imidazo[1,2-a]pyridine-2-carboxylique

### 16.1 : 6-(1H-pyrazol-3-yl)imidazo[1,2-a]pyridine-2-carboxylate d'éthyle

Ce produit est préparé dans les conditions analogues à celles décrites pour la préparation de l'intermédiaire 15 (étape 15.1) en remplaçant l'acide 1-(triisopropylsilyl)-pyrrole-3-boronique par l'acide-pyrrazole-3-boronique.

Spectre RMN ¹H (MeOD-d4, δ en ppm) : 8,89 (t, J = 1,2, 2,4, 1H), 8,45 (d, J = 0,6, 1H), 7,89 (d, J = 9,0, 1H), 7,76 (s large, 1H), 7,67 (d, J = 9,5, 1H), 6,77 (d, J = 2,4, 1H), 4,42 (q, J = 7,1, 2H), 1,43 (t, J = 7,1, 3H).

Spectre de masse (APCI): m/z= 257 [M+H]⁺.

### 16.2 : Acide 6-(1H-pyrazol-3-yl)imidazo[1,2-a]pyridine-2-carboxylique

128 mg de 6-(1*H*-pyrazol-3-yl)imidazo[1,2-*a*]pyridine-2-carboxylate d'éthyle sont saponifiés dans des conditions analogues à celles décrites pour la préparation de l'intermédiaire 11 (étape 11.3) pour donner 113 mg d'acide 6-(1*H-*pyrazol-3-yl)imidazo[1,2-*a*]pyridine-2-carboxylique.

Spectre RMN ¹H (DMSO-d6, δ en ppm) : 13,50-12,50 (s large, 1H), 9,03 (s, 1H), 8,40 (s, 1H), 7,83-7,80 (m, 2H), 7,63 (d, J = 9,4, 1H), 6,74 (s, 1H).

### Intermédiaire 17 : Acide 6-(1H-pyrazol-4-yl)imidazo[1,2-a]pyridine-2-carboxylique

### 17.1 : 6-(1H-pyrazol-4-yl)imidazo[1,2-a]pyridine-2-carboxylate d'éthyle

Ce produit est préparé dans les conditions analogues à celles décrites pour la préparation de l'intermédiaire 15 (étape 15.1) en remplaçant l'acide 1-(triisopropylsilyl)-pyrrole-3-boronique par l'acide-pyrrazole-4-boronique et en chauffant à 90 °C sous microondes pendant 37 minutes.

Spectre RMN ¹H (DMSO-d6, δ en ppm) : 13,10 (s large, 1H), 8,83 (s, 1H), 8,43 (s, 1H), 8,25 (s large, 1H), 7,94 (s large, 1H), 7,69-7,61 (m, 2H), 4,31 (q, J = 7,1, 2H), 1,32 (t, J = 7,1, 3H).

Spectre de masse (APCI): m/z= 257 [M+H]⁺.

### 17.2 : Acide 6-(1H-pyrazol-4-yl)imidazo[1,2-a]pyridine-2-carboxylique

128 mg de 6-(1*H-*pyrazol-4-yl)imidazo[1,2-*a*]pyridine-2-carboxylate d'éthyle sont saponifiés dans des conditions analogues à celles décrites pour la préparation de l'intermédiaire 11 (étape 11.3) pour donner 60 mg d'acide 6-(1*H*-pyrazol-4-yl)imidazo[1,2-*a*]pyridine-2-carboxylique.

Spectre RMN ¹H (DMSO-d6, δ en ppm) : 14,0-12,0 (s large, 1H), 8,84 (s, 1H), 8,36 (s, 1H), 8,10 (s, 2H), 7,64 (s, 2H).

### Intermédiaire 18 : Acide 6-(furan-2-yl)imidazo[1,2-a]pyridine-2-carboxylique

### 18.1 : 6-(Furan-2-yl)imidazo[1,2-a]pyridine-2-carboxylate d'éthyle

Ce produit est préparé dans les conditions analogues à celles décrites pour la préparation de l'intermédiaire 15 (étape 15.1) en remplaçant l'acide 1-(triisopropylsilyl)-pyrrole-3-boronique par l'acide-furanne-2-boronique.

Spectre RMN ¹H (DMSO-d6, δ en ppm) : 8,78 (s, 1H), 8,44 (s, 1H), 7,72 (dd, J = 1,8, 9,6, 1H), 7,63-7,60 (m, 2H), 6,89 (d, J = 3,4, 1H), 6,57 (dd, J = 1,8, 3,4, 1H), 4,42 (q, J = 7,1, 2H), 1,42 (t, J = 7,1, 3H).

Spectre de masse (APCI): m/z= 257 [M+H]⁺.

### 18.2 : Acide 6-(furan-2-yl)imidazo[1,2-a]pyridine-2-carboxylique

384 mg de 6-(furan-2-yl)imidazo[1,2-*a*]pyridine-2-carboxylate d'éthyle sont saponifiés dans des conditions analogues à celles décrites pour la préparation de l'intermédiaire 11 (étape 11.3) pour donner 256 mg d'acide 6-(furan-2-yl)imidazo[1,2-*a*]pyridine-2-carboxylique.

Spectre RMN ¹H (DMSO-d6, δ en ppm) : 8,86 (s, 1H), 8,38 (s, 1H), 7,80 (dd, J = 1,7, 9,5, 1H), 7,67-7,64 (m, 2H), 6,90 (d, J = 3,4, 1H), 6,60 (dd, J = 1,8, 3,4, 1H).

### Intermédiaire 19 : Acide 6-(furan-3-yl)imidazo[1,2-a]pyridine-2-carboxylique

### 19.1 : 6-(Furan-3-yl)imidazo[1,2-a]pyridine-2-carboxylate d'éthyle

Ce produit est préparé dans les conditions analogues à celles décrites pour la préparation de l'intermédiaire 15 (étape 15.1) en remplaçant l'acide 1-(triisopropylsilyl)-pyrrole-3-boronique par l'acide-furanne-3-boronique.

Spectre RMN ¹H (DMSO-d6, δ en ppm) : 8,86 (s, 1H), 8,45 (s, 1H), 8,28 (s, 1H), 7,82 (s, 1H), 7,66 (s, 2H), 6,95 (s, 1H), 4,31 (q, J = 7,1, 2H), 1,33 (t, J = 7,1, 3H).

Spectre de masse (APCI): m/z= 257 [M+H]⁺.

### 19.2 : Acide 6-(furan-3-yl)imidazo[1,2-a]pyridine-2-carboxylique

384 mg de 6-(furan-3-yl)imidazo[1,2-*a*]pyridine-2-carboxylate d'éthyle sont saponifiés dans des conditions analogues à celles décrites pour la préparation de l'intermédiaire 11 (étape 11.3) pour donner 287 mg d'acide 6-(furan-3-yl)imidazo[1,2-*a*]pyridine-2-carboxylique.

Spectre RMN ¹H (DMSO-d6, δ en ppm) : 8,86 (s, 1H), 8,38 (s, 1H), 8,27 (s, 1H), 7,81 (s, 1H), 7,64 (s, 2H), 6,95 (s, 1H).

Spectre de masse (APCI): m/z= 229 [M+H]⁺.

### Intermédiaire 20 : Acide 6-[5-(hydroxyméthyl)furan-2-yl]imidazo[1,2-a]pyridine-2-carboxylique

20.1 : 6-(5-Formylfuran-2-yl)imidazo[1,2-*a*]pyridine-2-carboxylate d'éthyle 2 g de 6-iodo-imidazo[1,2-*a*]pyridine-2-carboxylate d'éthyle, 1,42 g d'acide 5-formyl-furanne-2-boronique et 231 mg de [1,1'-bis(diphenylphosphino)ferrocène]dichloropalladium sont dégazés sous vide puis mis en suspension, sous argon, dans un mélange dégazé de 30 mL de dioxanne et 9,4 mL d'une solution aqueuse 2N de carbonate de sodium. Le mélange réactionnel est chauffé 5 heures à 90 °C, puis agité 16 heures à 20 °C et concentré à sec. Le résidu est chromatographié sur silice en éluant par un mélange d'acétate d'éthyle et d'hexane (90/10), par de l'acétate d'éthyle puis par un mélange (99/1) d'acétate d'éthyle et de méthanol. Les fractions contenant le produit attendu sont réunies et concentrées à sec sous pression réduite pour donner 884 mg de 6-(5-formylfuran-2-yl)imidazo[1,2-*a*]pyridine-2-carboxylate d'éthyle.

Spectre RMN ¹H (DMSO-d6, δ en ppm) : 9,64 (s, 1H), 9,20 (s, 1H), 8,66 (s, 1H), 7,86-7,74 (m, 2H), 7,72 (d, J = 3,8, 1H), 7,37 (d, J = 3,8, 1H), 4,33 (q, J = 7,0, 2H), 1,33 (t, J = 7,1, 3H).

Spectre de masse (APCI): m/z= 285 [M+H]⁺.

### 20.2 : 6-[5-(Hydroxyméthyl)furan-2-yl]imidazo[1,2-a]pyridine-2-carboxylate d'éthyle

A une suspension de 770 mg de 6-(5-formylfuran-2-yl)imidazo[1,2-*a*]pyridine-2-carboxylate d'éthyle dans 15 mL d'éthanol, on ajoute 123 mg de borohydrure de sodium. Le mélange réactionnel est agité à 25 °C pendant 90 minutes puis dilué et agité avec 10 mL de dichlorométhane et 3 mL d'une solution aqueuse demi-saturée de carbonate de sodium. La phase organique est séparée, séchée sur sulfate de magnésium, filtrée et concentrée sous pression réduite. Le résidu est chromatographié sur silice en éluant par un mélange de dichlorométhane et de méthanol (98/2). Les fractions contenant le produit attendu sont réunies et concentrées à sec sous pression réduite. Le solide obtenu est trituré dans 5 mL de dichlorométhane, filtré et séché pour donner 403 mg de 6-(5-formylfuran-2-yl)imidazo[1,2-*a*]pyridine-2-carboxylate d'éthyle sous la forme d'un solide blanc.

Spectre RMN ¹H (DMSO-d6, δ en ppm) : 8,89 (s, 1H), 8,60 (s, 1H), 7,70 (m, 2H), 6,98 (d, J = 3,3, 1H), 6,45 (d, J = 3,3, 1H), 5,30 (t, J = 5,3, 1H), 4,47 (d, J = 5,6, 2H), 4,32 (q; J = 7,1, 2H), 1,32 (t, J = 7,1, 3H).

Spectre de masse (APCI): m/z= 287 [M+H]⁺.

### 20.3 : Acide 6-[5-(hydroxyméthyl)furan-2-yl]imidazo[1,2-a]pyridine-2-carboxylique

400 mg de 6-[5-(hydroxyméthyl)furan-2-yl]imidazo[1,2-*a*]pyridine-2-carboxylate d'éthyle sont saponifiés dans des conditions analogues à celles décrites pour la préparation de l'intermédiaire 11 (étape 11.3) pour donner 346 mg d'acide 6-[5-(hydroxyméthyl)furan-2-yl]imidazo[1,2-*a*]pyridine-2-carboxylique sous la forme d'un solide blanc.
Spectre RMN ¹H (DMSO-d6, δ en ppm) : 9,06 (s, 1H), 8,73 (s, 1H), 8,03 (d, J = 9,5, 1H), 7,82 (d, J = 9,5, 1H), 7,09 (d, J = 3,3, 1H), 6,49 (d, J = 3,2, 1H), 4,49 (s, 2H).
Spectre de masse (APCI): m/z= 259 [M+H]⁺.

### Intermédiaire 21 : Acide 6-(thiophèn-3-yl)imidazo[1,2-a]pyridine-2-carboxylique

### 21.1 : 6-(Thiophén-3-yl)imidazo[1,2-a]pyridine-2-carboxylate d'éthyle

Ce produit est préparé dans les conditions analogues à celles décrites pour la préparation de l'intermédiaire 15 (étape 15.1) en remplaçant l'acide 1-(triisopropylsilyl)-pyrrole-3-boronique par l'acide-thiophène-3-boronique (catalyseur : dichloro-bis(triphénylphosphine)-palladium.
Spectre RMN ¹H (DMSO-d6, δ en ppm) : 1,34 (d, J=7,1 Hz, 3H), 4,32 (q, J=7,1 Hz, 2H), 7,56 (dd, J=5,0, 1,4 Hz, 1H), 7,68 (d, J=9,8 Hz, 1H), 7,73 (dd, J=5,0, 3,0 Hz, 1H), 7,78 (dd, J=9,8, 1,8 Hz, 1H), 7,97 (dd, J=3,0, 1,4 Hz, 1H), 8,48 (s, 1H), 8,98 (s large, 1H).
Spectre de masse (LC-MS-DAD-ELSD) : m/z 273 [M+H]⁺.

### 21.2 : Acide 6-(thiophèn-3-yl)imidazo[1,2-a]pyridine-2-carboxylique

310 mg de 6-(thiophén-3-yl)imidazo[1,2-*a*]pyridine-2-carboxylate d'éthyle sont saponifiés dans des conditions analogues à celles décrites pour la préparation de l'intermédiaire 11 (étape 11.3) pour donner 250 mg d'acide 6-(thiophèn-3-yl)imidazo[1,2-*a*]pyridine-2-carboxylique.

Spectre RMN ¹H (DMSO-d6, δ en ppm) : 7,57 (d, J=5,4 Hz, 1H), 7,66 (d, J=9,8 Hz, 1H), 7,73 (dd, J=5,4, 2,8 Hz, 1H), 7,76 (dd, J=9,8, 2,0 Hz, 1H), 7,97 (d large, J=2,0 Hz, 1H), 8,41 (s, 1H), 8,99 (s large, 1 H).

Spectre de masse (LC-MS-DAD-ELSD) : m/z 245 [M+H]⁺.

### Intermédiaire 22 : Acide 6-(oxazol-2-yl)imidazo[1,2-a]pyridine-2-carboxylique

### 22.1 : 6-(Oxazol-2-yl)imidazo[1,2-a]pyridine-2-carboxylate d'éthyle

1 g de 6-iodo-imidazo[1,2-*a*]pyridine-2-carboxylate d'éthyle, 350 mg de tétrakis-(triphénylphosphine)palladium(0) et 360 mg de chlorure e lithium sont dégazés sous vide puis mis en suspension, sous argon, dans 15 mL de dioxanne dégazé. Après ajout de 5 g de 2-(tri-n-butylstannyl)oxazole, le mélange réactionnel est chauffé à 90 °C pendant 3,5 heures, puis refroidi, dilué et agité avec un mélange de 100 mL de solution aqueuse 1M de fluorure de potassium et 200 mL d'acétate d'éthyle. La phase aqueuse est extraite par 200 mL d'acétate d'éthyle et les phases organiquee réunies sont lavées par de la saumure et séchées sur sulfate de sodium, filtrées et concentrées à sec sous pression réduite. Le résidu est chromatographié sur silice en éluant par un gradient d'acétate d'éthyle et d'hexane (de 80/20 à 100/0). Les fractions contenant le produit attendu sont réunies et concentrées à sec sous pression réduite pour donner 530 mg de 6-(oxazol-2-yl)imidazo[1,2-*a*]pyridine-2-carboxylate d'éthyle sous la forme d'une poudre jaune.

Spectre RMN ¹H (DMSO-d6, δ en ppm) : 9,30 (d, J = 0,8, 1H), 8,68 (s, 1H), 8,30 (s, 1H), 7,85 (dd, J = 1,7, 9,5, 1H), 7,79 (d, J = 9,5, 1H), 7,44 (d, J = 0,6, 1H), 4,33 (q, J = 7,0, 2H), 1,33 (t, J = 7,1, 3H).

Spectre de masse (APCI): m/z= 258 [M+H]⁺.

### 22.2 : Acide 6-(oxazol-2-yl)imidazo[1,2-a]pyridine-2-carhoxylique

512 mg de 6-(oxazol-2-yl)imidazo[1,2-a]pyridine-2-carboxylate d'éthyle sont saponifiés dans des conditions analogues à celles décrites pour la préparation de l'intermédiaire 11 (étape 11.3) pour donner 365 mg d'acide 6-(oxazol-2-yl)imidazo[1,2-a]pyridine-2-carboxylique sous la forme d'un solide blanc.

Spectre RMN ¹H (DMSO-d6, δ en ppm) : 9,41 (s, 1H), 8,73 (s, 1H), 8,34 (s, 1H), 8,05 (dd, J = 1,5, 9,5, 1H), 7,86 (d, J = 9,5, 1H), 7,48 (s, 1H).

### Intermédiaire 23 : Acide 6-(1H-1,2,4-triazol-3-yl)imidazo[1,2-a]pyridine-2-carboxylique

### 23.1 : 6-[Ethoxy(imino)méthyl]imidazo[1,2-a]pyridine-2-carboxylate d'éthyle

470 mg d'éthanethiolate de sodium sont ajoutés à une solution de 1 g de 6-cyanoimidazo[1,2-*a*]-pyridine-2-carboxylate d'éthyle (J. Med. Chem. (1998), 41(22), 4317) dans un mélange de 15 mL d'éthanol et de 10 mL de dichlorométhane refroidie à 0 °C. Le mélange réactionnel est agité 5 heures à 25 °C, filtré et le filtrat évaporé à sec. Le résidu est chromatographié sur silice en éluant par un mélange de dichlorométhane et de méthanol (98/2) pour donner 625 mg de 6-[éthoxy(imino)méthyl]imidazo[1,2-*a*]pyridine-2-carboxylate d'éthyle sous la forme d'un solide jaune pâle.

Spectre RMN ¹H (DMSO-d6, δ en ppm) : 9,17 (s, 1H), 9,04 (s, 1H), 8,64 (s, 1H), 7,84 (m, 1H), 7,68 (m, 1H), 4,33 (q, J = 7,1, 4H), 1,34 (t = 7,2, 6H).

Spectre de masse (APCI): m/z= 262 [M+M]⁺.

### 23.2 : 6-[Hydrazino(imino)méthyl]imidazo[1,2-a]pyridine-2-carboxylate d'éthyle

A une solution de 625 mg de 6-[éthoxy(imino)méthyl]imidazo[1,2-*a*]pyridine-2-carboxylate d'éthyle dans 12 mL d'éthanol, on ajoute goutte à goutte à 0-5 °C 0,2 mL d'hydrate d'hydrazine. Le mélange réactionnel est agité 2 heures puis on ajoute 73 µL d'hydrate d'hydrazine et agite encore 2 heures en laissant remonter la température à 25 °C. Le mélange réactionnel est concentré à sec sous pression réduite et le résidu séché pour donné 600 mg de 6-[hydrazino(imino)méthyl]imidazo[1,2-*a*]pyridine-2-carboxylate d'éthyle qui est utilisé sans autre purification dans la suite de la synthèse.

Spectre RMN ¹H (DMSO-d6, δ en ppm) : 8,77 (s large, 1H), 8,49 (s, 1H), 7,70 (m, 1H), 7,53 (d, J = 9,6, 1H), 5,67 (s, 2H), 5,15 (s large, 2H), 4,33 (q, J = 7,1, 2H), 1,32 (t = 7,1, 3H).

Spectre de masse (APCI): m/z= 248 [M+H]⁺.

### 23.3 : 6-(1H-1,2,4-triazol-3-yl)imidazo[1,2-a]pyridine-2-carboxylate d'éthyle

Une suspension de 580 mg de 6-[hydrazino(imino)méthyl]imidazo[1,2-*a*]pyridine-2-carboxylate d'éthyle dans 6 mL d'acide formique est chauffée 20 heures à 85 °C. Le mélange réactionnel est concentré à moins de 20 % de son volume initial et dilué par 20 mL d'eau. On ajoute à 0-5 °C du carbonate de sodium solide jusqu'à atteindre pH 8-9. Le précipité est essoré puis purifié par chromatographie sur silice en éluant par un mélange de dichlorométhane et de méthanol (98/2) pour donner 320 mg de : 6-(1*H*-1,2,4-triazol-3-yl)imidazo[1,2-*a*]pyridine-2-carboxylate d'éthyle.

Spectre RMN ¹H (DMSO-d6, δ en ppm) : 14,5-14,0 (s large, 1H), 9,25 (s, 1H), 8,69 (s, 1H), 8,63 (s large, 1H), 7,94 (dd, J = 9,5, 1,5, 1H), 7,73 (d, J = 9,5, 1H), 4,33 (q, J = 7,0, 2H), 1,33 (t = 7,0, 3H)

Spectre de masse (APCI): m/z= 258 [M+H]⁺.

### 23.4 : Acide 6-(1H-1,2,4-triazol-3-yl)imidazo[1,2-a]pyridine-2-carboxylique

320 mg de 6-(1*H*-1,2,4-triazol-3-yl)imidazo[1,2-*a*]pyridine-2-carboxylate d'éthyle sont saponifiés dans des conditions analogues à celles décrites pour la préparation de l'intermédiaire 11 (étape 11.3) pour donner 238 mg d'acide 6-(1*H*-1,2,4-triazol-3-yl)imidazo[1,2-*a*]pyridine-2-carboxylique sous la forme d'un solide blanc cassé.

Spectre RMN ¹H (DMSO-d6, δ en ppm) : 14,5-14,2 (s large, 1H), 9,26 (s, 1H), 8,66-8,62 (m, 2H), 7,91 (d, J = 9,1, 1H), 7,73 (d, J = 9,6, 1H).

### Intermédiaire 24 : Acide 6-(1H-1,2,3-triazol-4-yl)imidazo[1,2-a]pyridine-2-carboxylique

### 24.1 : 6-[(Triméthylsilyl)éthynyl]imidazo[1,2-a]pyridine-2-carboxylate d'éthyle

Un mélange de 4 g de 6-iodo-imidazo[1,2-*a*]pyridine-2-carboxylate d'éthyle, 2,63 mL d'éthynyltrimethylsilane, 888 mg de dichloro-bis(triphénylphosphine)palladium est dégazé sous vide. On ajoute 240 mg de N,N-diméthylformamide dégazé, 3,52 mL de triéthylamine. Le mélange réactionnel est dégazé sous argon puis agité à 50 °C pendant 50 heures puis refroidi, dilué par 20 mL d'eau. Le précipité est essoré et lavé par 5 mL d'eau puis chromatographié sur silice en éluant par des mélanges d'acétate d'éthyle et d'hexane (de 50/50 à 90/10). Les fractions contenant le produit attendu sont réunies et concentrées à sec sous pression réduite pour donner 3,6 g de 6-[(triméthylsilyl)éthynyl]imidazo[1,2-*a*]pyridine-2-carboxylate d'éthyle sous la forme d'un solide blanc cassé.

Spectre RMN ¹H (DMSO-d6, δ en ppm) : 8,61 (s, 1H), 8,22 (s, 1H), 7,36 (d, J = 9,5, 1H), 7,07 (dd, J = 9,5, 1,7, 1H), 4,07 (q, J = 7,1, 2H), 1,08 (t, J = 7,1, 3H), 0,01 (s, 9H).

Spectre de masse (APCI): m/z= 287 [M+H]⁺.

### 24.2 : 6-Ethynylimidazo[1,2-a]pyridine-2-carboxylate d'éthyle

A une solution de 500 mg de 6-[(triméthylsilyl)éthynyl]imidazo[1,2-*a*]pyridine-2-carboxylate d'éthyle dans 10 mL de tétrahydrofuranne anhydre, refroidie à 0 °C on ajoute gouute à goutte 1,58 mL dune solution 1M de fluorure de tétrabutylammonium dans le tétrahydrofuranne. Le mélange réactionnel est agité pendant 30 minutes puis on ajoute 5 mL d'eau et extrait 3 fois par 20 mL de dichlorométhane. Le produit est purifié par chromatographie sur silice en éluant par des mélanges d'acétate d'éthyle et d'hexane (de 1/3 à 1/1). Les fractions contenant le produit attendu sont réunies et concentrées à sec sous pression réduite pour donner 280 mg de 6-éthynylimidazo[1,2-a]pyridine-2-carboxylate d'éthyle sous la forme d'un solide jaune.

Spectre RMN ¹H (DMSO-d6, δ en ppm) : 8,86 (d, J = 1,0, 1H), 8,50 (d, J = 0,6, 1H), 7,63 (d, J = 9,4, 1H), 7,37 (d, J = 1,7, 9,4, 1H), 4,32 (m, 3H), 1,32 (t, J = 7,1 Hz, 3H).

Spectre de masse (APCI): m/z= 215 [M+H]⁺.

### 24.3 : 6-(1H-1,2,3-triazol-4-yl)imidazo[1,2-a]pyridine-2-carboxylate d'éthyle

A une solution de 220 mg de 6-éthynylimidazo[1,2-*a*]pyridine-2-carboxylate d'éthyle et 0,21 mL d'azidotriméthylsilane dans 4 mL d'un mélange (9/1) de N,N-diméthylformamide et de méthanol on ajoute 9,8 mg d'iodure cuivreux. Le mélange réactionnel est agité 2 heures à 100 °C puis refroidi et dilué par 4 mL de dichlorométhane puis filtré sur alumine et concentré à sec. Le résidu est chromatographié sur silice en éluant par un mélange de dichlorométhane et d'éthanol (97/3). Les fractions contenant le produit attendu sont réunies et concentrées à sec sous pression réduite pour donner 125 mg de : 6-(1*H*-1,2,3-triazol-4-yl)imidazo[1,2-*a*]pyridine-2-carboxylate d'éthyle sous la forme d'un solide blanc cassé.

Spectre RMN ¹H (DMSO-d6, δ en ppm) : 15,5-15,0 (s large, 1H), 9,14 (dd, J = 1,1, 1,5, 1H), 8,60 (d, J = 0,5, 1H), 8,40 (s large, 1H), 7,82 (dd, J = 1,7, 9,5, 1H), 7,75 (d, J = 9,5, 1H), 4,33 (q, J = 7,1, 2H), 1,33 (t, J = 7,1, 3H).

Spectre de masse (APCI): m/z= 258 [M+H]⁺.

### 24.4 : Acide 6-(1H-1,2,3-triazol-4-yl)imidazo[1,2-a]pyridine-2-carboxylique

125 mg de 6-(1*H*-1,2,3-triazol-3-yl)imidazo[1,2-*a*]pyridine-2-carboxylate d'éthyle sont saponifiés dans des conditions analogues à celles décrites pour la préparation de l'intermédiaire 11 (étape 11.3) pour donner 72 mg d'acide 6-(1*H*-1,2,3-triazol-3-yl)midazo[1,2-*a*]pyridine-2-carboxylique sous la forme d'un solide blanc.

Spectre RMN ¹H (DMSO-d6, δ en ppm) : 16,0-15,0 (s large, 1H), 9,23 (s, 1H), 8,62 (s, 1H), 8,46 (s large, 1H), 7,96 (dd, J = 1,4, 9,5, 1H), 7,80 (d, J = 9,5, 1H).

### Intermédiaire 25 : Bromhydrate (1:1) de 6-(4,4,5,5-tétraméthyl-1,3,2-dioxaborolan-2-yl)imidazo[1,2-a]pyridine-2-carboxylate d'éthyle

A une solution de 4 g de 2-amino-5-(4,4,5,5-tétraméthyl-1,3,2-dioxaborolan-2-yl)pyridine dans 40 mL de 1,2-diméthoxyéthane on ajoute 4,26 g de 3-bromo-2-oxo-propionate d'éthyle. Le mélange réactionnel est agité 40 heures à 20 °C. Le précipité est essoré, lavé par un peu de 1,2-diméthoxyéthane et du pentane puis repris dans 50 mL d'éthanol et chauffé au reflux pendant 1 heure. Le mélange réactionnel est concentré à sec sous pression réduite. L'huile obtenue est redissoute dans de l'éther éthylique et la solution concentrée sous pression réduite. Le solide est essoré et lavé par un peu d'éther éthylique pour donner 3,78 g de bromhydrate (1:1) de 6-(4,4,5,5-tétraméthyl-1,3,2-dioxaborolan-2-yl)-imidazo-[1,2-*a*]pyridine-2-carboxylate d'éthyle sous la forme d'un solide blanc.

Spectre RMN ¹H (DMSO-d6, δ en ppm) : 1,27 - 1,38 (m, 15H), 4,36 (q, J=7,3 Hz, 2H), 7,59 (d, J=9,3 Hz, 1H), 7,67 (d, J=9,3 Hz, 1H), 8,68 (s, 1H), 8,97 (s, 1H).

Spectre de masse (IE) : m/z 316 [M]⁺, 244 [M- CO₂Et+H]⁺.

### Intermédiaire 26 : Acide 2-éthoxycarbonyl-imidazo[1,2-a]pyridine-6-boronique

A une solution de 2,5 g de 2-amino-5-(4,4,5,5-tétraméthyl-1,3,2-dioxaborolan-2-yl)pyridine dans 50 mL de 1,2-diméthoxyéthane on ajoute 2,14 mL de 3-bromo-2-oxo-propionate d'éthyle. Le mélange réactionnel est agité 3,5 heures à 25 °C puis on ajoute 50 mL d'éthanol et chauffe au reflux pendant 16 heures.Le mélange réactionnel est refroidi et concentré à sec. Le résidu est mis en suspension dans 100 mL d'eau à 0 °C et traité en agitant vigoureusement par du carbonate de sodium solide jusqu'à atteindre un pH de 8-9. Le précipité est essoré et lavé par 100 mL d'eau à 0 °C puis dissout dans 150 mL de méthanol. La solution est séchée sur sulfate de mlagnésium, filtrée, concentrée et séchée sous vide pour donner 2,36 g d'acide 2-éthoxycarbonyl-imidazo[1,2-*a*]pyridine-6-boronique sous la forme d'un solide crème.

Spectre RMN ¹H (DMSO-d6, δ en ppm) : 8,82 (d, J = 0,9, 1H), 8,58 (s, 1H), 8,35 (s, 2H) 7,61 (m, 2H), 4,33 (m, 2H), 1,32 (m, 3H)

Spectre de masse (APCI): m/z= 235 [M+H]⁺.

Les tableaux qui suivent illustrent les structures chimiques (tableau 1), les caractéristiques spectroscopiques et méthodes de synthèse (tableau 2) de quelques exemples de composés selon l'invention.

Dans ce tableau,
- le rapport entre parenthèses est le rapport (acide:base), «HCl » représente un composé sous forme chlorhydrate, « TFA » représente un composé sous forme trifluoroacétate et le rapport indiqué entre parenthèse est le rapport (acide : base), le signe « - » signifie que le composé se présente sous forme base ;
- « -CH₃ » signifie méthyle,

**Tableau 1**

| | | | | | | | |
|---|---|---|---|---|---|---|---|
| 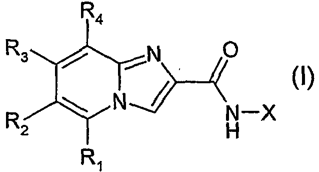 | | | | | | | |

| **Ex** | **R₁** | **R₂** | **R₃** | **R₄** | **X** | **sel** | **Méthode** |
|---|---|---|---|---|---|---|---|
| **1** | H | 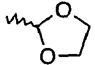 | H | H | Ph | - | ex 1 |
| **2** | H | 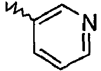 | H | H | Ph | - | ex 2 |
| **3** | H | 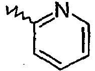 | H | H | Ph | - | ex 3 |
| **4** | H | 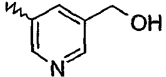 | H | H | Ph | - | ex 4 |
| **5** | H | 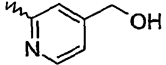 | H | H | Ph | HCl (1:1) | ex 5 |
| **6** | H | 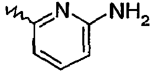 | H | H | Ph | HCl (1 :1) | ex 6 |
| **7** | H | 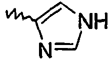 | H | H | Ph | HCl (1 :1) | ex 7 |
| **8** | H | 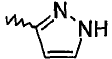 | H | H | Ph | - | ex 8 |
| **9** | H | 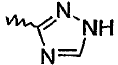 | H | H | Ph | - | ex 9 |
| **10** | H | 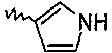 | H | H | Ph | - | ex 10 |
| **11** | H | 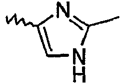 | H | H | Ph | - | ex 11 |
| **12** | H | 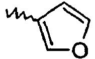 | H | H | Ph | - | ex 12 |
| **13** | H | 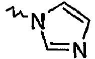 | H | H | Ph | TFA (1 :1) | ex 13 |
| **14** | H | 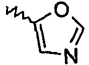 | H | H | Ph | - | ex 14 |
| **15** | H | 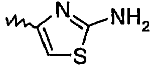 | H | H | Ph | - | ex 15 |
| **16** | H | 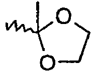 | H | H | Ph | - | ex 16 |
| **17** | H | 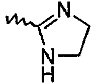 | H | H | Ph | HCl (1:1) ; HCl (2:1) | ex 17 |
| **18** | H | 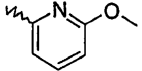 | H | H | Ph | - | ex 18 |
| **19** | -CH₃ | 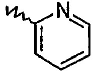 | H | H | Ph | - | ex 19 |
| **20** | H | 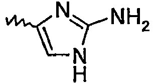 | H | H | Ph | TFA (1 :1) | ex 20 |
| **21** | H | 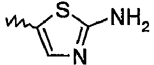 | H | H | Ph | - | ex 21 |
| **22** | H | 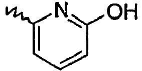 | H | H | Ph | - | ex 22 |
| **23** | H | 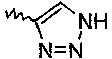 | H | H | Ph | - | ex 23 |
| **24** | H | 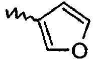 | H | H | 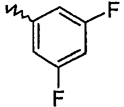 | - | ex 24 |
| **25** | H | 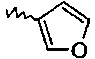 | H | H | 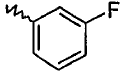 | - | ex 25 |
| **26** | H | 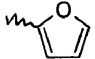 | H | H | Ph | - | Analogue ex 3 |
| **27** | H | 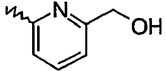 | H | H | Ph | HCl (1:1) | Analogue ex 5 |
| **28** | H | 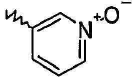 | H | H | Ph | HCl (1:1) | Analogue ex 5 |
| **29** | H | 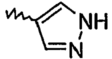 | H | H | Ph | - | Analogue ex 8 |
| **30** | H | 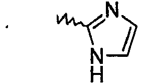 | H | H | Ph | TFA (1 :1) | Analogue ex 9 |
| **31** | H | 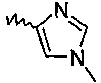 | H | H | Ph | - | Analogue ex 7 |
| **32** | H | 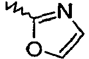 | H | H | Ph | - | Analogue ex 3 |
| **33** | H | 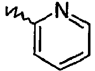 | H | H | 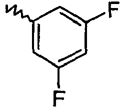 | - | Analogue ex 3 |
| **34** | H | 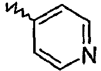 | H | H | Ph | - | Analogue ex. 2 |
| **35** | H | 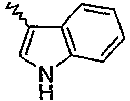 | H | H | Ph | - | Analogue ex 8 |
| **36** | H | 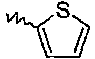 | H | H | Ph | - | Analogue ex 7 |
| **37** | H | 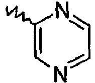 | H | H | Ph | - | Analogue ex 7 |
| **38** | H | 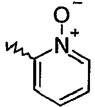 | H | H | Ph | - | Analogue ex 18 |
| **39** | H | 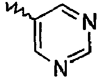 | H | H | Ph | - | Analogue interm. 20 et 1 |
| **40** | H | 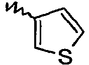 | H | H | Ph | - | Analogue ex 8 |
| **41** | H | 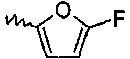 | H | H | Ph | - | Analogue interm. 11 et 1 |
| **42** | H | 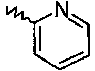 | H | H | 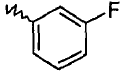 | - | Analogue ex 25 |
| **43** | H | 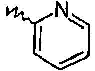 | H | H | 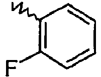 | - | Analogue ex 25 |
| **44** | H | 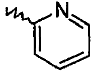 | H | H | 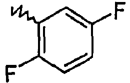 | - | Analogue ex 25 |
| **45** | H | 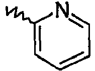 | H | H | 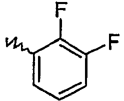 | - | Analogue ex 25 |
| **46** | H | 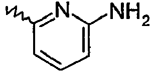 | H | H | 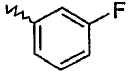 | - | Analogue ex 25 |
| **47** | H | 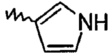 | H | H | 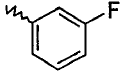 | - | Analogue ex 25 |
| **48** | H | 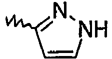 | H | H | 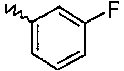 | - | Analogue ex 25 |
| **49** | H | 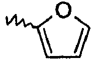 | H | H | 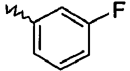 | - | Analogue ex 25 |
| **50** | H | 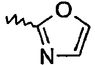 | H | H | 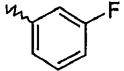 | - | Analogue ex 25 |
| **51** | H | 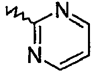 | H | H | Ph | - | Analogue ex 18 |
| **52** | H | 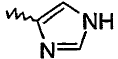 | H | H | 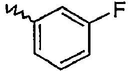 | - | Analogue ex 25 |
| **53** | H | 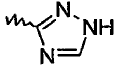 | H | H | 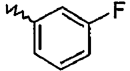 | - | Analogue ex 25 |
| **54** | H | 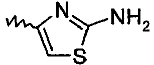 | H | H | 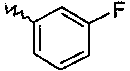 | - | Analogue ex 25 |
| **55** | H | 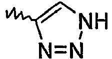 | H | H | 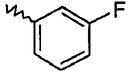 | - | Analogue ex 25 |
| **56** | H | 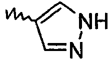 | H | H | 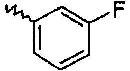 | - | Analogue ex 25 |
| **57** | H | 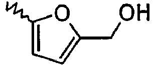 | H | H | 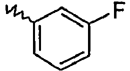 | - | Analogue ex 25 |
| **58** | H | 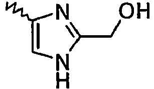 | H | H | Ph | - | Analogue ex 4 |
| **59** | H | 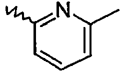 | H | H | 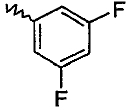 | - | Analogue ex 25 |
| **60** | H | 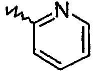 | H | H | 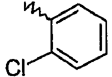 | - | Analogue ex 25 |
| **61** | H | 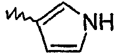 | H | H | 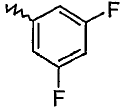 | - | Analogue ex 25 |
| **62** | H | 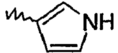 | H | H | 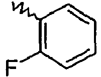 | - | Analogue ex 25 |
| **63** | H | 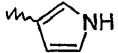 | H | H | 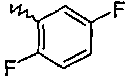 | - | Analogue ex 25 |
| **64** | H | 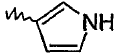 | H | H | 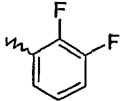 | - | Analogue ex 25 |
| **65** | H | 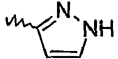 | H | H | 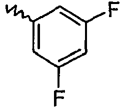 | - | Analogue ex 25 |
| **66** | H | 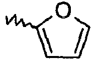 | H | H | 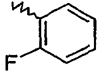 | - | Analogue ex 25 |
| **67** | H | 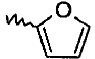 | H | H | 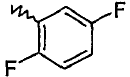 | - | Analogue ex 25 |
| **68** | H | 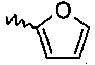 | H | H | 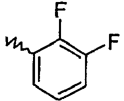 | - | Analogue ex 25 |
| **69** | H | 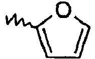 | H | H | 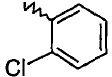 | - | Analogue ex 25 |
| **70** | H | 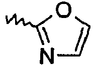 | H | H | 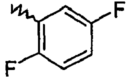 | - | Analogue ex 25 |
| **71** | H | 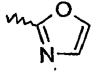 | H | H | 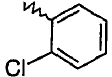 | - | Analogue ex 25 |
| **72** | H | 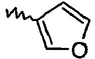 | H | H | 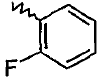 | - | Analogue ex 25 |
| **73** | H | 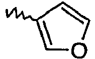 | H | H | 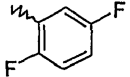 | - | Analogue ex 25 |
| **74** | H | 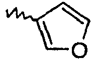 | H | H | 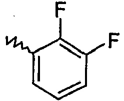 | - | Analogue ex 25 |
| **75** | H | 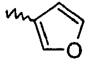 | H | H | 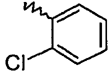 | - | Analogue ex 25 |
| **76** | H | 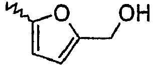 | H | H | 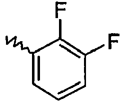 | - | Analogue ex 25 |
| **77** | H | 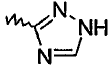 | H | H | 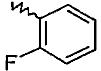 | - | Analogue ex 25 |
| **78** | H | 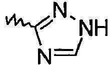 | H | H | 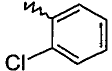 | - | Analogue ex 25 |
| **79** | H | 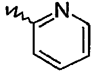 | H | H | 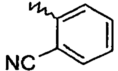 | - | Analogue ex 25 |
| **80** | H | 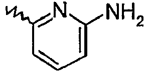 | H | H | 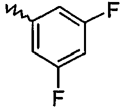 | HCl (2:1) | Analogue ex 25 |
| **81** | H | 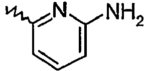 | H | H | 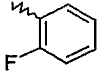 | HCl (2:1) | Analogue ex 25 |
| **82** | H | 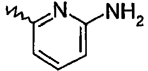 | H | H | 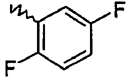 | HCl (2:1) | Analogue ex 25 |
| **83** | H | 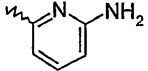 | H | H | 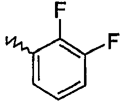 | HCl (2:1) | Analogue ex 25 |
| **84** | H | 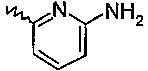 | H | H | 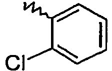 | HCl (2:1) | Analogue ex 25 |
| **85** | H | 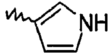 | H | H | 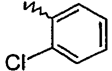 | - | Analogue ex 25 |
| **86** | H | 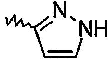 | H | H | 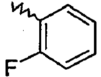 | - | Analogue ex 25 |
| **87** | H | 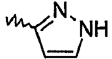 | H | H | 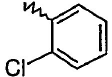 | - | Analogue ex 25 |
| **88** | H | 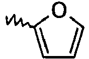 | H | H | 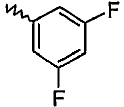 | - | Analogue ex 25 |
| **89** | H | 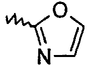 | H | H | 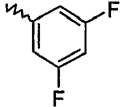 | - | Analogue ex 25 |
| **90** | H | 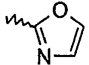 | H | H | 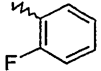 | - | Analogue ex 25 |
| **91** | H | 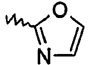 | H | H | 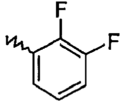 | - | Analogue ex 25 |
| **92** | H | 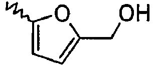 | H | H | 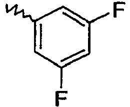 | - | Analogue ex 25 |
| **93** | H | 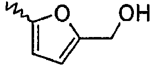 | H | H | 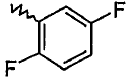 | - | Analogue ex 25 |
| **94** | H | 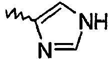 | H | H | 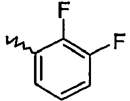 | - | Analogue ex 25 |
| **95** | H | 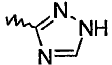 | H | H | 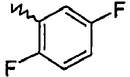 | - | Analogue ex 25 |
| **96** | H | 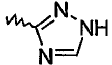 | H | H | 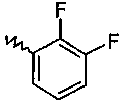 | - | Analogue ex 25 |
| **97** | H | 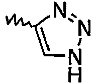 | H | H | 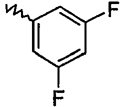 | - | Analogue ex 25 |
| **98** | H | 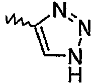 | H | H | 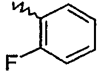 | - | Analogue ex 25 |
| **99** | H | 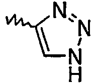 | H | H | 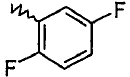 | - | Analogue ex 25 |
| **100** | H | 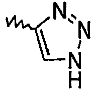 | H | H | 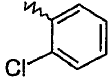 | - | Analogue ex 25 |

**Tableau 2**

| **Ex** | **Caractérisations** |
|---|---|
| **1** | Spectre RMN ¹H (DMSO-d6, δ en ppm) : de 3,97 à 4,14 (m, 4H), 5,87 (s, 1H), 7,10 (t, J = 7,5 Hz, 1H), 7,33 (t, J = 7,5 Hz, 2H), 7,41 (dd, J = 2,0 et 9,5 Hz, 1H), 7,69 (d, J = 9,5 Hz, 1H), 7,90 (d, J = 7,5 Hz, 2H), 8,54 (s, 1H), 8,77 (s large, 1H), 10,3 (s, 1H). |
| | Spectre de masse (LC-MS-DAD-ELSD) : m/z 310 [M+H]⁺. |
| **2** | Spectre RMN ¹H (DMSO-d6, δ en ppm) : 7,10 (t, J = 8,0 Hz, 1H), 7,35 (t large, J = 8,0 Hz, 2H), 7,56 (dd large, J = 5,0 et 8,0 Hz, 1H), de 7,76 à 7,83 (m, 2H), 7,91 (d large, J = 8,0 Hz, 2H), 8,16 (td, J = 1,5 et 8,0 Hz, 1H), 8,52 (s, 1H), 8,64 (dd, J = 1,5 et 5,0 Hz, 1H), 8,98 (m, 1H), 9,09 (t, J = 1,5 Hz, 1H), 10,3 (s, 1H). |
| | Spectre de masse (LC-MS-DAD-ELSD) : m/z 315 [M+H]⁺. |
| **3** | Spectre RMN ¹H (DMSO-d6, δ en ppm) : 7,10 (t, J = 7,5 Hz, 1H), 7,36 (t, J = 7,5 Hz, 2H), 7,43 (dd, J = 5,0 et 8,0 Hz, 1H), 7,78 (d, J = 9,5 Hz, 1H), 7,91 (d, J = 7,5 Hz, 2H), 7,97 (dt, J = 2,0 et 8,0 Hz, 1H), 8,03 (d, J = 9,5 Hz, 1H), 8,12 (dd, J = 2,0 et 8,0 Hz, 1H), 8,62 (s, 1H), 8,72 (d large, J = 5,0 Hz, 1H), 9,43 (s, 1H), 10,3 (s, 1H). |
| | Spectre de masse (LC-MS-DAD-ELSD) : m/z 315 [M+H]⁺. |
| **4** | Spectre RMN ¹H (DMSO-d6, δ en ppm) : 4,64 (d, J = 5,5 Hz, 2H), 5,41 (t, J = 5,5 Hz, 1H), 7,10 (t, J = 7,5 Hz, 1H), 7,35 (t, J = 7,5 Hz, 2H), 7,79 (s, 2H), 7,90 (d, J = 7,5 Hz, 2H), 8,08 (s large, 1H), 8,53 (s, 1H), 8,59 (s large, 1H), 8,84 (s large, 1H), 9,10 (s large, 1H), 10,25 (s, 1H). |
| | Spectre de masse (LC-MS-DAD-ELSD) : m/z 345 [M+H]⁺; m/z 389 [M+HCO₂]⁻. |
| **5** | Spectre RMN ¹H (DMSO-d6, δ en ppm) : 4,68 (s, 2H), 7,12 (t, J = 8,0 Hz, 2H), 7,38 (t, J = 8,0 Hz, 2H), 7,42 (d large, J = 5,0 Hz, 1H), 7,82 (d, J = 9,5 Hz, 1H), 7,89 (d, J = 8,0 Hz, 2H), 8,00 (s large, 1H), 8,21 (d large, J = 9,5 Hz, 1H), 8,67 (d, J = 5,0 Hz, 1H), 8,72 (s, 1H), 9,51 (s large, 1H), 10,45 (s large, 1H). |
| | Spectre de masse (LC-MS-DAD-ELSD) : m/z 345 [M+H]⁺. |
| **6** | Spectre RMN ¹H (DMSO-d6, δ en ppm) : 6,89 (d large, J=7,9 Hz, 1H), 7,11 (tt, J=7,6, 1,2 Hz, 1H), 7,23 (dd, J=7,9, 1,1 Hz, 1H), 7,32 - 7,41 (m, 2H), 7,62 (m très étalé, 3H), 7,84 (d, J=9,6 Hz, 1H), 7,81 - 7,97 (m, 4H), 8,67 (s, 1H), 9,29 (t, J=1,1 Hz, 1H), 10,45 (s, 1H). |
| | Spectre de masse (LC-MS-DAD-ELSD) : m/z 330 [M+H]⁺. |
| **7** | Spectre RMN ¹H (DMSO-d6, δ en ppm) : 7,10 (t, J = 7,5 Hz, 1H), 7,37 (t, J = 7,5 Hz, 2H), 7,80 (s, 1H), 7,90 (d, J = 7,5 Hz, 2H), 8,09 (s large, 1H), 8,62 (s, 1H), 8,84 (m étalé, 1H), 9,09 (s, 1H), 10,3 (s, 1H). |
| | Spectre de masse (LC-MS-DAD-ELSD) : m/z 304 [M+H]⁺. |
| **8** | Spectre RMN ¹H (DMSO-d6, δ en ppm) : 6,76 (d, J = 3,0 Hz, 1H), 7,10 (t, J = 7,5 Hz, 1H), 7,34 (t, J = 7,5 Hz, 2H), 7,71 (d, J = 9,5 Hz, 1H), de 7,81 à 7,92 (m, 4H), 8,51 (s, 1H), 9,09 (s, 1H), 10,2 (s, 1H), 13,05 (m étalé, 1H). |
| | Spectre de masse (LC-MS-DAD-ELSD) : m/z 304 [M+H]⁺, m/z 302 [M-H]⁻. |
| **9** | Spectre RMN ¹H (DMSO-d6, δ en ppm) : 7,10 (t, J = 7,5 Hz, 1H), 7,36 (t, J = 7,5 Hz, 2H), 7,78 (d, J = 9,5 Hz, 1H), 7,90 (d, J = 7,5 Hz, 2H), 7,99 (dd, J = 1,5 et 9,5 Hz, 1H), 8,61 (s large, 1H), 8,68 (s, 1H), 9,30 (s large, 1H), 10,25 (s, 1H), 14,0 (m très étalé, 1H). |
| | Spectre de masse (LC-MS-DAD-ELSD) : m/z 305 [M+H]⁺, m/z 303 [M-H]⁻. |
| **10** | Spectre RMN ¹H (DMSO-d6, δ en ppm) : 6,48 (s large, 1H), 6,89 (s large, 1H), 7,10 (t, J = 7,5 Hz, 1H), 7,30 (s large, 1H), 7,34 (t, J = 7,5 Hz, 2H), 7,60 (d, J = 9,5 Hz, 1H), 7,68 (dd, J = 1,5 et 9,5 Hz, 1H), 7,89 (d, J = 7,5 Hz, 2H), 8,40 (s, 1H), 8,76 (s large, 1H), 10,15 (s large, 1H), 11,05 (m étalé, 1H). |
| | Spectre de masse (LC-MS-DAD-ELSD) : m/z 303 [M+H]⁺, m/z 301 [M-H]⁻. |
| **11** | Spectre RMN ¹H (DMSO-d6, δ en ppm) : 2,33 (s, 3H), 7,09 (t, J = 7,5 Hz, 1H), 7,33 (t, J = 7,5 Hz, 2H), 7,54 (s large, 1H), 7,61 (d, J = 9,5 Hz, 1H), 7,72 (dd, J = 1,5 et 9,5 Hz, 1H), 7,89 (d, J = 7,5 Hz, 2H), 8,53 (s, 1H), 9,93 (s large, 1H), 10,2 (s, 1H), 11,95 (m étalé, 1H). |
| | Spectre de masse (LC-MS-DAD-ELSD) : m/z 318 [M+H]⁺. |
| **12** | Spectre RMN ¹H (DMSO-d6, δ en ppm) : 6,99 (s large, 1H), 7,10 (t, J = 7,5 Hz, 1H), 7,33 (t, J = 7,5 Hz, 2H), 7,69 (s large, 2H), 7,82 (t, J = 1,5 Hz, 1H), 7,90 (d, J = 7,5 Hz, 2H), 8,30 (s large, 1H), 8,42 (s, 1H), 8,92 (s large, 1H), 10,25 (s, 1H). |
| | Spectre de masse (LC-MS-DAD-ELSD) : m/z 304 [M+H]⁺. |
| **13** | Spectre RMN ¹H (DMSO-d6, δ en ppm) : 7,11 (t, J = 8,0 Hz, 1H), 7,36 (t, J = 8,0 Hz, 2H), 7,65 (s large, 1H), 7,79 (dd, J = 2,0 et 9,5 Hz, 1H), 7,90 (m, 3H), 8,05 (s large, 1H), 8,60 (s, 1H), 9,11 (s large, 1H), 9,20 (s large, 1H), 10,3 (s, 1H). |
| | Spectre de masse (LC-MS-DAD-ELSD) : m/z 304 [M+H]⁺. |
| **14** | Spectre RMN ¹H (DMSO-d6, δ en ppm) : 7,10 (t, J = 7,5 Hz, 1H), 7,35 (t, J = 7,5 Hz, 2H), 7,78 (m, 3H), 7,90 (d, J = 7,5 Hz, 2H), 8,54 (s, 1H), 8,60 (s, 1H), 9,07 (s large, 1H), 10,25 (s, 1H). |
| | Spectre de masse (LC-MS-DAD-ELSD) : m/z 305 [M+H]⁺. |
| **15** | Spectre RMN ¹H (DMSO-d6, δ en ppm) : 7,10 (t, J = 7,5 Hz, 1H), 7,15 (m, 3H), 7,33 (t, J = 7,5 Hz, 2H), 7,64 (d, J = 9,5 Hz, 1H), 7,81 (dd, J =1,5 et 9,5 Hz, 1H), 7,89 (d, J = 7,5 Hz, 2H), 8,61 (s, 1H), 8,92 (s large, 1H), 10,2 (s, 1H). |
| | Spectre de masse (LC-MS-DAD-ELSD) : m/z 336 [M+H]⁺, m/z 334 [M-H]⁻. |
| **16** | Spectre RMN ¹H (DMSO-d6, δ en ppm) : 1,64 (s, 3H), 3,82 (m, 2H), 4,03 (m, 2H), 7,10 (t, J = 7,5 Hz, 1H), 7,34 (t, J = 7,5 Hz, 2H), 7,39 (dd, J = 1,5 et 9,5 Hz, 1H), 7,64 (d, J = 9,5 Hz, 1H), 7,90 (d, J = 7,5 Hz, 2H), 8,53 (s, 1H), 8,68 (s large, 1H), 10,2 (s large, 1H). |
| | Spectre de masse (LC-MS-DAD-ELSD) : m/z 324 [M+H]⁺. |
| **17** | Sel HCl (1:1) : |
| | Spectre RMN ¹H (DMSO-d6, δ en ppm) : 4,04 (s, 4H), 7,12 (dt, J=7,3, 1,2 Hz, 1H), 7,31 - 7,40 (m, 2H), 7,82 (dd, J=9,6, 1,9 Hz, 1H), 7,87 - 7,95 (m, 3H), 8,76 (d, J=0,8 Hz, 1H), 9,47 (dd, J=1,9, 0,8 Hz, 1H), 10,46 (s, 1H), 10,89 (s large, 2H). |
| | Spectre de masse (LC-MS-DAD-ELSD) : m/z 305 [M+H]⁺. |
| **18** | Spectre RMN ¹H (DMSO-d6, δ en ppm) : 4,01 (s, 3H), 6,85 (d, J=8,0 Hz, 1H), 7,10 (t, J=7,7 Hz, 1H), 7,35 (t, J=7,7 Hz, 2H), 7,61 (d, J=8,0 Hz, 1H), 7,76 (d, J=9,8 Hz, 1H), 7,85 (t, J=8,0 Hz, 1H), 7,91 (d, J=7,7 Hz, 2H), 8,11 (d large, J=9,8 Hz, 1H), 8,61 (s, 1H), 9,40 (s large, 1H), 10,26 (s, 1H). |
| | Spectre de masse (LC-MS-DAD-ELSD) : m/z 345 [M+H]⁺. |
| **19** | Spectre RMN ¹H (DMSO-d6, δ en ppm) : 2,74 (s, 3H), 7,10 (tt, dd, J=7,6, 1,2 Hz, 1H), 7,36 (t large , J=7,6 Hz, 2H), 7,45 (ddd, J=7,6, 4,8, 1,2 Hz, 1H), 7,57 (d, J=9,3 Hz, 1H), 7,64 - 7,71 (m, 2H), 7,89 - 8,00 (m, 3H), 8,53 (d, J=0,8 Hz, 1H), 8,74 (ddd, J=4,8, 1,9, 0,9 Hz, 1H), 10,30 (s, 1H). |
| | Spectre de masse (LC-MS-DAD-ELSD) : m/z 329 [M+H]⁺. |
| **20** | Spectre RMN ¹H (DMSO-d6, δ en ppm) : 7,10 (t, J=7,7 Hz, 1H), 7,27 - 7,49 (m, 6H), 7,62 (dd, J=9,6, 2,2 Hz, 1H), 7,72 (d, J=9,6 Hz, 1H), 7,84 (d large, J=7,7 Hz, 2H), 8,49 (d, J=0,8 Hz, 1H), 8,84 (s large, 1H), 9,98 (s large, 1H), 12,51 (m étalé, 1H). |
| | Spectre de masse (LC-MS-DAD-ELSD) : m/z 319 [M+H]⁺. |
| **21** | Spectre RMN ¹H (DMSO-d6, δ en ppm) : 7,09 (tt, J=7,8, 1,2 Hz, 1H), 7,29 ( s large, 2H), 7,34 (t large, J=7,8 Hz, 2H), 7,49 (s, 1H), 7,63 (d, J=9,5 Hz, 1H), 7,67 (dd, J=9,5, 2,0 Hz, 1H), 7,89 (d large, J=7,8 Hz, 2H), 8,45 (s, 1H), 8,62 (s large, 1H), 10,19 (s, 1H). |
| | Spectre de masse (LC-MS-DAD-ELSD) : m/z 336 [M+H]⁺ , m/z 334 [M-H]⁻. |
| **22** | Spectre RMN ¹H (DMSO-d6, δ en ppm) : 6,52 (d large, J=8,4 Hz, 1H), 6,99 (m étalé, 1H), 7,10 (t large, J=7,7 Hz, 1H), 7,35 (t, J=7,7 Hz, 2H), 7,66 (t large, J=8,4 Hz, 1H), 7,74 (d, J=9,4 Hz, 1H), 7,84 (d large, J=9,4 Hz, 1H), 7,90 (d, J=7,7 Hz, 2H), 8,58 (s, 1H), 9,16 (s large, 1H), 10,29 (s, 1H), 11,8 (m très étalé, 1H). |
| **23** | Spectre RMN ¹H (DMSO-d6, δ en ppm) : 7,10 (t, J=7,8 Hz, 1H), 7,35 (t, J=7,8 Hz, 2H), 7,75 (d, J=9,5 Hz, 1H), 7,87 (dd, J=9,5, 1,7 Hz, 1H), 7,90 (d, J=7,8 Hz, 2H), 8,42 (s, 1H), 8,57 (s, 1H), 9,20 (s large, 1H), 10,26 (s, 1H), 15,3 (m très étalé, 1H). |
| | Spectre de masse (LC-MS-DAD-ELSD) : m/z 305 [M+H]⁺, m/z 303 [M-H]⁻. |
| **24** | Spectre RMN ¹H (DMSO-d6, δ en ppm) : 6,93 (tt, J=9,5, 2,4 Hz, 1H), 6,98 (s, 1H), 7,70 (s, 2H), 7,74 (dd, J=9,5, 2,4 Hz, 2H), 7,82 (s, 1H), 8,29 (s, 1H), 8,47 (s, 1H), 8,93 (s, 1H), 10,74 (s, 1H). |
| | Spectre de masse (LC-MS-DAD-ELSD) : m/z 340 [M+H]⁺. |
| **25** | Spectre RMN ¹H (DMSO-d6, δ en ppm) : 6,92 (td, J=8,3, 2,3, 1,0 Hz, 1H), 6,98 (dd, J=1,5, 0,9 Hz, 1H), 7,38 (td, J=8,3, 6,8 Hz, 1H), 7,69 (d, J=1,4 Hz, 2H), 7,74 (ddd, J=8,3, 2,3, 0,9 Hz, 1H), 7,82 (t, J=1,5 Hz, 1H), 7,88 (dt, J=12,3, 2,3 Hz, 1H), 8,29 (dd, J=1,5, 0,9 Hz, 1H), 8,45 (s, 1H), 8,93 (t, J=1,4 Hz, 1H), 10,50 (s, 1H). |
| | Spectre de masse (LC-MS-DAD-ELSD) : m/z 322 [M+H]⁺. |
| **26** | Spectre RMN ¹H (DMSO-d6, δ en ppm) : 6,68 (dd, J = 2,0 et 3,5 Hz, 1H), 7,07 (d, J = 3,5 Hz, 1H), 7,10 (t, J = 7,5 Hz, 1H), 7,35 (t, J = 7,5 Hz, 2H), 7,71 (d, J = 9,5 Hz, 1H), 7,77 (dd, J = 2,0 et 9,5 Hz, 1H), 7,84 (d, J = 2,0 Hz, 1H), 7,90 (d, J = 7,5 Hz, 2H), 8,58 (s, 1H), 9,00 (s large, 1H), 10,3 (s, 1H). |
| | Spectre de masse (LC-MS-DAD-ELSD) : m/z 304 [M+H]⁺. |
| **27** | Spectre RMN ¹H (DMSO-d6, δ en ppm) : 4,69 (s, 2H), 7,11 (t, J = 8,0 Hz, 1H), 7,37 (t, J = 8,0 Hz, 2H), 7,51 (d, J = 8,0 Hz, 1H), 7,79 (d, J = 9,5 Hz, 1H), 7,89 (m, 3H), 7,98 (t, J = 8,0 Hz, 1H), 8,17 (d large, 9,5 Hz, 1H), 8,67 (s, 1H), 9,42 (s large, 1H), 10,35 (s large, 1H). |
| | Spectre de masse (LC-MS-DAD-ELSD) : m/z 345 [M+H]⁺. |
| **28** | Spectre RMN ¹H (DMSO-d6, δ en ppm) : 7,11 (t, J = 7,5 Hz, 1H), 7,37 (t, J = 7,5 Hz, 2H), 7,59 (dd, J = 6,0 et 8,0 Hz, 1H), 7,74 (d large, J = 8,0 Hz, 1H), 7,80 (d, J = 9,5 Hz, 1H), 7,85 (dd, J = 2,0 et 9,5 Hz, 1H), 7,90 (d, J = 7,5 Hz, 2H), 8,30 (d large, J = 6,0 Hz, 1H), 8,55 (s, 1H), 8,71 (t, J = 1,5 Hz, 1H), 9,20 (s large, 1H), 10,4 (s large, 1H). |
| | Spectre de masse (LC-MS-DAD-ELSD) : m/z 331 [M+H]⁺; m/z 329 [M-H]⁻. |
| **29** | Spectre RMN ¹H (DMSO-d6, δ en ppm) : 7,10 (t, J = 8,0 Hz, 1H), 7,33 (t, J = 8,0 Hz, 2H), 7,69 (m, 2H), 7,89 (d, J = 8,0 Hz, 2H), 8,11 (m étalé, 2H), 8,40 (s, 1H), 8,90 (s, 1H), 10,2 (s, 1H), 13,05 (m étalé, 1H). |
| | Spectre de masse (LC-MS-DAD-ELSD) : m/z 304 [M+H]⁺, m/z 302 [M-H]⁻. |
| **30** | Spectre RMN ¹H (DMSO-d6, δ en ppm) : 7,11 (t, J = 7,5 Hz, 1H), 7,37 (t, J = 7,5 Hz, 2H), 7,60 (s, 2H), de 7,83 à 7,95 (m, 4H), 8,70 (s, 1H), 9,23 (s, 1H), 10,3 (s, 1H) (signaux élargis). |
| | Spectre de masse (LC-MS-DAD-ELSD) : m/z 304 [M+H]⁺, m/z 302 [M-H]⁻. |
| **31** | Spectre RMN ¹H (DMSO-d6, δ en ppm) : 3,71 (s, 3H), 7,10 (t, J = 7,5 Hz, 1H), 7,34 (t, J = 7,5 Hz, 2H), 7,65 (d, J = 9,5 Hz, 1H), 7,69 (s, 1H), 7,71 (s, 1H), 7,73 (dd, J = 1,5 et 9,5 Hz, 1H), 7,89 (d, J = 7,5 Hz, 2H), 8,55 (s, 1H), 8,97 (s large, 1H), 10,2 (s large, 1H). |
| | Spectre de masse (LC-MS-DAD-ELSD) : m/z 318 [M+H]⁺. |
| **32** | Spectre RMN ¹H (DMSO-d6, δ en ppm) : 7,10 (t, J = 7,5 Hz, 1H), 7,35 (t, J = 7,5 Hz, 2H), 7,46 (s, 1H), 7,81 (d, J = 9,5 Hz, 1H), 7,90 (m, 3H), 8,31 (s, 1H), 8,67 (s, 1H), 9,38 (s large, 1H), 10,3 (s, 1H). |
| | Spectre de masse (LC-MS-DAD-ELSD) : m/z 305 [M+H]⁺. |
| **33** | Spectre RMN ¹H (DMSO-d6, δ en ppm) : 6,94 (tt, J=9,3, 2,4 Hz, 1H), 7,43 (ddd, J=7,2, 4,8, 1,3 Hz, 1H), 7,69 - 7,82 (m, 3H), 7,92 - 8,05 (m, 2H), 8,12 (dd, J=9,6, 1,9 Hz, 1H), 8,66 (d, J=1,0 Hz, 1H), 8,71 (ddd, J=4,8, 1,8, 1,0 Hz, 1H), 9,43 (dd, J=1,9, 1,0 Hz, 1H), 10,79 (s, 1H). |
| | Spectre de masse (LC-MS-DAD-ELSD) : m/z 351 [M+H]⁺. |
| **34** | Spectre RMN ¹H (DMSO-d6, δ en ppm) : 7,10 (t, J = 8,0 Hz, 1H), 7,35 (t large, J = 8,0 Hz, 2H), de 7,77 à 7,87 (m, 4H), 7,91 (d large, J = 8,0 Hz, 2H), 8,54 (s, 1H), 8,71 (m, 2H), 9,22 (s large, 1H), 10,3 (s, 1H). |
| | Spectre de masse (LC-MS-DAD-ELSD) : m/z 315 [M+H]⁺. |
| **35** | Spectre RMN ¹H (DMSO-d6, δ en ppm) : 7,10 (tt, J=7,7, 1,2 Hz,1H), 7,13 - 7,26 (m, 2H), 7,35 (t large, J=7,7 Hz, 1H), 7,50 (d large, J=7,3 Hz, 1H), 7,71 (dt, J=9,5, 0,9 Hz, 1H), 7,79 (dd, J=9,5, 2,2 Hz, 1H), 7,87 (d, J=2,8 Hz, 1H), 7,91 (d large, J=7,8 Hz, 2H), 8,00 (d large, J=7,8 Hz, 1H), 8,58 (d, J=0,8 Hz, 1H), 8,99 (s large, 1H), 10,21 (s, 1H), 11,50 (s large, 1H). |
| | Spectre de masse (LC-MS-DAD-ELSD) : m/z 353 [M+H]⁺, m/z 351 [M-H]⁻. |
| **36** | Spectre RMN ¹H (DMSO-d6, δ en ppm) : 7,10 (t large, J=7,7 Hz, 1H), 7,20 (dd, J=5,4, 3,7 Hz, 1H), 7,35 (t, J=7,7 Hz, 2H), 7,59 (dd, J=3,7, 1,5 Hz, 1H), 7,63 (d, J=5,4, 1,5 Hz, 1H), 7,66 - 7,78 (m, 2H), 7,90 (m, 2H), 8,52 (s, 1H), 9,01 (s large, 1H), 10,25 (s, 1H). |
| **37** | Spectre RMN ¹H (DMSO-d6, δ en ppm) : 7,10 (t large, J=7,8 Hz, 1H), 7,35 (t, J=7,8 Hz, 2H), 7,82 (d, J=9,8 Hz, 1H), 7,91 (d large, J=7,8 Hz, 2H), 8,14 (dd, J=9,8, 1,9 Hz, 1H), 8,63 (s large, 1H), 8,68 (d large, J=2,7 Hz, 1H), 8,77 (dd, J=2,7, 1,5 Hz,1H), 9,32 (d large, J=1,5 Hz, 1H), 9,51 (dd, J=1,9, 1,0 Hz, 1H), 10,31 (s, 1H). |
| | Spectre de masse (LC-MS-DAD-ELSD) : m/z 316 [M+H]⁺. |
| **38** | Spectre RMN ¹H (DMSO-d6, δ en ppm) : 7,10 (tt, J=7,7, 1,2 Hz, 1H), 7,35 (t large, J=7,7 Hz, 2H), 7,43 - 7,53 (m, 2H), 7,71 (dd, J=9,6, 0,9 Hz, 1H), 7,79 - 7,86 (m, 2H), 7,91 (d large, J=7,7 Hz, 2H), 8,42 (m, 1H), 8,61 (d, J=0,8 Hz, 1H), 9,36 (s large, 1H), 10,28 (s, 1H). |
| | Spectre de masse (LC-MS-DA.D-ELSD) : m/z 331 [M+H]⁺. |
| **39** | Spectre RMN ¹H (DMSO-d6, δ en ppm) : 7,10 (t large, J=7,7 Hz, 1H), 7,35 (t, J=7,7 Hz, 2H), 7,82 (d large, J=9,8 Hz, 1H), 7,86 (dd, J=9,8, 1,5 Hz, 1H), 7,91 (d, J=7,7 Hz, 2H), 8,52 (s, 1H), 9,18 (s large, 1H), 9,22 (s, 2H), 9,25 (s, 1H), 10,30 (s, 1H). |
| | Spectre de masse (LC-MS-DAD-ELSD) : m/z 316 [M+H]⁺. |
| **40** | Spectre RMN ¹H (DMSO-d6, δ en ppm) : 7,10 (t large, J=7,8 Hz, 1H), 7,35 (t, J=7,8 Hz, 2H), 7,59 (dd, J=5,1, 1,5 Hz, 1H), 7,71 (d, J=9,8 Hz, 1H), 7,74 (dd, J=5,1, 2,9 Hz, 1H), 7,81 (dd, J=9,8, 2,0 Hz, 1H), 7,90 (m, 2H), 7,99 (dd, J=2,9, 1,5 Hz, 1H), 8,46 (s, 1H), 9,05 (s large, 1H), 10,24 (s, 1H). |
| | Spectre de masse (LC-MS-DAD-ELSD) : m/z 320 [M+H]⁺. |
| **41** | Spectre RMN ¹H (DMSO-d6, δ en ppm) : 5,99 (dd, J=7,0, 3,6 Hz, 1H), 7,06 (t, J=3,6 Hz, 1H), 7,09 (tt, J=7,7, 1,4 Hz, 1H), 7,33 (t, J=7,7 Hz, 2H), 7,67 - 7,76 (m, 2H), 7,89 (m, 2H), 8,53 (s, 1H), 8,91 (s, 1H), 10,22 (s, 1 H). |
| | Spectre de masse (LC-MS-DAD-ELSD) : m/z 322 [M+H]⁺. |
| **42** | Spectre RMN ¹H (DMSO-d6, δ en ppm) : 6,92 (m, 1H), 7,33 - 7,45 (m, 2H), 7,76 (m, 2H), 7,89 (dt, J=11,6, 2,4 Hz, 1H), 7,96 (td, J=7,8, 1,6 Hz, 1H), 8,03 (dt, J=7,8, 1,6 Hz, 1H), 8,12 (dd, J=9,6, 1,9 Hz, 1H), 8,64 (d, J=0,8 Hz, 1H), 8,71 (ddd, J=4,8, 1,8, 1,0 Hz, 1H), 9,43 (dd, J=1,9, 0,8 Hz, 1H), 10,55 (s, 1H). |
| | Spectre de masse (LC-MS-DAD-ELSD) : m/z 333 [M+H]⁺. |
| **43** | Spectre RMN ¹H (DMSO-d6, δ en ppm) : 7,16 - 7,38 (m, 3H), 7,43 (ddd, J=7,5, 4,7, 1,3 Hz, 1H), 7,81 (d, J=9,6 Hz, 1H), 7,96 (td, J=7,5, 1,3 Hz, 1H), 8,03 (d large, J=7,5 Hz, 1H), 8,06 - 8,15 (m, 2H), 8,65 (s, 1H), 8,71 (d large, J=4,7 Hz, 1H), 9,43 (s large, 1H), 9,85 (s large, 1H). |
| | Spectre de masse (LC-MS-DAD-ELSD) : m/z 333 [M+H]⁺. |
| **44** | Spectre RMN ¹H (DMSO-d6, δ en ppm) : 7,06 (m, 1H), 7,45 - 7,47 (m, 2H), 7,82 (d large, J=9,8 Hz, 1H), 7,91 - 8,06 (m, 3H), 8,14 (dd, J=9,8, 1,7 Hz, 1H), 8,69 (d, J=0,9 Hz, 1H), 8,71 (d large, J=5,4 Hz, 1H), 9,43 (s large, 1H), 9,89 (s large, 1H). |
| | Spectre de masse (LC-MS-DAD-ELSD) : m/z 351 [M+H]⁺. |
| **45** | Spectre RMN ¹H (DMSO-d6, δ en ppm) : 7,17 - 7,33 (m, 2H), 7,43 (ddd, J=7,9, 4,8, 1,0 Hz, 1H), 7,77 (m partiellement masqué, 1H), 7,80 (dt, J=9,6, 0,9 Hz, 1H), 7,96 (dt, J=7,9, 1,8 Hz, 1H), 8,03 (dt, J=7,9, 1,0 Hz, 1H), 8,13 (dd, J=9,6, 1,8 Hz, 1H), 8,66 (d, J=0,9 Hz, 1H), 8,71 (ddd, J=4,8, 1,8, 1,0 Hz, 1H), 9,43 (dd, J=1,8, 0,9 Hz, 1H), 10,09 (s large, 1H). |
| | Spectre de masse (LC-MS-DAD-ELSD) : m/z 351 [M+H]⁺. |
| **46** | Spectre RMN ¹H (DMSO-d6, δ en ppm) : 6,07 (s large, 2H), 6,49 (dd, J=8,3, 0,9 Hz, 1H), 6,92 (td large, J= 8,3, 2,5 Hz, 1H), 7,09 (dd, J=7,4, 0,9 Hz, 1H), 7,38 (td, J=8,3, 6,9 Hz, 1H), 7,52 (dd, J=8,3, 7,4 Hz, 1H), 7,70 (dt, J=9,6, 0,9 Hz, 1H), 7,75 (dd large, J=8,3, 1,8 Hz, 1H), 7,88 (dt, J=11,7, 2,5 Hz, 1H), 7,99 (dd, J=9,7, 1,8 Hz, 1H), 8,62 (d, J=0,9 Hz, 1H), 9,19 (dd, J=1,8, 0,9 Hz, 1H), 10,52 (s large, 1H). |
| | Spectre de masse (LC-MS-DAD-ELSD) : m/z 348 [M+H]⁺. |
| **47** | Spectre RMN ¹H (DMSO-d6, δ en ppm): 6,47 (td, J=2,8, 1,7 Hz, 1H), 6,84 - 6,95 (m, 2H), 7,31 (dt, J=2,8, 1,7 Hz, 1H), 7,37 (td, J=8,3, 6,8 Hz, 1H), 7,60 (dt, J=9,4, 0,9 Hz, 1H), 7,67 (dd, J=9,4, 1,6 Hz, 1H), 7,74 (d large, J=8,3 Hz, 1H), 7,88 (dt, J=12,1, 2,6 Hz, 1H), 8,42 (d, J=0,9 Hz, 1H), 8,76 (s large, 1H), 10,45 (s large, 1H), 11,06 (m étalé, 1H). |
| | Spectre de masse (LC-MS-DAD-ELSD) : m/z 321 [M+H]⁺. |
| **48** | Spectre RMN ¹H (DMSO-d6, δ en ppm): 6,77 (s large, 1H), 6,92 (td, J=8,5, 2,6 Hz, 1H), 7,38 (td, J=8,3, 6,8 Hz, 1H), 7,67 - 7,78 (m, 2H), 7,84 - 7,92 (m, 3H), 8,55 (s, 1H), 9,09 (s large, 1H), 10,51 (s large, 1H), 13,05 (s large, 1H). |
| | Spectre de masse (LC-MS-DAD-ELSD): m/z 322 [M+H]⁺, m/z 320 [M-H]⁻. |
| **49** | Spectre RMN ¹H (DMSO-d6, δ en ppm) : 6,66 (dd, J=3,4, 1,9 Hz, 1H), 6,85 - 6,97 (tdd, J=8,5, 2,5, 1,0 Hz, 1H), 7,05 (dd, J=3,4, 0,8 Hz, 1H), 7,38 (td, J=8,3, 6,8 Hz, 1H), 7,66-7,79 (m, 3H), 7,83 (dd, J=1,9, 0,8 Hz, 1H), 7,87 (d, J=11,9, 2,5 Hz, 1H), 8,58 (d, J=0,6 Hz, 1H), 8,99 (t, J=1,5 Hz, 1H), 10,51 (s large, 1H). |
| | Spectre de masse (LC-MS-DAD-ELSD): m/z 322 [M+H]⁺. |
| **50** | Spectre RMN ¹H (DMSO-d6, δ en ppm) : 6,92 (tdd, J=8,5, 2,6, 1,0 Hz, 1H), 7,38 (td, J=8,5, 6,8 Hz, 1H), 7,45 (d, J=0,9 Hz, 1H), 7,76 (ddd, J=8,5, 2,6, 1,0 Hz, 1H), 7,81 (dt, J=9,5, 1,0 Hz, 1H), 7,88 (dt, J=12,0, 2,6 Hz,1H 7,92 (dd, J=9,5, 1,9 Hz, 1H), 8,31 (d, J=0,9 Hz, 1H), 8,69 (d, J=1,0 Hz, 1H), 9,38 (dd, J=1,9, 1,0 Hz, 1H), 10,58 (s large, 1H). |
| | Spectre de masse (LC-MS-DAD-ELSD) : m/z 323 [M+H]⁺. |
| **51** | Spectre RMN ¹H (DMSO-d6, δ en ppm) : 7,10 (tt, J=7,5, 1,3 Hz, 1H), 7,35 (t, J=7,8 Hz, 2H), 7,52 (t, J=4,9 Hz, 1H), 7,79 (d, J=9,8 Hz, 1H), 7,91 (m, 2H), 8,30 (dd, J=9,8, 1,7 Hz, 1H), 8,74 (s, 1H), 8,96 (d, J=4,9 Hz, 2H), 9,69 (s large, 1H), 10,31 (s, 1H). |
| | Spectre de masse (LC-MS-DAD-ELSD) : m/z 316 [M+H]⁺. |
| **52** | Spectre RMN ¹H (DMSO-d6, δ en ppm) : 6,91 (tdd, J=8,3, 2,2, 1,0 Hz, 1H), 7,37 (td, J=8,3, 6,8 Hz, 1H), 7,65 (dt, J=9,5, 0,9 Hz, 1H), 7,69 - 7,79 (m, 3H), 7,81 (dd, J=9,5, 1,7 Hz, 1H), 7,87 (dt, J=11,7, 2,2 Hz, 1H), 8,58 (d, J=0,9 Hz, 1H), 9,00 (dd, J=1,7, 0,9 Hz, 1H), 10,46 (s, 1H), 12,28 (m étalé, 1H). |
| | Spectre de masse (LC-MS-DAD-ELSD) : m/z 322 [M+H]⁺, m/z 320 [M-H]⁻. |
| **53** | Spectre RMN ¹H (DMSO-d6, δ en ppm) : 6,92 (tdd, J=8,4, 2,6, 1,1 Hz, 1H), 7,38 (td, J=8,4, 6,9 Hz, 1H), 7,72 - 7,81 (m, 2H), 7,88 (ddt, J=12,1, 2,2 Hz, 1H), 7,99 (d large, J=9,6 Hz, 1H), 8,69 (s, 1H), 8,71 (s large, 1H), 9,32 (s large, 1H), 10,54 (s large, 1H), 14,26 (m étalé, 1H). |
| | Spectre de masse (LC-MS-DAD-ELSD) : m/z 323 [M+H]⁺. |
| **54** | Spectre RMN ¹H (DMSO-d6, δ en ppm) : 6,91 (tdd, J=8,5, 2,6, 1,0 Hz, 1H), 7,38 (td, J=8,5, 6,8 Hz, 1H), 7,67 - 7,82 (m, 4H), 7,86 (dt, J=12,0, 2,2 Hz, 1H), 8,55 (s, 1H), 8,86 (s large, 1H), 8,90 (m étalé, 2H), 10,61 (s, 1H). |
| | Spectre de masse (LC-MS-DAD-ELSD) : m/z 354 [M+H]⁺, m/z 352 [M-H]⁻. |
| **55** | Spectre RMN ¹H (DMSO-d6, δ en ppm) : 6,92 (tdd, J=8,5, 2,6, 1,0 Hz, 1H), 7,38 (td, J=8,3, 6,8 Hz, 1H), 7,73 - 7,79 (m, 2H), 7,84 - 7,92 (m, 2H), 8,44 (m étalé, 1H), 8,60 ( s large, 1H), 9,20 (s large, 1H), 10,53 (s large, 1H), 15,24 (m étalé, 1H). |
| | Spectre de masse (LC-MS-DAD-ELSD) : m/z 323 [M+H]⁺. |
| **56** | Spectre RMN ¹H (DMSO-d6, δ en ppm): 6,86 - 6,98 (tdd, J=8,4, 2,7, 0,8 Hz, 1H), 7,37 (td, J=8,4, 6,8 Hz, 1H), 7,63 - 7,78 (m, 3M, 7,88 (dt, J=11,8, 2,3 Hz, 1H), 7,99 (m étalé, 1H), 8,24 (m étalé, 1H), 8,44 (s, 1H), 8,90 (t, J=1,4 Hz, 1H), 10,48 (s large, 1H), 13,07 (m étalé, 1H). |
| | Spectre de masse (LC-MS-DAD-ELSD) : m/z 322 [M+H]⁺, m/z 320 [M-H]⁻. |
| **57** | Spectre RMN ¹H (DMSO-d6, δ en ppm) : 4,49 (d, J=5,5 Hz, 2H), 5,30 (t, J=5,5 Hz, 1H), 6,47 (d, J=3,4 Hz, 1H), 6,91 (tdd, J=8,4, 2,3, 0,9 Hz, 1H), 6,98 (d, J=3,4 Hz, 1H), 7,37 (td, J=8,4, 6,8 Hz, 1H), 7,67 - 7,77 (m, 3H), 7,87 (ddd, J=12,1, 2,3 Hz, 1H), 8,60 (s, 1H), 8,95 (t, J=1,4 Hz, 1H), 10,50 (s, 1H). |
| | Spectre de masse (LC-MS-DAD-ELSD) : m/z 352 [M+H]⁺, m/z 350 [M-H]⁻. |
| **58** | Spectre RMN ¹H (DMSO-d6, δ en ppm) : 4,53 (d, J=5,6 Hz, 2H), 5,37 - 5,47 (m, 1H), 7,09 (t, J=7,6 Hz, 1H), 7,34 (t, J=7,6 Hz, 2H), 7,59 - 7,67 (m, 2H), 7,78 (dd, J=9,5, 1,5 Hz, 1H), 7,89 (d, J=7,6 Hz, 2H), 8,54 (s, 1H), 8,96 (s, 1H), 10,16 (s large, 1H), 12,15 (m étalé, 1H). |
| | Spectre de masse (LC-MS-ES+/-) : m/z 332 [M+H]⁻, m/z 334 [M+H]⁺ |
| **59** | Spectre RMN ¹H (DMSO-d6, δ en ppm) : 2,58 (s, 3H), 6,90 - 6,97 (m, 1H), 7,29 (d, J=6,8 Hz, 1H), 7,75 (d, J=9,5 Hz, 3H) 7,79 - 7,87 (m, 2H) 8,10 (dd, J=9,5, 1,7 Hz, 1H), 8,67 (s, 1H), 9,37 (s large, 1H), 10,77 (s large, 1H). |
| | Spectre de masse (LC-MS-ES+/-) : m/z 363 [M+H]⁻, m/z 365 [M+H]⁺ |
| **60** | Spectre RMN ¹H (DMSO-d6, δ en ppm) : 7,21 (td, J=7,9, 1,6 Hz, 1H), 7,38 - 7,47 (m, 2H), 7,59 (dd, J=7,9, 1,6 Hz, 1H), 7,82 (d, J=9,6 Hz, 1H), 7,92 - 8,07 (m, 2H), 8,14 (dd, J=9,6, 1,8 Hz, 1H), 8,36 (dd, J=8,3, 1,6 Hz, 1H), 8,67 - 8,74 (m, 2H), 9,43 (s large, 1H), 9,96 (s large, 1H). |
| | Spectre de masse (LC-MS-ES+/-) : m/z 349 [M+H]⁺ |
| **61** | Spectre RMN ¹H (DMSO-d6, δ en ppm) : 6,47 (m, 1H), 6,88 (m, 1H), 6,91 (tt, J=9,2, 2,4 Hz, 1H), 7,31 (m, 1H), 7,60 (d, J=9,5 Hz, 1H), 7,68 (dd, J=9,5, 1,8 Hz, 1H), 7,74 (m, 2H), 8,44 (s, 1H), 8,76 (s large, 1H), 10,69 (s large, 1H), 11,06 (m étalé, 1H). |
| | Spectre de masse (LC-MS-ES+/-) : m/z 337 [M+H]⁻, m/z 339 [M+H]⁺ |
| **62** | Spectre RMN ¹H (DMSO-d6, δ en ppm) : 6,47 (m, 1H), 6,87 (m, 1H), 7,13 - 7,38 (m, 4H), 7,64 (d, J=9,5 Hz, 1H), 7,70 (dd, J=9,5, 1,7 Hz, 1H), 8,13 (td, J=7,8, 2,0 Hz, 1H), 8,43 (s, 1H), 8,76 (s large, 1H), 9,76 (s large, 1H), 11,05 (m étalé, 1H). |
| | Spectre de masse (LC-MS-ES+/-) : m/z 319 [M+H]⁻, m/z 321 [M+H]⁺ |
| **63** | Spectre RMN ¹H (DMSO-d6, δ en ppm) : 6,47 (m, 1H), 6,87 (m, 1H), 7,03 (m, 1H), 7,32 (m, 1H), 7,40 (ddd, J=10,4, 9,1, 5,0 Hz, 1H), 7,65 (d, J=9,5 Hz, 1H), 7,71 (dd, J=9,5, 1,7 Hz, 1H), 8,04 (ddd, J=10,4, 6,2, 3,3 Hz, 1H), 8,46 (s, 1H), 8,76 (s large, 1H), 9,81 (s large, 1H), 11,07 (m étalé, 1H). |
| | Spectre de masse (LC-MS-ES+/-) : m/z 337 [M+H]⁻, m/z 339 [M+H]⁺ |
| **64** | Spectre RMN ¹H (DMSO-d6, δ en ppm) : 6,47 (m, 1H), 6,87 (m, 1H), 7,17 - 7,29 (m, 2H), 7,32 (m, 1H), 7,64 (d, J=9,5 Hz, 1H), 7,70 (dd, J=9,5, 1,7 Hz, 1H), 7,83 (m, 1H), 8,44 (s, 1H), 8,76 (s large, 1H), 9,98 (s large, 1H), 11,05 (m étalé, 1H). |
| | Spectre de masse (LC-MS-ES+/-): m/z 337 [M+H]⁻, m/z 339 [M+H]⁺ |
| **65** | Spectre RMN ¹H (DMSO-d6, δ en ppm) : 6,76 (d, J=2,2 Hz, 1H), 6,93 (tt, J=9,4, 2,4 Hz, 1H), 7,67 - 7,94 (m, 5H), 8,56 (s, 1H), 9,09 (s large, 1H), 10,74 (s large, 1H), 13,05 (m étalé, 1H). |
| | Spectre de masse (LC-MS-ES+/-) : m/z 338 [M+H]⁻, m/z 340 [M+H]⁺ |
| **66** | Spectre RMN ¹H (DMSO-d6, δ en ppm) : 6,66 (dd, J=3,4, 1,9 Hz, 1H), 7,06 (d, J=3,4 Hz, 1H), 7,16 - 7,39 (m, 3H), 7,77 (m, 2H), 7,83 (d, J=1,9 Hz, 1H), 8,04 - 8,14 (m, 1H), 8,59 (s, 1H), 8,98 (s large, 1H), 9,80 (s large, 1H). |
| | Spectre de masse (LC-MS-ES+/-) : m/z 322 [M+H]⁺ |
| **67** | Spectre RMN ¹H (DMSO-d6, δ en ppm) : 6,66 (dd, J=3,4, 1,9 Hz, 1H), 6,97 - 7,13 (m, 2H), 7,40 (ddd, J=10,5, 9,1, 5,0 Hz, 1H), 7,72 - 7,82 (m, 2H), 7,83 (dd, J=1,9, 0,9 Hz, 1H), 8,01 (ddd, J=10,3, 6,2, 3,2 Hz, 1H), 8,62 (s, 1H), 8,98 (s large, 1H), 9,84 (s large, 1H). |
| | Spectre de masse (LC-MS-ES+/-) : m/z 340 [M+H]⁺ |
| **68** | Spectre RMN ¹H (DMSO-d6, δ en ppm) : 6,66 (dd, J=3,4, 1,9 Hz, 1H), 7,06 (d, J=3,4 Hz, 1H), 7,17 - 7,34 (m, 2H), 7,70 - 7,87 (m, 4H), 8,61 (s, 1H), 8,99 (s large, 1H), 10,04 (s large, 1H). |
| | Spectre de masse (LC-MS-ES+/-) : m/z 340 [M+H]⁺ |
| **69** | Spectre RMN ¹H (DMSO-d6, δ en ppm) : 6,66 (dd, J=3,4, 1,8 Hz, 1H), 7,07 (d, J=3,4 Hz, 1H), 7,20 (m, 1H), 7,37 - 7,47 (m, 1H), 7,58 (dd, J=8,2, 1,6 Hz, 1H), 7,78 (m, 2H), 7,81 - 7,86 (m, 1H), 8,36 (dd, J=8,2, 1,6 Hz, 1H), 8,62 (s, 1H), 8,96 - 9,03 (s large, 1H), 9,92 (s large, 1H). |
| | Spectre de masse (LC-MS-ES+/-) : m/z 338 [M+H]⁺ |
| **70** | Spectre RMN ¹H (DMSO-d6, δ en ppm) : 7,06 (m, 1H), 7,40 (ddd, J=10,4, 9,1, 5,1 Hz, 1H), 7,46 (d, J=0,9 Hz, 1H), 7,85 (d, J=9,6 Hz, 1H), 7,85 (dd, J=9,6, 1,7 Hz, 1H), 7,97 (m, 1H), 8,31 (d, J=0,9 Hz, 1H), 8,72 (s, 1H), 9,37 (s large, 1H), 9,91 (s large, 1H). |
| | Spectre de masse (LC-MS-ES+/-) : m/z 341 [M+H]⁺ |
| **71** | Spectre RMN ¹H (DMSO-d6, δ en ppm) : 7,17 - 7,25 (m, 1H), 7,38 - 7,47 (m, 2H), 7,58 (dd, J=8,0, 1,4 Hz, 1H), 7,85 (d, J=9,5 Hz, 1H), 7,92 (dd, J=9,5, 1,7 Hz, 1H), 8,29 - 8,34 (m, 2H), 8,72 (s, 1H), 9,38 (s large, 1H), 9,95 (s large, 1H). |
| | Spectre de masse (LC-MS-ES+/-) : m/z 339 [M+H]⁺ |
| **72** | Spectre RMN ¹H (DMSO-d6, δ en ppm) : 6,95 - 7,00 (m, 1H), 7,14 - 7,39 (m, 3H), 7,68 - 7,78 (m, 2H), 7,80 - 7,84 (m, 1H), 8,06 - 8,15 (m, 1H), 8,30 (s large, 1H), 8,46 (s, 1H), 8,92 (s large, 1H), 9,80 (s large, 1H). |
| | Spectre de masse (LC-MS-ES+/-) : m/z 322 [M+H]⁺ |
| **73** | Spectre RMN ¹H (DMSO-d6, δ en ppm) : 6,96 - 7,11 (m, 2H), 7,33 - 7,46 (m, 1H), 7,69-7,79 (m, 2H), 7,79 - 7,85 (m, 1H), 7,98 - 8,07 (m, 1H), 8,29 (s large, 1H), 8,49 (s, 1H), 8,92 (s large, 1H), 9,85 (s large, 1H). |
| | Spectre de masse (LC-MS-ES+/-) : m/z 340 [M+H]⁺ |
| **74** | Spectre RMN ¹H (DMSO-d6, δ en ppm) : 6,93 - 7,00 (m, 1H), 7,20 - 7,29 (m, 2H), 7,67 - 7,86 (m, 4H), 8,29 (s large, 1H), 8,47 (s, 1H), 8,92 (s large, 1H), 10,04 (s large, 1H). |
| | Spectre de masse (LC-MS-ES+/-) : m/z 340 [M+H]⁺ |
| **75** | Spectre RMN ¹H (DMSO-d6, δ en ppm) : 6,98 (dd, J=1,9, 0,9 Hz, 1H), 7,20 (td, J=7,9, 1,6 Hz, 1H), 7,42 (td, J=7,9 1,6 Hz, 1H), 7,58 (dd, J=7,9, 1,6 Hz, 1H), 7,72 (dd, J=9,5, 1,7 Hz, 1H), 7,76 (d, J=9,5 Hz, 1H), 7,82 (t, J=1,9 Hz, 1H), 8,30 (s large, 1H), 8,37 (dd, J=7,9, 1,6 Hz, 1H), 8,49 (s, 1H), 8,93 (s large, 1H), 9,93 (s large, 1H). |
| | Spectre de masse (LC-MS-ES+/-) : m/z 338 [M+H]⁺ |
| **76** | Spectre RMN ¹H (DMSO-d6, δ en ppm) : 4,49 (d, J=5,3 Hz, 2H), 5,30 (t, J=5,3 Hz, 1H), 6,47 (d, J=3,4 Hz, 1H), 6,99 (d, J=3,4 Hz, 1H), 7,21 - 7,28 (m, 2H), 7,73 - 7,83 (m, 3H), 8,62 (s, 1H), 8,94 (s large, 1H), 10,03 (s large, 1H). |
| | Spectre de masse (LC-MS-ES+/-) : m/z 368 [M+H]⁻, m/z 370 [M+H]⁺ |
| **77** | Spectre RMN ¹H (DMSO-d6, δ en ppm) : 7,14 - 7,40 (m, 3H), 7,82 (d, J=9,5 Hz, 1H), 7,97 (dd, J=9,5, 1,7 Hz, 1H), 8,03 - 8,15 (m, 1H), 8,63 (m large, 1H), 8,71 (s, 1H), 9,31 (s large, 1H), 9,84 (s large, 1H), 14,19 - 14,39 (m étalé, 1H). |
| | Spectre de masse (LC-MS-ES+/-) : m/z 321 [M+H]⁻, m/z 323 [M+H]⁺ |
| **78** | Spectre RMN ¹H (DMSO-d6, δ en ppm) : 7,17 - 7,24 (m, 1H), 7,38 - 7,45 (m, 1H), 7,58 (dd, J=8,0,1,4 Hz, 1H), 7,81 (d, J=9,5 Hz, 1H), 7,99 (dd, J=9,5, 1,7 Hz, 1H), 8,35 (dd, J=8,2, 1,4 Hz, 1H), 8,61 - 8,67 (m étalé, 1H), 8,73 (s, 1H), 9,33 (s large, 1H), 9,95 (s large, 1H), 14,14 - 14,42 (m étalé, 1H). |
| | Spectre de masse (LC-MS-ES+/-) : m/z 337 [M+H]⁻, m/z 339 [M+H]⁺ |
| **79** | Spectre RMN ¹H (DMSO-d6, δ en ppm) : 7,36 - 7,46 (m, 2H), 7,72 - 7,83 (m, 2H), 7,87 (dd, J=7,8, 1,2 Hz, 1H), 7,94 - 8,06 (m, 3H), 8,14 (dd, J=9,8, 1,7 Hz, 1H), 8,64 - 8,78 (m, 2H), 9,43 (s, 1H), 10,46 (s large, 1H). |
| | Spectre de masse (LC-MS-ES+/-) : m/z 340 [M+H]⁺ |
| **80** | Spectre RMN ¹H (DMSO-d6, δ en ppm) : 6,89 - 7,03 (m, 2H), 7,25 (d, J=7,4 Hz, 1H), 7,65 - 8,04 (m, 7H), 8,69 (s, 1H), 9,33 (s, 1H), 10,90 (s large, 1H). |
| | Spectre de masse (LC-MS-ES+/-) : m/z 366 [M+H]⁺ |
| **81** | Spectre RMN ¹H (DMSO-d6, δ en ppm) : 6,88 (d, J=8,7 Hz, 1H), 7,16 - 7,40 (m, 4H), 7,6 (m étalé, 2H), 7,82 - 7,98 (m, 3H), 8,00 - 8,10 (m, 1H), 8,67 (s, 1H), 9,27 (s, 1H), 9,94 (s large, 1H). |
| | Spectre de masse (LC-MS-ES+/-) : m/z 348 [M+H]⁺ |
| **82** | Spectre RMN ¹H (DMSO-d6, δ en ppm) : 6,89 (d, J=8,7 Hz, 1H), 7,02 - 7,12 (m, 1H), 7,22 (d, J=7,4 Hz, 1H), 7,41 (ddd, J=10,3, 9,2, 5,0 Hz, 1H), 7,6 (m, 2H), 7,84 - 8,02 (m, 4H), 8,69 (s, 1H), 9,27 (s, 1H), 9,96 (s large, 1H). |
| | Spectre de masse (LC-MS-ES+/-) : m/z 366 [M+H]⁺ |
| **83** | Spectre RMN ¹H (DMSO-d6, δ en ppm) : 6,98 (d, J=8,4 Hz, 1H), 7,20 - 7,35 (m, 3H), 7,69 - 7,78 (m, 1H), 7,87 - 8,01 (m, 3H), 7,95 (m étalé, 2H), 8,69 (s, 1H), 9,31 (s, 1H), 10,23 (s large, 1H). |
| | Spectre de masse (LC-MS-ES+/-) : m/z 366 [M+H]⁺ |
| **84** | Spectre RMN ¹H (DMSO-d6, δ en ppm) : 6,89 (d, J=8,7 Hz, 1H), 7,17 - 7,27 (m, 2H), 7,38 - 7,48 (m, 1H), 7,59 (dd, J=7,9, 1,4 Hz, 1H), 7,60 (m, 2H), 7,83 - 7,98 (m, 3H), 8,32 (dd, J=8,2, 1,4 Hz, 1H), 8,68 (s, 1H), 9,28 (s, 1H), 10,01 (s large, 1H). |
| | Spectre de masse (LC-MS-ES+/-) : m/z 364 [M+H]⁺ |
| **85** | Spectre RMN ¹H (DMSO-d6, δ en ppm) : 6,47 (m, 1H), 6,87 (m, 1H), 7,19 (td, J=7,9, 1,6 Hz, 1H), 7,32 (m, 1H), 7,41 (td, J=7,9, 1,6 Hz, 1H), 7,58 (dd, J=7,9, 1,6 Hz, 1H), 7,65 (d, J=9,5 Hz, 1H), 7,71 (dd, J=9,5, 1,6 Hz, 1H), 8,39 (dd, J=7,9, 1,6 Hz, 1H), 8,46 (s, 1H), 8,76 (s large, 1H), 9,91 (s large, 1H), 11,05 (m étalé, 1H). |
| | Spectre de masse (LC-MS-ES+/-) : m/z 335 [M+H]⁻, m/z 337 [M+H]⁺ |
| **86** | Spectre RMN ¹H (DMSO-d6, δ en ppm) : 6,77 (d, J=2,3 Hz, 1H), 7,15 - 7,41 (m, 3H), 7,70 - 7,95 (m, 3H), 8,10 (m, 1H), 8,56 (s, 1H), 9,08 (s large, 1H), 9,81 (s large, 1H), 13,04 (m étalé, 1H). |
| | Spectre de masse (LC-MS-ES+/-) : m/z 320 [M+H]⁻, m/z 322 [M+H]⁺ |
| **87** | Spectre RMN ¹H (DMSO-d6, δ en ppm) : 6,78 (s large, 1H), 7,20 (td, J=8,0, 1,4 Hz, 1H), 7,36 - 7,47 (m, 1H), 7,58 (dd, J=8,0, 1,4 Hz, 1H), 7,75 (d, J=9,5 Hz, 1H), 7,86 (s large, 1H), 7,91 (d large, J=9,5 Hz, 1H), 8,37 (dd, J=8,0, 1,4 Hz, 1H), 8,59 (s, 1H), 9,09 (s large, 1H), 9,94 (s, 1H), 13,06 (s large, 1H). |
| | Spectre de masse (LC-MS-ES+/-) : m/z 336 [M+H]⁻, m/z 338 [M+H]⁺ |
| **88** | Spectre RMN ¹H (DMSO-d6, δ en ppm) : 6,66 (dd, J=3,4, 1,7 Hz, 1H), 6,93 (tt, J=9,4, 2,4 Hz, 1H), 7,05 (d, J=3,4 Hz, 1H), 7,67 - 7,80 (m, 4H) 7,83 (m, 1H), 8,60 (s, 1H), 8,99 (s large, 1H), 10,74 (s large, 1H). |
| | Spectre de masse (LC-MS-ES+/-) : m/z 338 [M+H]⁻, m/z 340 [M+H]⁺ |
| **89** | Spectre RMN ¹H (DMSO-d6, δ en ppm) : 6,94 (tt, J=9,5, 2,2 Hz, 1H), 7,46 (d, J=0,9 Hz, 1H), 7,75 (m, 2H), 7,81 (d, J=9,6 Hz, 1H), 7,92 (dd, J=9,6, 1,9 Hz, 1H), 8,31 (d, J=0,9 Hz, 1H), 8,71 (s large, 1H), 9,38 (s large, 1H), 10,81 (s large, 1H). |
| | Spectre de masse (LC-MS-ES+/-) : m/z 339 [M+H]⁻, m/z 341 [M+H]⁺ |
| **90** | Spectre RMN ¹H (DMSO-d6, δ en ppm) : 7,16 - 7,39 (m, 3H), 7,46 (d, J=0,8 Hz, 1H), 7,84 (d, J=9,5 Hz, 1H), 7,92 (dd, J=9,6, 1,9 Hz, 1H), 8,05 (m, 1H), 8,31 (d, J=0,8 Hz, 1H), 8,69 (s, 1H), 9,37 (s large, 1H), 9,87 (s large, 1H). |
| | Spectre de masse (LC-MS-ES+/-) : m/z 323 [M+H]⁺ |
| **91** | Spectre RMN ¹H (DMSO-d6, δ en ppm) : 7,18 - 7,33 (m, 2H), 7,46 (s, 1H), 7,74 (m, 1H), 7,84 (d, J=9,5 Hz, 1H), 7,92 (d, J=9,5 Hz, 1H), 8,31 (s, 1H), 8,70 (s, 1H), 9,37 (s, 1H), 10,12 (s, 1H), (signaux larges) |
| | Spectre de masse (LC-MS-ES+/-) : m/z 339 [M+H]⁻, m/z 341 [M+H]⁺ |
| **92** | Spectre RMN ¹H (DMSO-d6, δ en ppm) : 4,49 (d, J=5,6 Hz, 2H), 5,30 (t, J=5,6 Hz, 1H), 6,46 (d, J=3,4 Hz, 1H), 6,92 (tt, J=9,3, 2,4 Hz, 1H), 6,98 (d, J=3,4 Hz, 1H), 7,65 - 7,83 (m, 4H), 8,64 (s, 1H), 8,98 (s large, 1H), 10,74 (s large, 1H). |
| | Spectre de masse (LC-MS-ES+/-) : m/z 368 [M+H]⁻, m/z 370 [M+H]⁺ |
| **93** | Spectre RMN ¹H (DMSO-d6, δ en ppm) : 4,48 (d, J=5,3 Hz, 2H), 5,30 (t, J=5,3 Hz, 1H), 6,47 (d, J=3,3 Hz, 1H), 6,96 - 7,11 (m, 1H), 6,99 (d, J=3,3 Hz, 1H), 7,35 - 7,46 (m, 1H), 7,76 (m, 2H), 7,96 - 8,05 (m, 1H), 8,64 (s, 1H), 8,95 (s large, 1H), 9,85 (s large, 1H). |
| | Spectre de masse (LC-MS-ES+/-) : m/z 368 [M+H]⁻, m/z 370 [M+H]⁺ |
| **94** | Spectre RMN ¹H (DMSO-d6, δ en ppm) : 7,19 - 7,28 (m, 2H), 7,65 - 7,72 (m, 2H), 7,75 - 7,86 (m, 3H), 8,59 (s, 1H), 8,99 (s large, 1H), 10,27 - 11,48 (m étalé, 1H). |
| | Spectre de masse (LC-MS-ES+/-) : m/z 338 [M+H]⁻, m/z 340 [M+H]⁺ |
| **95** | Spectre RMN ¹H (DMSO-d6, δ en ppm) : 7,05 (m, 1H), 7,40 (m, 1H), 7,79 (d, J=9,5 Hz, 1H), 7,94 - 8,06 (m, 2H), 8,71 (s, 1H), 8,73 (s, 1H), 9,32 (s large, 1H), 9,89 (s large, 1H), 14,27 (s large, 1H). |
| | Spectre de masse (LC-MS-ES+/-) : m/z 339 [M+H]⁻, m/z 341 [M+H]⁺ |
| **96** | Spectre RMN ¹H (DMSO-d6, δ en ppm) : 7,20 - 7,30 (m, 2H) 7,73 - 7,82 (m, 2H) 8,00 (dd, J=9,5, 1,7 Hz 1H) 8,71 (s, 2H) 9,32 (s large, 1H) 10,08 (s large, 1H) 14,25 (s large, 1H) |
| | Spectre de masse (LC-MS-ES+/-) : m/z 339 [M+H]⁻, m/z 341 [M+H]⁺ |
| **97** | Spectre RMN ¹H (DMSO-d6, δ en ppm) : 6,93 (tt, J=9,4, 2,2 Hz, 1H), 7,68 - 7,82 (m, 3H), 7,88 (dd, J=9,5, 1,7 Hz, 1H), 8,43 (m étalé, 1H), 8,62 (s, 1H), 9,21 (s large, 1H), 10,76 (s large, 1H), 15,31 (m étalé, 1H). |
| | Spectre de masse (LC-MS-ES+/-) : m/z 339 [M+H]⁻, m/z 341 [M+H]⁺ |
| **98** | Spectre RMN ¹H (DMSO-d6, δ en ppm) : 7,15 - 7,41 (m, 3H), 7,81 (d, J=9,5 Hz, 1H), 7,89 (dd, J=9,5, 1,7 Hz, 1H), 8,10 (m, 1H), 8,43 (m étalé, 1H), 8,61 (s, 1H), 9,20 (s large, 1H), 9,82 (s large, 1H), 15,31 (m étalé, 1H). |
| | Spectre de masse (LC-MS-ES+/-) : m/z 321 [M+H]⁻, m/z 323 \|M+H]⁺ |
| **99** | Spectre RMN ¹H (DMSO-d6, δ en ppm) : 7,05 (m, 1H), 7,40 (m, 1H), 7,82 (d, J=9,5 Hz, 1H), 7,82 (dd, J=9,5, 1,7 Hz, 1H), 8,01 (m, 1H), 8,43 (m étalé, 1H), 8,64 (s, 1H), 9,20 (s large, 1H), 9,88 (s large, 1H), 15,31 (m étalé, 1H). |
| | Spectre de masse (LC-MS-ES+/-) : m/z 339 [M+H]⁻, m/z 341 [M+H]⁺ |
| **100** | Spectre RMN ¹H (DMSO-d6, δ en ppm) : 7,20 (m, 1H), 7,42 (m, 1H), 7,59 (m, 1H), 7,77-7,97 (m, 2H), 8,31 - 8,51 (m, 2H), 8,66 (s, 1H), 9,21 (s, 1H), 9,95 (s, 1H), 15,33 (m étalé, 1H), (signaux larges) |
| | Spectre de masse (LC-MS-ES+/-) : m/z 337 [M+H]⁻, m/z 339 [M+H]⁺ |

Les composés selon l'invention ont fait l'objet d'essais pharmacologiques permettant de déterminer leur effet modulateur sur NOT.

### Evaluation de l'activité in vitro sur cellules N2A

L'activité des composés selon l'invention a été évaluée sur une lignée cellulaire (N2A) exprimant de manière endogène le récepteur de souris Nurr1 et transfectée de manière stable avec l'élément de réponse liant NOT (NBRE) couplé au gène rapporteur luciférase. Les EC₅₀ sont comprises entre 0,01 et 1000 nM. Les essais ont été réalisés selon le mode opératoire décrit ci dessous.
La lignée cellulaire Neuro-2A provient de source commerciale standard (ATCC). Le clone Neuro-2A a été obtenu à partir d'une tumeur spontanée provenant d'une souche de souris A albino par R.J Klebe et col. Cette lignée Neuro-2A est ensuite stablement transfectée avec 8NBRE-luciférase. Les cellules N2A-8NBRE sont cultivées jusqu'à confluence dans des flacons de culture de 75 cm² contenant du DMEM supplémenté par 10% de sérum foetal de veau, 4,5 g/L de glucose et 0,4 mg/ml de Généticine. Après une semaine de culture les cellules sont récupérées par de la trypsine 0,25% pendant 30 secondes puis remises en suspension dans du DMEM sans rouge de phénol contenant 4,5g/L de glucose, 10% de sérum délipidé Hyclone et déposées dans des plaques blanches 96 puits à fond transparent. Les cellules sont déposées à raison de 60.000 par puits dans 75 µL pendant 24 heures avant l'addition des produits. Les produits sont appliqués dans 25µL et incubés 24 heures supplémentaires. Le jour de la mesure, on ajoute à chaque puits un volume équivalent (100µL) de Steadylite, puis on attend 30 minutes pour obtenir une lyse complète des cellules et la production maximale du signal. Les plaques sont ensuite mesurées dans un compteur de luminescence pour microplaques après avoir été scellées par un film adhésif. Les produits sont préparés sous forme de solution stock à 10⁻² M, puis dilués dans 100% de DMSO. Chaque concentration de produit est préalablement diluée dans du milieu de culture avant incubation avec les cellules contenant ainsi 0,625% final de DMSO.
Par exemple, les composés n° 3, 17, 24, 29, 45 et 53 ont montré une EC₅₀ de respectivement 0,9 nM, 26,8 nM, 0,9 nM, 1,1 nM, 1,2 nM et 0,4 nM.

Il apparaît donc que les composés selon l'invention ont un effet modulateur de NOT.

Les composés selon l'invention peuvent donc être utilisés pour la préparation de médicaments pour leur application en thérapeutique dans le traitement ou la prévention de maladies impliquant les récepteurs NOT.

Ainsi, selon un autre de ses aspects, l'invention a pour objet des médicaments qui comprennent un composé de formule (I), ou un sel d'addition de ce dernier à un acide pharmaceutiquement acceptable.

Ces médicaments trouvent leur emploi en thérapeutique, notamment dans le traitement et la prévention des maladies neurodégénératives telles que par exemple la maladie de Parkinson, d'Alzheimer, les tauopathies (ex. la paralysie progressive supranucléaire, la démence fronto temporale, la dégénérescence corticobasale, la maladie de Pick); les traumatismes cérébraux comme l'ischémie et les traumatismes crâniens et l'épilepsie ; les maladies psychiatriques comme la schizophrénie, la dépression, la dépendance à une substance, les troubles du déficit de l'attention et de l'hyperactivité ; les maladies inflammatoires du système nerveux central comme la sclérose en plaque, encéphalite, myélite et encéphalomyélite et autres maladies inflammatoires comme les pathologies vasculaires, l'athérosclérose, les inflammations des articulations, l'arthrose, l'arthrite rhumatoïde ; l'ostéoarthrite, la maladie de Crohn, colite ulcéreuse; les maladies inflammatoires allergiques telle que l'asthme, les maladies auto-immunes comme le diabète de type 1, lupus, sclérodermies, Syndrome de Guillain-Barré, maladie d'Addison et autres maladies immuno-médiées; l'ostéoporose; les cancers.

Ainsi, un objet de la présente invention vise un composé de formule (I) telle que définie précédemment pour le traitement des maladies, troubles et désordres précités.

Selon un autre de ses aspects, la présente invention concerne l'utilisation d'un composé de formule (I) telle que définie précédemment, pour la préparation d'un médicament destiné au traitement et à la prévention de l'un(e) des maladies, troubles ou désordres cités ci-dessus.

Ces composés pourraient être aussi utilisés comme traitement associé à des greffes et/ou transplantations de cellules souches.
Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscarmellose sodique | 6,0 mg |
| Amidon de maïs | 15,0 mg |
| Hydroxypropyl-méthylcellulose | 2,25 mg |
| Stéarate de magnésium | 3,0 mg |

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

Il est entendu que l'ensemble des objets de l'invention définis ci-dessus, notamment médicaments, compositions pharmaceutiques, porte également plus particulièrement sur les sous-ensembles de composés définis précédemment.

## Revendications

1. Composés de formule (I): 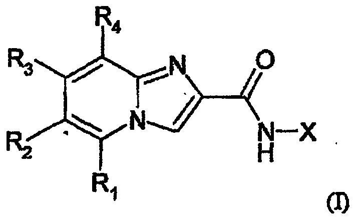 dans laquelle :
X représente un groupe phényle éventuellement substitué par un ou plusieurs groupes choisis indépendamment les uns des autres parmi les atomes ou groupes suivants : halogène, (C₁-C₆)alcoxy, (C₁-C₆)alkyle, NRaRb, cyano, (C₁-C₆)alcoxycarbonyl, les groupes (C₁-C₆)alkyle et (C₁-C₆)alcoxy étant éventuellement substitués par un ou plusieurs atomes d'halogène;
R₁ représente un atome d'hydrogène, un halogène, un groupe (C₁-C₆)alcoxy, un groupe (C₁-C₆)alkyle, un groupe NRaRb; les groupes alkyles et alcoxy pouvant éventuellement être substitués par un ou plusieurs halogène, hydroxy, amino, ou groupe (C₁-C₆)alcoxy ;
R₂ représente un groupe hétérocyclique aromatique, insaturé, partiellement saturé ou totalement saturé, éventuellement substitué par un ou plusieurs groupes choisis indépendamment les uns des autres parmi les atomes ou groupes suivants : hydroxy, (C₁-C₆)alcoxy, halogène, cyano, NRaRb, -CO-R₅, -CO-NR₆R₇, -CO-O-R₈, -NR₉-CO-R₁₀ (C₃-C₇)cycloakyle(C₁-C₆)alkyle, (C₃-C₇)cycloalkyle(C₁-C₆)alcoxy, un groupe (C₁-C₆)alkyle, éventuellement substitué par un ou plusieurs hydroxy ou NRaRb, un groupe oxido ;
R₃ représente un atome d'hydrogène, un groupe (C₁-C₆)alkyle, un groupe (C₁-C₆)alcoxy ou un atome d'halogène ;
R₄ représente un atome d'hydrogène, un groupe (C₁-C₄)alkyle, un groupe (C₁-C₆)alcoxy ou un atome de fluor;
R₅ représente un atome d'hydrogène, un groupe phényle ou un groupe (C₁-C₆)alkyle ;
R₆ et R₇, identiques ou différents, représentent un atome d'hydrogène ou un groupe (C₁-C₆)alkyle ou forment avec l'atome d'azote un cycle de 4 à 7 chaînons incluant éventuellement un autre hétéroatome choisi parmi N, O ou S ;
R₈ représente un groupe (C₁-C₆)alkyle ;
R₉ et R₁₀, identiques ou différents, représentent un atome d'hydrogène ou un groupe (C₁-C₆)alkyle ;
Ra et Rb sont indépendamment l'un de l'autre, hydrogène, (C₁-C₆)alkyle ou forment avec l'atome
d'azote qui les porte un cycle de 4 à 7 chaînons, incluant éventuellement un autre hétéroatome choisi parmi N, O ou S;
un groupe hétérocylique représente un groupe monocyclique aromatique, insaturé, partiellement saturé ou totalement saturé comportant de 5 à 10 atomes dont 1 à 4 hétéroatomes choisi parmi N, O et S ;
à l'exception du *N*-(4-bromophényl)-6-(1-méthyl-2-pipéridinyl)imidazo[1,2-*a*]pyridine-2-carboxamide ;
à l'état de base ou de sel d'addition à un acide.

2. Composés de formule (I) selon la revendication 1, **caractérisé en ce que** :
X représente un groupe phényle et éventuellement susbtitué par un ou plusieurs atomes d'halogène ou groupes cyano ;
à l'état de base ou de sel d'addition à un acide.

3. Composés de formule (I) selon la revendication 1 ou 2, **caractérisé en ce que** :
R₂ représente un groupe pyrrole, furane, thiophène, pyrazole, imidazole, triazole, tetrazole, oxazole, isoxazole, oxadiazole, thiazole, isothiazole, thiadiazole, pyridine, pyrimidine, pyrazine, pyridazine, triazine, éventuellement substitué par un ou plusieurs groupes choisis parmi les atomes ou groupes suivants : hydroxy, (C₁-C₆)alkoxy, oxido, halogène -NRaRb, (C₁-C₆)akyle lui-même éventuellement substitué par un groupe hydroxy,
Ra et Rb sont indépendamment l'un de l'autre, hydrogène, (C₁-C₆)alkyle ;
à l'état de base ou de sel d'addition à un acide.

4. Composés de formule (I) selon la revendication 1 ou 2, **caractérisé en ce que** :
R₂ représente un groupe dioxolane, pyridine, imidazole, pyrazole, triazole, pyrrole, furane, oxazole, indole, imidazoline, thiophène, pyrazine, pyrimidine, thiazole éventuellement substitué par un ou plusieurs groupes choisis parmi les atomes ou groupes suivants : hydroxy, (C₁-C₆)alkoxy, oxido, halogène -NRaRb, (C₁-C₆)alkyle lui-même éventuellement substitué par un groupe hydroxy,
Ra et Rb sont indépendamment l'un de l'autre, hydrogène, (C₁-C₆)alkyle,
à l'état de base ou de sel d'addition à un acide.

5. Composés de formule (I) selon l'une quelconque des revendications précédentes, **caractérisé en ce que** R₁ représente un atome d'hydrogène ou un groupe (C₁-C₆)alkyle ;
à l'état de base ou de sel d'addition à un acide.

6. Composés de formule (I) selon l'une quelconque des revendications précédentes, **caractérisé en ce que** R₃ et R₄ représentent un atome d'hydrogène ;
à l'état de base ou de sel d'addition à un acide.

7. Composés de formule (I) selon l'une quelconque des revendications précédentes, **caractérisé en ce que** :
X représente un groupe phényle éventuellement substitué par un ou plusieurs atomes de fluor, de chlore ou par un groupe cyano ;
R₂ représente un groupe dioxolane, pyridine, imidazole, pyrazole, triazole, pyrrole, furane, oxazole, indole, imidazoline, thiophène, pyrazine, pyrimidine, thiazole, éventuellement substitué par un ou plusieurs groupes hydroxy, méthyle, hydroxyméthyle, méthoxy, halogène, NH₂, (C₁-C₆)alkyle, oxido,
R₁ représente un atome d'hydrogène ou un groupe méthyle ;
R₃ et R₄ représentent un atome d'hydrogène ;
à l'état de base ou de sel d'addition à un acide.

8. Composés de formule (I) selon l'une quelconque des revendications précédentes, **caractérisé en ce qu'**il est choisi parmi :
• 6-(1,3-Dioxolan-2-yl)-*N*-phénylimidazo[1,2-*a*]pyridine-2-carboxamide
• *N*-Phényl-6-(pyridin-3-yl)imidazo[1,2-*a*]pyridine-2-carboxamide
• *N*-Phényl-6-(pyridin-2-yl)imidazo[1,2-*a*]pyridine-2-carboxamide
• 6-[5-(Hydroxyméthyl)pyridin-3-yl]-*N*-phénylimidazo[1,2-*a*]pyridine-2-carboxamide
• 6-[4-(Hydroxyméthyl)pyridin-2-yl]-*N*-phénylimidazo[1,2-*a*]pyridine-2-carboxamide et son chlorhydrate (1:1)
• 6-(6-Aminopyridin-2-yl)-*N*-phénylimidazo[1,2-*a*]pyridine-2-carboxamide et son chlorhydrate (1:1)
• 6-(1*H*-Imidazol-4-yl)-*N*-phénylimidazo[1,2-*a*]pyridine-2-carboxamide et son chlorhydrate (1:1)
• *N*-Phényl-6-(1*H*-pyrazol-3-yl)imidazo[1,2-*a*]pyridine-2-carboxamide
• *N*-Phényl-6-(1*H*-1,2,4-triazol-3-yl)imidazo[1,2-*a*]pyridine-2-carboxamide
• *N*-Phényl-6-(1*H*-pyrrol-3-yl)imidazo[1,2-*a*]pyridine-2-carboxamide
• 6-(2-Méthyl-1*H*-imidazol-4-yl)-*N*-phénylimidazo[1,2-*a*]pyridine-2-carboxamide
• 6-(3-Furyl)-*N*-phénylimidazo[1,2-*a*]pyridine-2-carboxamide
• 6-(1*H*-Imidazol-1-yl)-*N*-phénylimidazo[1,2-*a*]pyridine-2-carboxamide et son trifluoroacetate (1:1)
• 6-(Oxazol-5-yl)-*N*-phénylimidazo[1,2-*a*]pyridine-2-carboxamide
• 6-(2-Amino-thiazol-4-yl)-*N*-phénylimidazo[1,2-*a*]pyridine-2-carboxamide
• 6-(2-Méthyl-1,3-dioxolan-2-yl)-*N*-phénylimidazo[1,2-*a*]pyridine-2-carboxamide
• 6-(4,5-Dihydro-1*H*-imidazol-2-yl)-N-phénylimidazo[1,2-*a*]pyridine-2-carboxamide et son chlorhydrate (1:1)
• 6-(6-Méthoxypyridin-2-yl)-*N*-phénylimidazo[1,2-*a*]pyridine-2-carboxamide
• 5-Méthyl-*N*-phényl-6-(pyridin-2-yl)imidazo[1,2-*a*]pyridine-2-carboxamide
• 6-(2-Amino-1*H*-imidazol-4-yl)-*N*-phénylimidazo[1*,2-a*]pyridine-2-carboxamide et son trifluoroacétate (1:1)
• 6-(2-Amino-thiazol-5-yl)-*N*-phénylimidazo[1,2-*a*]pyridine-2-carboxamide
• 6-(6-Hydroxypyridin-2-yl)-*N*-phénylimidazo[1,2-*a*]pyridine-2-carboxamide
• *N*-Phényl-6-(1*H*-1,2,3-triazol-4-yl)imidazo[1,2-*a*]pyridine-2-carboxamide
• *N*-(3,5-Difluorophényl)-6-(furan-3-yl)imidazo[1,2-*a*]pyridine-2-carboxamide
• *N*-(3-Fluorophényl)-6-(furan-3-yl)imidazo[1,2-*a*]pyridine-2-carboxamide
• 5-(2-Furyl)-*N*-phénylimidazo[1,2-*a*]pyridine-2-carboxamide
• 6-[6-(Hydroxyméthyl)pyridin-2-yl]-*N*-phénylimidazo[1,2-*a*]pyridine-2-carboxamide et son chlorhydrate (1:1)
• 6-(1-Oxidopyridin-3-yl)-*N*-phénylimidazo[1,2-*a*]pyridine-2-carboxamide et son chlorhydrate (1:1)
• *N*-Phényl-6-(1*H*-pyrazol-4-yl)imidazo[1,2-*a*]pyridine-2-carboxamide
• 6-(1*H*-Imidazol-2-yl)-*N*-phénylimidazo[1,2-*a*]pyridine-2-carboxamide, chlorhydrate (1:1)
• 6-(1-Méthyl-1*H*-imidazol-4-yl)-*N*-phénylimidazo[1,2-*a*]pyridine-2-carboxamide
• 6-(Oxazol-2-yl)-*N*-phénylimidazo[1,2-*a*]pyridine-2-carboxamide
• *N*-(3,5-Difluorophényl)-6-(pyridin-2-yl)imidazo[1,2-*a*]pyridine-2-carboxamide
• *N*-Phényl-6-(pyridin-4-yl)imidazo[1,2-*a*]pyridine-2-carboxamide
• 6-(1H-Indol-3-yl)-*N*-phénylimidazo[1,2-*a*]pyridine-2-carboxamide
• *N*-Phényl-6-(thiophén-2-yl)imidazo[1,2-*a*]pyridine-2-carboxamide
• *N*-Phényl-6-(pyrazin-2-yl)imidazo[1,2-*a*]pyridine-2-carboxamide
• 6-(1-Oxidopyridin-2-yl)-*N*-phénylimidazo[1,2-*a*]pyridine-2-carboxamide
• *N*-Phényl-6-(pyrimidin-5-yl)imidazo[1,2-*a*]pyridine-2-carboxamide
• *N*-Phényl-6-(thièn-3-yl)imidazo[1,2-*a*]pyridine-2-carboxamide
• 6-(5-Fluoro-2-furyl)-*N*-phénylimidazo[1,2-*a*]pyridine-2-carboxamide
• *N*-(3-Fluorophényl)-6-(pyridin-2-yl)imidazo[1,2-*a*]pyridine-2-carboxamide
• *N*-(2-Fluorophényl)-6-(pyridin-2-yl)imidazo[1,2-*a*]pyridine-2-carboxamide
• *N*-(2,5-Difluorophényl)-6-(pyridin-2-yl)imidazo[1,2-*a*]pyridine-2-carboxamide
• *N*-(2,3-Difluorophényl)-6-(pyridin-2-yl)imidazo[1,2-*a*]pyridine-2-carboxamide
• 6-(6-Aminopyridin-2-yl)-*N*-(3-fluorophényl)imidazo[1,2-*a*]pyridine-2-carboxamide
• *N*-(3-Fluorophényl)-6-(1*H*-pyrrol-3-yl)imidazo[1,2-*a*]pyridine-2-carboxamide
• *N*-(3-Fluorophényl)-6-(1*H*-pyrazol-3-yl)imidazo[1,2-*a*]pyridine-2-carboxamide
• *N*-(3-Fluorophényl)-6-(furan-2-yl)imidazo[1,2-*a*]pyridine-2-carboxamide
• *N*-(3-Fluorophényl)-6-(oxazol-2-yl)imidazo[1,2-*a*]pyridine-2-carboxamide
• *N*-Phényl-6-(pyrimidin-2-yl)imidazo[1,2-a]pyridine-2-carboxamide
• *N*-(3-Fluorophényl)-6-(1*H*-imidazol-4-yl)imidazo[1,2-*a*]pyridine-2-carboxamide
• *N*-(3-Fluorophényl)-6-(1*H*-1,2,4-triazol-3-yl)imidazo[1,2-*a*]pyridine-2-carboxamide
• 6-(2-Amino-thiazol-4-yl)-*N*-(3-fluorophényl)imidazo[1,2-*a*]pyridine-2-carboxamide
• *N*-(3-Fluorophényl)-6-(1*H*-1,2,3-triazol-4-yl)imidazo[1,2-*a*]pyridine-2-carboxamide
• *N*-(3-Fluorophényl)-6-(1*H*-pyrazol-4-yl)imidazo[1,2-*a*]pyridine-2-carboxamide
• *N*-(3-Fluorophényl)-6-[5-(hydroxyméthyl)furan-2-yl]imidazo[1,2-*a*]pyridine-2-carboxamide
• 6-[2-(Hydroxyméthyl)-1*H*-imidazol-4-yl]-*N*-phénylimidazo[1,2-*a*]pyridine-2-carboxamide
• *N*-(3,5-Difluorophényl)-6-(6-méthylpyridin-2-yl)imidazo[1,2-*a*]pyridine-2-carboxamide
• *N*-(2-Chlorophényl)-6-(pyridin-2-yl)imidazo[1,2-*a*]pyridine-2-carboxamide
• *N*-(3,5-Difluorophényl)-6-(1*H*-pyrrol-3-yl)imidazo[1,2-*a*]pyridine-2-carboxamide
• *N*-(2-Fluorophényl)-6-(1*H*-pyrrol-3-yl)imidazo[1,2-*a*]pyridine-2-carboxamide
• *N-*(2,5-Difluorophényl)-6-(1*H*-pyrrol-3-yl)imidazo[1,2-*a*]pyridine-2-carboxamide
• *N*-(2,3-Difluorophényl)-6-(1*H*-pyrrol-3-yl)imidazo[1,2-*a*]pyridine-2-carboxamide
• *N*-(3,5-Difluorophényl)-6-(1*H*-pyrazol-3-yl)imidazo[1,2-*a*]pyridine-2-carboxamide
• *N*-(2-Fluorophényl)-6-(furan-2-yl)imidazo[1,2-*a*]pyridine-2-carboxamide
• *N*-(2,5-Difluorophényl)-6-(furan-2-yl)imidazo[1,2-*a*]pyridine-2-carboxamide
• *N*-(2,3-Difluorophényl)-6-(furan-2-yl)imidazo[1,2-*a*]pyridine-2-carboxamide
• *N*-(2-Chlorophényl)-6-(furan-2-yl)imidazo[1,2-*a*]pyridine-2-carboxamide
• *N*-(2,5-Difluorophényl)-6-(oxazol-2-yl)imidazo[1,2-*a*]pyridine-2-carboxamide
• *N*-(2-Chlorophényl)-6-(oxazol-2-yl)imidazo[1,2-*a*]pyridine-2-carboxamide
• *N*-(2-Fluorophényl)-6-(furan-3-yl)imidazo[1,2-*a*]pyridine-2-carboxamide
• *N*-(2,5-Difluorophénl)-6-(furan-3-yl)imidazo[1,2-*a*]pyridine-2-carboxamide
• *N*-(2,3-Difluorophényl)-6-(furan-3-yl)imidazo[1,2-*a*]pyridine-2-carboxamide
• *N*-(2-Chlorophényl)-6-(furan-3-yl)imidazo[1,2-*a*]pyridine-2-carboxamide
• *N*-(2,3-Difluorophényl)-6-[5-(hydroxyméthyl)furan-2-yl]imidazo[1,2-*a*]pyridine-2-carboxamide
• *N*-(2-Fluorophényl)-6-(1*H*-1,2,4-triazol-3-yl)imidazo[1,2-*a*]pyridine-2-carboxamide
• *N*-(2-Chlorophényl)-6-(1*H*-1,2,4-triazol-3-yl)imidazo[1,2-*a*]pyridine-2-carboxamide
• *N*-(2-Cyanophényl)-6-(pyridin-2-yl)imidazo[1,2-*a*]pyridine-2-carboxamide
• 6-(6-Aminopyridin-2-yl)-*N*-(3,5-difluorophényl)imidazo[1,2-*a*]pyridine-2-carboxamide et son dichlorhydrate
• 6-(6-Aminopyridin-2-yl)-*N*-(2-fluorophényl)imidazo[1,2-*a*]pyridine-2-carboxamide et son dichlorhydrate
• 6-(6-Aminopyridin-2-yl)-*N*-(2,5-difluorophényl)imidazo[1,2-*a*]pyridine-2-carboxamide et son dichlorhydrate
• 6-(6-Aminopyridin-2-yl)-*N*-(2,3-difluorophényl)imidazo[1,2-*a*]pyridine-2-carboxamide et son dichlorhydrate
• 6-(6-Aminopyridin-2-yl)-*N*-(2-chlorophényl)imidazo[1,2-*a*]pyridine-2-carboxamide et son dichlorhydrate
• *N*-(2-Chlorophényl)-6-(1*H*-pyrrol-3-yl)imidazo[1,2-*a*]pyridine-2-carboxamide
• *N*-(2-Fluorophényl)-6-(1*H*-pyrazol-3-yl)imidazo[1,2-*a*]pyridine-2-carboxamide
• *N*-(2-Chlophényl)-6-(1*H*-pyrazol-3-yl)imidazo[1,2-*a*]pyridine-2-carboamide
• *N*-(3,5-Difluorophényl)-6-(furan-2-yl)imidazo[1,2-*a*]pyridine-2-carboxamide
• *N*-(3,5-Difluorophényl)-6-(oxazol-2-yl)imidazo[1,2-*a*]pyridine-2-carboxamide
• *N*-(2-Fluorophényl)-6-(oxazal-2-yl)imidazo[1,2-*a*]pyridine-2-carboxamide
• *N*-(2,3-Difluorophényl)-6-(oxazol-2-yl)imidazo[1,2-*a*]pyridine-2-carboxamide
• *N*-(3,5-Difluorophényl)-6-[5-(hydroxyméthyl)furan-2-yl]imidazo[1,2-*a*]pyridine-2-carboxamide
• *N*-(2,5 Difluorophényl)-6-[5-(hydroxyméthyl)furan-2-yl]imidazo[1,2-*a*]pyridine-2-carboxamide
• *N*-(2,3-Difluorophényl)-6-(1*H*-imidazol-4-yl)imidazo[1,2-*a*]pyridine-2-carboxamide
• *N*-(2,5-Difluorophényl)-6-(1*H*-1,2,4-triazol-3-yl)imidazo[1,2-*a*]pyridine-2-carboxamide
• *N*-(2,3-Difluorophényl)-6-(1*H*-1,2,4-triazol-3-yl)imidazo[1,2-*a*]pyridine-2-carboxamide
• *N*-(3,5-Difluorophényl)-6-(1*H*-1,2,3-triazol-4-yl)-imidazo[1,2-*a*]pyridine-2-carboxamide
• *N*-(2-Fluorophényl)-6-(1*H*-1,2,3-triazol-4-yl)imidazo[1,2-*a*]pyridine-2-carboxamide
• *N*-(2,5-Difluorophényl)-6-(1*H*-1,2,3-triazol-4-yl)-imidazo[1,2-*a*]pyridine-2-carboxamide
• *N*-(2-Chlorophényl)-6-(1*H*-1,2,3-triazol-4-yl)imidazo[1,2-*a*]pyridine-2-carboxamide ;
et leurs sels d'addition à un acide.

9. Médicament, **caractérisé en ce qu'**il comprend un composé de formule (I) selon l'une quelconque des revendications 1 à 8, ou un sel d'addition de ce composé à un acide pharmaceutiquement acceptable.

10. Composition pharmaceutique, **caractérisée en ce qu'**elle comprend un composé de formule (I) selon l'une quelconque des revendications 1 à 8, ou un sel pharmaceutiquement acceptable, de ce composé, ainsi qu'au moins un excipient pharmaceutiquement acceptable.

11. Utilisation d'un composé de formule (I) selon l'une quelconque des revendications 1 à 8, pour la préparation d'un médicament destiné au traitement et à la prévention des maladies neurodégénératives.

12. Utilisation d'un composé de formule (I) selon l'une quelconque des revendications 1 à 8, pour la préparation d'un médicament destiné au traitement et à la prévention des traumatismes cérébraux et de l'épilepsie.

13. Utilisation d'un composé de formule (I) selon l'une quelconque des revendications 1 à 8, pour la préparation d'un médicament destiné au traitement et à la prévention des maladies psychiatriques.

14. Utilisation d'un composé de formule (I) selon l'une quelconque des revendications 1 à 8, pour la préparation d'un médicament destiné au traitement et à la prévention des maladies inflammatoires.

15. Utilisation d'un composé de formule (I) selon l'une quelconque des revendications 1 à 8, pour la préparation d'un médicament destiné au traitement et à la prévention de l'ostéoporose.

16. Utilisation d'un composé de formule (I) selon l'une quelconque des revendications 1 à 8, pour la préparation d'un médicament destiné au traitement et à la prévention des cancers.

17. Utilisation d'un composé de formule (I) selon l'une quelconque des revendications 1 à 8, pour la préparation d'un médicament destiné au traitement et à la prévention de la maladie de Parkinson, d'Alzheimer, des tauopathies, de la sclérose en plaque.

18. Utilisation d'un composé de formule (I) selon l'une quelconque des revendications 1 à 8, pour la préparation d'un médicament destiné au traitement et à la prévention de la schizophrénie, la dépression, la dépendance à une substance les troubles du déficit de l'attention et de l'hyperactivité.

19. Composés
6-Bromo-N-phénylimidazo[1,2-*a*]pyridine-2-carboxamide
6-Iodo-N-phénylimidazo[1,2-*a*]pyridine-2-carboxamide
N-Phényl-6-cyano-imidazo[1,2-*a*]pyridine-2-carboxamide
6-Triméthylstannyl-N-phénylimidazo[1,2-*a*]pyridine-2-carboxamide
N-phényl-6-(4,4,5,5-tétraméthyl-1,3,2-dioxaborotan-2-yl)-imidazo[1,2-*a*]pyridine-2-carboxamide et son bromhydrate (1:1)
Chlorhydrate (1:1) de l'acide 2-phénylcarbamoyl-imidazo[1,2-*a*]pyridine-6-boronique
N-phényl-6-vinylimidazo[1,2-*a*]pyridine-2-carboxamide
6-Formyl-N-phénylimidazo[1,2-*a*]pyridine-2-carboxamide
6-(2-Bromo-acétyl)-N-phényl-imidazo[1,2-*a*]pyridine-2-carboxamide
6-(1-Ethoxyvinyl)-N-phénylimidazo[1,2-*a*]pyridine-2-carboxamide
N-(3,5-difluorophényl)-6-iodoimidazo[1,2-*a*]pyridine-2-carboxamide
Acide 6-(6-{[(1,1-diméthyléthoxy)carbonyl]amino}pyridin-2-yl)imidazo[1,2-*a*]pyridine-2-carboxylique
6-(6-Aminopyridin-2-yl)imidazo[1,2-*a*]pyridine-2-carboxylate d'éthyle
6-(6-{[(1,1-Diméthyléthoxy)carbonyl]amino}pyridin-2-yl)imidazo[1,2-*a*]pyridine-2-carboxylate d'éthyle
6-(6-{bis[(1,1-diméthyléthoxy)carbonyl]amino}pyridin-2-yl)imidazo[1,2-*a*]pyridine-2-carboxylate d'éthyle
Acide 6-(pyridin-2-yl)imidazo[1,2-*a*]pyridine-2-carboxylique
(6-Pyridin-2-yl)imidazo[1,2-*a*]pyridine-2-carboxylate d'éthyle
Acide 6-(1-triphénylméthyl-1*H*-imidazol-4-yl)imidazo[1,2-a]pyridine-2-carboxylique
6-(1-Triphénylméthyl-1H-imidazol-4-yl)imidazo[1*,2-a*]pyridine-2-carboxylate d'éthyle
Acide 6-(2-{[(1,1-diméthyléthoxy)carbonyl]amino}-thiazol-4-yl)imidazo[1,2-*a*]pyridine-2-carboxylique
6-(2-{[(1,1-Diméthyléthoxy)carbonyl]amino}-thiazol-4-yl)imidazo[1*,2-a*]pyridine-2-carboxylate d'éthyle
Acide 6-(1*H*-pyrrol-3-yl)imidazo[1,2-a]pyridine-2-carboxylique et son chlorhydrate (1:1)
6-[1-(Triisopropylsilyl)-1*H*-pyrrol-3-yl]imidazo[1,2-a]pyridine-2-carboxylate d'éthyle
Acide 6-(1*H*-pyrazol-3-yl)imidazo[1,2-*a*]pyridine-2-carboxylique
6-(1*H*-pyrazol-3-yl)imidazo[1,2-*a*]pyridine-2-carboxylate d'éthyle
Acide 6-(1*H*-pyrazol-4-yl)imidazo[1,2-*a*]pyridine-2-carboxylique
6-(1*H*-pyrazol-4-yl)imidazo[1,2-*a*]pyridine-2-carboxylate d'éthyle
Acide 6-(furan-2-yl)imidazo[1,2-*a*]pyridine-2-carboxylique
6-(Furan-2-yl)imidazo[1,2-*a*]pyridine-2-carboxylate d'éthyle
Acide 6-(furan-3-yl)imidazo[1,2-*a*]pyridine-2-carboxylique
6-(Furan-3-yl)imidazo[1,2-*a*]pyridine-2-carboxylate d'éthyle
Acide 6-[5-(hydroxyméthyl)furan-2-yl]imidazo[1,2-*a*]pyridine-2-carboxylique
6-(5-Formylfuran-2-yl)imidazo[1,2-*a*]pyridine-2-carboxylate d'éthyle
6-[5-(Hydroxyméthyl)furan-2-yl]imidazo[1,2-*a*]pyridine-2-carboxylate d'éthyle
Acide 6-(thiophèn-3-yl)imidazo[1,2-*a*]pyridine-2-carboxylique
6-(Thiophén-3-yl)imidazo[1,2-*a*]pyridine-2-carboxylate d'éthyle
Acide 6-(oxazol-2-yl)imidazo[1,2-*a*]pyridine-2-carboxylique
6-(Oxazol-2-yl)imidazo[1,2-*a*]pyridine-2-carboxylate d'éthyle
Acide 6-(1*H*-1,2,4-triazol-3-yl)imidazo[1,2-*a*]pyridine-2-carboxylique
6-[Ethoxy(imino)méthyl]imidazo[1,2-*a*]pyridine-2-carboxylate d'éthyle
6-[Hydrazino(imino)méthyl]imidazo[1,2-*a*]pyridine-2-carboxylate d'éthyle
6-(1*H*-1,2,4-triazol-3-yl)imidazo[1,2-*a*]pyridine-2-carboxylate d'éthyle
Acide 6-(1*H*-1,2,3-triazol-4-yl)imidazo[1,2-a]pyridine-2-carboxylique
6-(1*H*-1,2,3-triazol-4-yl)imidazo[1,2-*a*]pyridine-2-carboxylate d'éthyle
Acide 6-(1*H*-1,2,3-triazol-4-yl)imidazo[1,2-*a*]pyridine-2-carboxylique
Bromhydrate (1:1) de 6-(4,4,5,5-tétraméthyl-1,3,2-dioxaborolan-2-yl)imidazo[1,2-*a*]pyridine-2-carboxylate d'éthyle
Acide 2-éthoxycarbonyl-imidazo[1,2-*a*]pyridine-6-boronique.

20. Utilisation des composés selon la revendication 19, pour la préparation de composés de formule générale (I) telle que définie dans l'une des revendications 1 à 8.

## Patentansprüche

1. Verbindungen der Formel (I): 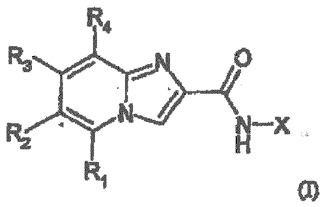 worin
X für eine Phenylgruppe steht, die gegebenenfalls durch eine oder mehrere Gruppen substituiert ist, die unabhängig voneinander aus den folgenden Atomen bzw. Gruppen ausgewählt sind: Halogen, (C₁-C₆) -Alkoxy, (C₁-C₆)-Alkyl, NRaRb, Cyano, (C₁-C₆) -Alkoxycarbonyl, wobei die (C₁-C₆) - Alkyl- und (C₁-C₆)-Alkoxygruppen gegebenenfalls durch ein oder mehrere Halogenatome substituiert sind;
R₁ für ein Wasserstoffatom, ein Halogen, eine (C₁-C₆)-Alkoxygruppe, eine (C₁-C₆)-Alkylgruppe oder eine NRaRb-Gruppe steht; wobei die Alkyl- und Alkoxygruppen gegebenenfalls durch ein bzw. eine oder mehrere Halogenatome, Hydroxygruppen, Aminogruppen oder (C₁-C₆)-Alkoxygruppen substituiert sind;
R₂ für eine ungesättigte, teilweise gesättigte oder vollständig gesättigte aromatische heterocyclische Gruppe steht, die gegebenenfalls durch eine oder mehrere Gruppen substituiert ist, die unabhängig voneinander aus den folgenden Atomen bzw. Gruppen ausgewählt sind: Hydroxy, (C₁-C₆)-Alkoxy, Halogen, Cyano, NRaRb, -CO-R₅, -CO-NR₆R₇, -CO-O-R₈, -NR₉-CO-R₁₀, (C₃-C₇) - Cycloalkyl- (C₁-C₆) -alkyl, (C₃-C₇) -Cycloalkyl- (C₁-C₆)-alkoxy, einer (C₁-C₆) -Alkylgruppe, die gegebenenfalls durch ein oder mehrere Hydroxy oder NRaRb substituiert ist, einer Oxidogruppe;
R₃ für ein Wasserstoffatom, eine (C₁-C₆)-Alkylgruppe, eine (C₁-C₆) -Alkoxygruppe oder ein Halogenatom steht;
R₄ für ein Wasserstoffatom, eine (C₁-C₄)-Alkylgruppe, eine (C₁-C₄)-Alkoxygruppe oder ein Fluoratom steht;
R₅ für ein Wasserstoffatom, eine Phenylgruppe oder eine (C₁-C₆)-Alkylgruppe steht;
R₆ und R₇ gleich oder verschieden sind und für ein Wasserstoffatom oder eine (C₁-C₆)-Alkylgruppe stehen oder mit dem Stickstoffatom einen 4- bis 7-gliedrigen Ring, der gegebenenfalls ein weiteres aus N, O und S ausgewähltes Heteroatom enthält, bilden;
R₈ für eine (C₁-C₆) -Alkylgruppe steht;
R₉ und R₁₀ gleich oder verschieden sind und für ein Wasserstoffatom oder eine (C₁-C₆)-Alkylgruppe stehen;
Ra und Rb unabhängig voneinander für Wasserstoff oder (C₁-C₆)-Alkyl stehen oder mit dem Stickstoffatom, das sie trägt, einen 4- bis 7-gliedrigen Ring, der gegebenenfalls ein weiteres aus N, O oder S ausgewähltes Heteroatom enthält, bilden;
eine heterocyclische Gruppe für eine ungesättigte, teilweise gesättigte oder vollständig gesättigte aromatische monocyclische Gruppe mit 5 bis 10 Atomen einschließlich 1 bis 4 aus N, O und S ausgewählten Heteroatomen steht;
mit Ausnahme von *N*-(4-Bromphenyl)-6-(1-methyl-2-piperidinyl)imidazo[1,2-*a*]pyridin-2-carboxamid;
in Basen- oder Säueradditionssalzform.

2. Verbindungen der Formel (I) nach Anspruch 1, **dadurch gekennzeichnet, dass**:
X für eine Phenylgruppe steht und gegebenenfalls durch ein oder mehrere Halogenatome oder Cyanogruppen substituiert ist;
in Basen- oder Säueradditionssalzform.

3. Verbindungen der Formel (I) nach Anspruch 1 oder 2, **dadurch gekennzeichnet, dass**:
R₂ für eine Pyrrol-, Furan-, Thiophen-, Pyrazol-, Imidazol-, Triazol-, Tetrazol-, Oxazol-, Isoxazol-, Oxadiazol-, Thiazol-, Isothiazol-, Thiadiazol-, Pyridin-, Pyrimidin-, Pyrazin-, Pyridazin- oder Triazingruppe steht, die gegebenenfalls durch eine oder mehrere Gruppen substituiert ist, die aus den folgenden Atomen bzw. Gruppen ausgewählt sind: Hydroxy, (C₁-C₆)-Alkoxy, Oxido, Halogen, -NRaRb, (C₁-C₆)-Alkyl, das selbst gegebenenfalls durch eine Hydroxylgruppe substituiert ist,
Ra und Rb unabhängig voneinander für Wasserstoff oder (C₁-C₆)-Alkyl stehen;
in Basen- oder Säueradditionssalzform.

4. Verbindungen der Formel (I) nach Anspruch 1 oder 2, **dadurch gekennzeichnet, dass**:
R₂ für eine Dioxolan-, Pyridin-, Imidazol-, Pyrazol-, Triazol-, Pyrrol-, Furan-, Oxazol-, Indol-, Imidazolin-, Thiophen-, Pyrazin-, Pyrimidin- oder Thiazolgruppe steht, die gegebenenfalls durch eine oder mehrere Gruppen substituiert ist, die aus den folgenden Atomen bzw. Gruppen ausgewählt sind: Hydroxy, (C₁-C₆) Alkoxy, Oxido, Halogen, -NRaRb, (C₁-C₆) -Alkyl, das selbst gegebenenfalls durch eine Hydroxylgruppe substituiert ist,
Ra und Rb unabhängig voneinander für Wasserstoff oder (C₁-C₆)-Alkyl stehen;
in Basen- oder Säueradditionssalzform.

5. Verbindungen der Formel (I) nach einem der vorhergehenden Ansprüche, **dadurch gekennzeichnet, dass** R₁ für ein Wasserstoffatom oder eine (C₁-C₆) - Alkylgruppe steht;
in Basen- oder Säueradditionssalzform.

6. Verbindungen der Formel (I) nach einem der vorhergehenden Ansprüche, **dadurch gekennzeichnet, dass** R₃ und R₄ für ein Wasserstoffatom stehen;
in Basen- oder Säueradditionssalzform.

7. Verbindungen der Formel (I) nach einem der vorhergehenden Ansprüche, **dadurch gekennzeichnet, dass**:
X für eine Phenylgruppe steht, die gegebenenfalls durch ein oder mehrere Fluor- oder Chloratome oder durch eine Cyanogruppe substituiert ist;
R₂ für eine Dioxolan-, Pyridin-, Imidazol-, Pyrazol-, Triazol-, Pyrrol-, Furan-, Oxazol-, Indol-, Imidazolin-, Thiophen-, Pyrazin-, Pyrimidin- oder Thiazolgruppe steht, die gegebenenfalls durch eine oder mehrere Hydroxy-, Methyl-, Hydroxymethyl-, Methoxy-, Halogen-, NH₂-, (C₁-C₆)-Alkyl-, Oxidogruppen substituiert ist;
R₁ für ein Wasserstoffatom oder eine Methylgruppe steht;
R₃ und R₄ für ein Wasserstoffatom stehen;
in Basen- oder Säueradditionssalzform.

8. Verbindungen der Formel (I) nach einem der vorhergehenden Ansprüche, **dadurch gekennzeichnet, dass** sie ausgewählt sind aus:
• 6-(1,3-Dioxolan-2-yl)-*N*-phenylimidazo[1,2-*a*]-pyridin-2-carboxamid
• *N*-Phenyl-6-(pyridin-3-yl)imidazo[1,2-*a*]pyridin-2-carboxamid
• *N*-Phenyl-6-(pyridin-2-yl)imidazo[1,2-*a*]pyridin-2-carboxamid
• 6-[5-(Hydroxymethyl)pyridin-3-yl]-*N-*phenylimidazo[1,2-*a*]pyridin-2-carboxamid
• 6-[4-(Hydroxymethyl)pyridin-2-yl]-*N-*phenylimidazo[1,2-*a*]pyridin-2-carboxamid und dessen Hydrochlorid (1:1)
• 6-(6-Aminopyridin-2-yl)-*N*-phenylimidazo[1,2-*a*]-pyridin-2-carboxamid und dessen Hydrochlorid (1:1)
• 6-(1*H*-Imidazol-4-yl)-*N*-phenylimidazo[1,2-*a*]pyridin-2-carboxamid und dessen Hydrochlorid (1:1)
• *N*-Phenyl-6-(1*H*-pyrazol-3-yl)imidazo[1,2-*a*]-pyridin-2-carboxamid
• *N*-Phenyl-6-(1*H*-1,2,4-triazol-3-yl)imidazo[1,2-*a*]-pyridin-2-carboxamid
• *N*-Phenyl-6-(1*H*-pyrrol-3-yl)imidazo[1,2-*a*]pyridin-2-carboxamid
• 6-(2-Methyl-1*H*-imidazol-4-yl)-*N-*phenylimidazo[1,2-*a*]pyridin-2-carboxamid
• 6-(3-Furyl)-*N*-phenylimidazo[1,2-*a*]pyridin-2-carboxamid
• 6-(1*H*-Imidazol-1-yl)-*N*-phenylimidazo[1,2-a]pyridin-2-carboxamid und dessen Trifluoracetat (1:1)
• 6-(Oxazol-5-yl)-*N*-phenylimidazo[1,2-*a*]pyridin-2-carboxamid
• 6-(2-Aminothiazol-4-yl)-*N*-phenylimidazo[1,2-*a*]-pyridin-2-carboxamid
• 6-(2-Methyl-1,3-dioxolan-2-yl)-*N-*phenylimidazo[1,2-*a*]pyridin-2-carboxamid
• 6-(4,5-Dihydro-1*H*-imidazol-2-yl)-*N-*phenylimidazo[1,2-*a*]pyridin-2-carboxamid und dessen Hydrochlorid (1:1)
• 6-(6-Methoxypyridin-2-yl)-*N*-phenylimidazo[1,2-*a*]-pyridin-2-carboxamid
• 5-Methyl-*N*-phenyl-6-(pyridin-2-yl)imidazo[1,2-*a*]-pyridin-2-carboxamid
• 6-(2-Amino-1*H*-imidazol-4-yl)-*N*-phenylimidazo[1,2-*a*]pyridin-2-carboxamid und dessen Trifluoracetat (1:1)
• 6-(2-Aminothiazol-5-yl)-*N*-phenylimidazo[1,2-*a*]-pyridin-2-carboxamid
• 6-(6-Hydroxypyridin-2-yl)-*N*-phenylimidazo[1,2-*a*]-pyridin-2-carboxamid
• *N*-Phenyl-6-(1*H*-1,2,3-triazol-4-yl)imidazo[1,2-*a*]-pyridin-2-carboxamid
• *N*-(3,5-Difluorphenyl)-6-(furan-3-yl)imidazo[1,2-*a*]pyridin-2-carboxamid
• *N*-(3-Fluorphenyl)-6-(furan-3-yl)imidazo[1,2-*a*]-pyridin-2-carboxamid
• 6-(2-Furyl)-*N*-phenylimidazo[1,2-*a*]pyridin-2-carboxamid
• 6-[6-(Hydroxymethyl)pyridin-2-yl]-*N-*phenylimidazo[1,2-*a*]pyridin-2-carboxamid und dessen Hydrochlorid (1:1)
• 6-(1-Oxidopyridin-3-yl)-*N*-phenylimidazo[1,2-*a*]-pyridin-2-carboxamid und dessen Hydrochlorid (1:1)
• *N*-Phenyl-6-(1*H*-pyrazol-4-yl)imidazo[1,2-*a*]-pyridin-2-carboxamid
• 6-(1*H*-Imidazol-2-yl)-*N*-phenylimidazo[1,2-*a*]-pyridin-2-carboxamid-hydrochlorid (1:1)
• 6-(1-Methyl-1*H*-imidazol-4-yl)-*N-*phenylimidazo[1,2-*a*]pyridin-2-carboxamid
• 6-(Oxazol-2-yl)-*N*-phenylimidazo[1,2-*a*]pyridin-2-carboxamid
• *N*-(3,5-Difluorphenyl)-6-(pyridin-2-yl)imidazo[1,2-*a*]pyridin-2-carboxamid
• *N*-phenyl-6-(pyridin-4-yl) imidazo[1,2-*a*]pyridin-2-carboxamid
• 6-(1*H*-Indol-3-yl)-*N*-phenylimidazo[1,2-*a*]pyridin-2-carboxamid
• *N*-Phenyl-6-(thiophen-2-yl)imidazo[1,2-*a*]pyridin-2-carboxamid
• *N*-phenyl-6-(pyrazin-2-yl)imidazo[1,2-*a*]pyridin-2-carboxamid
• 6-(1-Oxidopyridin-2-yl)-*N*-phenylimidazo[1,2-*a*] - pyridin-2-carboxamid
• *N*-Phenyl-6-(pyrimidin-5-yl)imidazo[1,2-*a*]pyridin-2-carboxamid
• *N*-Phenyl-6-(thien-3-yl)imidazo[1,2-*a*]pyridin-2-carboxamid
• 6-(5-Fluor-2-furyl)-*N*-phenylimidazo[1,2-*a*]-pyridin-2-carboxamid
• *N*-(3-Fluorphenyl)-6-(pyridin-2-yl)imidazo[1,2-*a*]-pyridin-2-carboxamid
• *N*-(2-Fluorphenyl)-6-(pyridin-2-yl)imidazo[1,2-*a*]-pyridin-2-carboxamid
• *N*-(2,5-Difluorphenyl)-6-(pyridin-2-yl)imidazo-[1,2-*a*]pyridin-2-carboxamid
• *N*-(2,3-Difluorphenyl)-6-(pyridin-2-yl)imidazo-[1,2-*a*]pyridin-2-carboxamid
• 6-(6-Aminopyridin-2-yl)-*N*-(3-fluorphenyl)imidazo-[1,2-*a*]pyridin-2-carboxamid
• *N*-(3-Fluorphenyl)-6-(1*H*-pyrrol-3-yl)imidazo[1,2-*a*]pyridin-2-carboxamid
• *N*-(3-Fluorphenyl)-6-(1*H*-pyrazol-3-yl)imidazo[1,2-*a*]pyridin-2-carboxamid
• *N*-(3-Fluorphenyl)-6-(furan-2-yl)imidazo[1,2-*a*]pyridin-2-carboxamid
• *N*-(3-Fluorphenyl)-6-(oxazol-2-yl)imidazo[1,2-*a*]-pyridin-2-carboxamid
• *N*-Phenyl-6-(pyrimidin-2-yl)imidazo[1,2-*a*]pyridin-2-carboxamid
• *N*-(3-Fluorphenyl)-6-(1*H*-imidazol-4-yl)imidazo-[1,2-*a*]pyridin-2-carboxamid
• *N*-(3-Fluorphenyl)-6-(1*H*-1,2,4-triazol-3-yl)-imidazo[1,2-*a*]pyridin-2-carboxamid
• 6-(2-Aminothiazol-4-yl)-*N*-(3-fluorphenyl)imidazo-[1,2-*a*]pyridin-2-carboxamid
• *N*-(3-Fluorphenyl)-6-(1*H*-1,2,3-triazol-4-yl)-imidazo[1,2-*a*]pyridin-2-carboxamid
• *N*-(3-Fluorphenyl)-6-(1*H*-pyrazol-4-yl)imidazo[1,2-*a*]pyridin-2-carboxamid
• *N*-(3-Fluorphenyl)-6-[5-(hydroxymethyl)furan-2-yl]imidazo[1,2-*a*]pyridin-2-carboxamid
• 6-[2-(Hydroxymethyl)-1*H*-imidazol-4-yl]-*N-*phenylimidazo[1,2-*a*]pyridin-2-carboxamid
• *N*-(3,5-Difluorphenyl)-5-(6-methylpyridin-2-yl)imidazo[1,2-*a*]pyridin-2-carboxamid
• *N*-(2-Chlorphenyl)-6-(pyridin-2-yl)imidazo[1,2-*a*]-pyridin-2-carboxamid
• *N*-(3,5-Difluorphenyl)-6-(1*H*-pyrrol-3-yl)imidazo-[1,2-*a*]pyridin-2-carboxamid
• *N*-(2-Fluorphenyl)-6-(1*H*-pyrrol-3-yl)imidazo[1,2-*a*]pyridin-2-carboxamid
• *N*-(2,5-Difluorphenyl)-6-(1*H*-pyrrol-3-yl)imidazo-[1,2-*a*]pyridin-2-carboxamid
• *N*-(2,3-Difluorphenyl)-6-(1*H*-pyrrol-3-yl)imidazo-[1,2-*a*]pyridin-2-carboxamid
• *N*-(3,5-Difluorphenyl)-6-(1*H*-pyrazol-3-yl)imidazo-[1,2-*a*]pyridin-2-carboxamid
• *N*-(2-Fluorphenyl)-6-(furan-2-yl)imidazo[1,2-*a*]-pyridin-2-carboxamid
• *N*-(2,5-Difluorphenyl)-6-(furan-2-yl)imidazo[1,2-*a*]pyridin-2-carboxamid
• *N*-(2,3-Difluorphenyl)-6-(furan-2-yl)imidazo[1,2-*a*]pyridin-2-carboxamid
• *N*-(2-chlorphenyl)-6-(furan-2-yl)imidazo[1,2-*a*]-pyridin-2-carboxamid
• *N*-(2,5-Difluorphenyl)-6-(oxazol-2-yl)imidazo[1,2-*a*]pyridin-2-carboxamid
• *N*-(2-Chlorphenyl)-6-(oxazol-2-yl)imidazo[1,2-*a*]-pyridin-2-carboxamid
• *N*-(2-Fluorphenyl)-6-(furan-3-yl)imidazo[1,2-*a*]-pyridin-2-carboxamid
• *N*-(2,5-Difluorphenyl)-6-(furan-3-yl)imidazo[1,2-*a*]pyridin-2-carboxamid
• *N*-(2,3-Difluorphenyl)-6-(furan-3-yl)imidazo[1,2-*a*]pyridin-2-carboxamid
• *N*-(2-Chlorphenyl)-6-(furan-3-yl)imidazo[1,2-*a*]-pyridin-2-carboxamid
• *N*-(2,3-Difluorphenyl)-6-[5-(hydroxymethyl)furan-2-yl]imidazo[1,2-*a*]pyridin-2-carboxamid
• *N*-(2-Fluorphenyl)-5-(1*H*-1,2,4-triazol-3-yl)-imidazo[1,2-*a*]pyridin-2-carboxamid
• *N*-(2-Chlorphenyl)-6-(1*H*-1,2,4-triazol-3-yl)-imidazo[1,2-*a*]pyridin-2-carboxamid
• *N*-(2-Cyanophenyl)-6-(pyridin-2-yl)imidazo[1,2-*a*]-pyridin-2-carboxamid
• 6-(6-Aminopyridin-2-yl)-*N*-(3,5-difluorphenyl)-imidazo[1,2-*a*]pyridin-2-carboxamid und dessen Dihydrochlorid
• 6-(6-Aminopyridin-2-yl)-*N*-(2-fluorphenyl)imidazo-[1,2-*a*]pyridin-2-carboxamid und dessen Dihydro-chlorid
• 6-(6-Aminopyridin-2-yl)-*N*-(2,5-difluorphenyl)-imidazo[1,2-*a*]pyridin-2-carboxamid und dessen Dihydrochlorid
• 6-(6-Aminopyridin-2-yl)-*N*-(2,3-difluorphenyl)-imidazo[1,2-*a*]pyridin-2-carboxamid und dessen Dihydrochlorid
• 6-(6-Aminopyridin-2-yl)-*N*-(2-chlorphenyl)imidazo-[1,2-*a*]pyridin-2-carboxamid und dessen Dihydrochlorid
• *N*-(2-Chlorphenyl)-6-(1*H*-pyrrol-3-yl)imidazo[1,2-*a*]pyridin-2-carboxamid
• *N*-(2-Fluorphenyl)-6-(1*H*-pyrazol-3-yl)imidazo[1,2-*a*]pyridin-2-carboxamid
• *N*-(2-Chlorphenyl)-6-(1*H*-pyrazol-3-yl)imidazo[1,2-*a*]pyridin-2-carboxamid
• *N*-(3,5-Difluorphenyl)-6-(furan-2-yl)imidazo[1,2-*a*]pyridin-2-carboxamid
• *N*-(3,5-Difluorphenyl)-6-(oxazol-2-yl)imidazo[1,2-*a*]pyridin-2-carboxamid
• *N*-(2-Fluorphenyl)-6-(oxazol-2-yl)imidazo[1,2-*a*]-pyridin-2-carboxamid
• *N*-(2,3-Difluorphenyl)-6-(oxazol-2-yl)imidazo[1,2-*a*]pyridin-2-carboxamid
• *N*-(3,5-Difluorphenyl)-6-[5-(hydroxymethyl)furan-2-yl]imidazo[1,2-*a*]pyridin-2-carboxamid
• *N*-(2,5-Difluorphenyl)-6-[5-(hydroxymethyl)furan-2-yl]imidazo[1,2-*a*]pyridin-2-carboxamid
• *N*-(2,3-Difluorphenyl)-6-(1*H*-imidazol-4-yl)imidazo[1,2-*a*]pyridin-2-carboxamid
• *N*-(2,5-Difluorphenyl)-5-(1*H*-1,2,4-triazol-3-yl)imidazo[1,2-*a*]pyridin-2-carboxamid
• *N*-(2,3-Difluorphenyl)-6-(1*H*-1,2,4-triazol-3-yl)imidazo[1,2-*a*]pyridin-2-carboxamid
• *N*-(3,5-Difluorphenyl)-6-(1*H*-1,2,3-triazol-4-yl)imidazo[1,2-*a*]pyridin-2-carboxamid
• *N*-(2-Fluorphenyl)-6-(1*H*-1,2,3-triazol-4-yl)-imidazo[1,2-*a*]pyridin-2-carboxamid
• *N*-(2,5-Difluorphenyl)-6-(1*H*-1,2,3-triazol-4-yl)-imidazo[1,2-*a*]pyridin-2-carboxamid
• *N*-(2-Chlorphenyl)-6-(1*H*-1,2,3-triazol-4-yl)-imidazo[1,2-*a*]pyridin-2-carboxamid;
und deren Säureadditionssalze.

9. Medikament, **dadurch gekennzeichnet, daß** es eine Verbindung der Formel (I) nach einem der Ansprüche 1 bis 8 oder ein Additionssalz dieser Verbindung mit einer pharmazeutisch unbedenklichen Säure enthält.

10. Pharmazeutische Zusammensetzung, **dadurch gekennzeichnet, daß** sie eine Verbindung der Formel (I) nach einem der Ansprüche 1 bis 8 oder ein pharmazeutisch unbedenkliches Salz dieser Verbindung sowie mindestens einen pharmazeutisch unbedenklichen Hilfsstoff enthält.

11. Verwendung einer Verbindung der Formel (I) nach einem der Ansprüche 1 bis 8 zur Herstellung eines Medikaments zur Behandlung und Prävention von neurodegenerativen Erkrankungen.

12. Verwendung einer Verbindung der Formel (I) nach einem der Ansprüche 1 bis 8 zur Herstellung eines Medikaments zur Behandlung und Prävention von Hirntraumen und Epilepsie.

13. Verwendung einer Verbindung der Formel (I) nach einem der Ansprüche 1 bis 8 zur Herstellung eines Medikaments zur Behandlung und Prävention von psychiatrischen Erkrankungen.

14. Verwendung einer Verbindung der Formel (I) nach einem der Ansprüche 1 bis 8 zur Herstellung eines Medikaments zur Behandlung und Prävention von entzündlichen Erkrankungen.

15. Verwendung einer Verbindung der Formel (I) nach einem der Ansprüche 1 bis 8 zur Herstellung eines Medikaments zur Behandlung und Prävention von Osteoporose.

16. Verwendung einer Verbindung der Formel (I) nach einem der Ansprüche 1 bis 8 zur Herstellung eines Medikaments zur Behandlung und Prävention von Krebserkrankungen.

17. Verwendung einer Verbindung der Formel (I) nach einem der Ansprüche 1 bis 8 zur Herstellung eines Medikaments zur Behandlung und Prävention von Parkinson-Krankheit, Alzheimer-Krankheit, Tauopathien und multipler Sklerose.

18. Verwendung einer Verbindung der Formel (I) nach einem der Ansprüche 1 bis 8 zur Herstellung eines Medikaments zur Behandlung und Prävention von Schizophrenie, Depression, Substanzabhängigkeit und Aufmerksamkeitsdefizit-Hyperaktivitätssyndrom.

19. Verbindungen
6-Brom-N-phenylimidazo[1,2-*a*]pyridin-2-carboxamid
6-Iod-N-phenylimidazo[1,2-*a*]pyridin-2-carboxamid
N-Phenyl-6-cyanoimidazo[1,2-*a*]pyridin-2-carboxamid
6-Trimethylstannyl-N-phenylimidazo[1,2-*a*]pyridin-2-carboxamid
N-Phenyl-6-(4,4,5,5-tetramethyl-1,3,2-dioxaborolan-2-yl)imidazo[1,2-*a*]pyridin-2-carboxamid und dessen Hydrobromid (1:1)
2-Phenylcarbamoylimidazo[1,2-*a*]pyridin-6-boronsäure-hydrochlorid (1:1)
N-Phenyl-5-vinylimidazo[1,2-*a*]pyridin-2-carboxamid
5-Formyl-N-phenylimidazo[1,2-*a*]pyridin-2-carboxamid
6-(2-Bromacetyl)-N-phenylimidazo[1,2-*a*]pyridin-2-carboxamid
6-(1-Ethoxyvinyl)-N-phenylimidazo[1,2-*a*]pyridin-2-carboxamid
N-(3,5-Difluorphenyl)-6-iodimidazo[1,2-*a*]pyridin-2-carboxamid
6-(6-{[(1,1-Dimethylethoxy)carbonyl]amino}pyridin-2-yl)imidazo[1,2-*a*]pyridin-2-carbonsäure
6-(6-Aminopyridin-2-yl)imidazo[1,2-*a*]pyridin-2-carbonsäureethylester
6-(6-{[(1,1-Dimethylethoxy)carbonyl]amino}pyridin-2-yl)imidazo[1,2-*a*]pyridin-2-carbonsäureethylester
6-(6-{Bis[(1,1-dimethylethoxy)carbonyl]amino}-pyridin-2-yl)imidazo[1,2-*a*]pyridin-2-carbonsäure-ethylester
6-(Pyridin-2-yl)imidazo[1,2-*a*]pyridin-2-carbonsäure
(6-Pyridin-2-yl)imidazo[1,2-*a*]pyridin-2-carbonsäureethylester
6-(1-Triphenylmethyl-1*H*-imidazol-4-yl)imidazo[1,2-*a*]pyridin-2-carbonsäure
6-(1-Triphenylmethyl-1*H*-imidazol-4-yl)imidazo[1,2-*a*]pyridin-2-carbonsäureethylester
6-(2-{[(1,1-Dimethylethoxy)carbonyl]amino}thiazol-4-yl)imidazo[1,2-*a*]pyridin-2-carbonsäure
6-(2-{[(1,1-Dimethylethoxy)carbonyl]amino}thiazol-4-yl)imidazo[1,2-*a*]pyridin-2-carbonsäureethylester
6-(1*H*-Pyrrol-3-yl)imidazo[1,2-*a*]pyridin-2-carbonsäure und dessen Hydrochlorid (1:1)
6-[1-(Triisopropylsilyl)-1*H*-pyrrol-3-yl]imidazo-[1,2-*a*]pyridin-2-carbonsäureethylester
6-(1*H*-Pyrazol-3-yl)imidazo[1,2-*a*]pyridin-2-carbonsäure
6-(1*H*-Pyrazol-3-yl)imidazo[1,2-*a*]pyridin-2-carbonsäureethylester
6-(1*H*-Pyrazol-4-yl)imidazo[1,2-*a*]pyridin-2-carbon-säure
6-(1*H*-Pyrazol-4-yl)imidazo[1,2-*a*]pyridin-2-carbon-säureethylester
6-(Furan-2-yl)imidazo[2,2-*a*]pyridin-2-carbonsäure
6-(Furan-2-yl)imidazo[1,2-*a*]pyridin-2-carbonsäure-ethylester
6-(Furan-3-yl)imidazo[1,2-*a*]pyridin-2-carbonsäure
5-(Furan-3-yl)imidazo[1,2-*a*]pyridin-2-carbonsäureethylester
6-[5-(Hydroxymethyl)furan-2-yl]imidazo[1,2-*a*]-pyridin-2-carbonsäure
6-(5-Formylfuran-2-yl)imidazo[1,2-*a*]pyridin-2-carbonsäureethylester
6-[5-(Hydroxymethyl)furan-2-yl]imidazo[1,2-*a*]-pyridin-2-carbonsäureethylester
6-(Thiophen-3-yl)imidazo[1,2-*a*]pyridin-2-carbonsäure
5-(Thiophen-3-yl)imidazo[1,2-*a*]pyridin-2-carbonsäureethylester
5-(Oxazol-2-yl)imidazo[1,2-*a*]pyridin-2-carbonsäure
6-(Oxazol-2-yl)imidazo[1,2-*a*]pyridin-2-carbonsäureethylester
6-(1*H*-1,2,4-Triazol-3-yl)imidazo[1,2-*a*]pyridin-2-carbonsäure
6-[Ethoxy(imino)methyl]imidazo[1,2-*a*]pyridin-2-carbonsäureethylester
6-[Hydrazino(imino)methyl]imidazo[1,2-*a*]pyridin-2-carbonsäureethylester
6-(1*H*-1,2,4-Triazol-3-yl)imidazo[1,2-*a*]pyridin-2-carbonsäureethylester
6-(1*H*-1,2,3-Triazol-4-yl)imidazo[1,2-*a*]pyridin-2-carbonsäure
6-(1*H*-1,2,3-Triazol-4-yl)imidazo[1,2-*a*]pyridin-2-carbonsäureethylester
6-(1*H*-1,2,3-Triazol-4-yl)imidazo[1,2-*a*]pyridin-2-carbonsäure
6-(4,4,5,5-Tetramethyl-1,3,2-dioxaborola-n-2-yl)-imidazo[1,2-*a*]pyridin-2-carbonsäureethylesterhydrobromid (1:1)
2-Ethoxycarbonylimidazo[1,2-a]pyridin-6-boronsäure:

20. Verwendung der Verbindungen nach Anspruch 19 zur Herstellung von Verbindungen der allgemeinen Formel (I) gemäß einem der Ansprüche 1 bis 8.

## Claims

1. Compounds of formula (I): 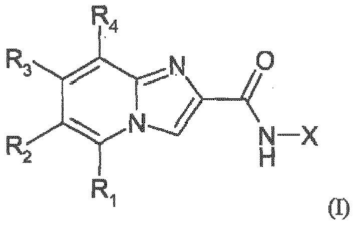 in which:
X represents a phenyl group optionally substituted with one or more groups chosen, independently of each other, from the following atoms or groups: halogen, (C₁-C₆)alkoxy, (C₁-C₆)alkyl, NRaRb, cyano, (C₁-C₆)alkoxycarbonyl, the groups (C₁-C₆)alkyl and (C₁-C₆)alkoxy being optionally substituted with one or more halogen atoms;
R₁ represents a hydrogen atom, a halogen, a group (C₁-C₆)alkoxy, a group (C₁-C₆)alkyl or a group NRaRb; the alkyl and alkoxy groups possibly being substituted with one or more halogen, hydroxyl, amino, or a group (C₁-C₆)alkoxy;
R₂ represents an unsaturated, partially saturated or totally saturated aromatic heterocyclic group, optionally substituted with one or more groups chosen, independently of each other, from the following atoms or groups: hydroxyl, (C₁-C₆)alkoxy, halogen, cyano, NRaRb, -CO-R₅, -CO-NR₆R₇, -CO-O-R₈, -NR₉-CO-R₁₀ (C₃-C₇)cycloalkyl(C₁-C₆)alkyl, (C₃-C₇)cycloalkyl(C₁-C₆)alkoxy, a group (C₁-C₆)alkyl, optionally substituted with one or more hydroxyl or NRaRb, an oxido group;
R₃ represents a hydrogen atom, a group (C₁-C₆)alkyl, a group (C₁-C₆)alkoxy or a halogen atom;
R₄ represents a hydrogen atom, a group (C₁-C₄)alkyl, a group (C₁-C₄)alkoxy or a fluorine atom;
R₅ represents a hydrogen atom, a phenyl group or a group (C₁-C₆)alkyl;
R₆ and R₇, which may be identical or different, represent a hydrogen atom or a group (C₁-C₆)alkyl or form, with the nitrogen atom, a 4- to 7-membered ring optionally including another heteroatom chosen from N, O and S;
R₈ represents a group (C₁-C₆)alkyl;
R₉ and R₁₀, which may be identical or different, represent a hydrogen atom or a group (C₁-C₆)alkyl,
Ra and Rb are, independently of each other, hydrogen or (C₁-C₆)alkyl or form, with the nitrogen atom that bears them, a 4- to 7-membered ring, optionally including another heteroatom chosen from N, O and S;
a heterocyclic group represents an unsaturated, partially saturated or totally saturated monocyclic aromatic group comprising from 5 to 10 atoms including 1 to 4 heteroatoms chosen from N, O and S;
with the exception of *N-*(4-bromophenyl)-6-(1-methyl-2-piperidinyl)imidazo[1,2-*a*]pyridine-2-carboxamide;
in the form of the base or of an acid-addition salt.

2. Compounds of formula (I) according to Claim 1, **characterized in that**:
X represents a phenyl group optionally substituted with one or more halogen atoms or cyano groups;
in the form of the base or of an acid-addition salt.

3. Compounds of formula (I) according to Claim 1 or 2, **characterized in that**:
R₂ represents a pyrrole, furan, thiophene, pyrazole, imidazole, triazole, tetrazole, oxazole, isoxazole, oxadiazole, thiazole, isothiazole, thiadiazole, pyridine, pyrimidine, pyrazine, pyridazine or triazine group, optionally substituted with one or more groups chosen from the following atoms or groups: hydroxyl, (C₁-C₆)alkoxy, oxido, halogen, -NRaRb, (C₁-C₆)alkyl which is itself optionally substituted with a hydroxyl group,
Ra and Rb are, independently of each other, hydrogen or (C₁-C₆)alkyl;
in the form of the base or of an acid-addition salt.

4. Compounds of formula (I) according to Claim 1 or 2, **characterized in that**:
R₂ represents a dioxolane, pyridine, imidazole, pyrazole, triazole, pyrrole, furan, oxazole, indole, imidazoline, thiophene, pyrazine, pyrimidine or thiazole group optionally substituted with one or more groups chosen from the following atoms or groups: hydroxyl, (C₁-C₆)alkoxy, oxido, halogen, -NRaRb, (C₁-C₆)alkyl which is itself optionally substituted with a hydroxyl group,
Ra and Rb are, independently of each other, hydrogen or (C₁-C₆)alkyl,
in the form of the base or of an acid-addition salt.

5. Compounds of formula (I) according to any one of the preceding claims, **characterized in that** R₁ represents a hydrogen atom or a group (C₁-C₆)alkyl;
in the form of the base or of an acid-addition salt.

6. Compounds of formula (I) according to any one of the preceding claims, **characterized in that** R₃ and R₄ represent a hydrogen atom;
in the form of the base or of an acid-addition salt.

7. Compounds of formula (I) according to any one of the preceding claims, **characterized in that**:
X represents a phenyl group optionally substituted with one or more fluorine or chlorine atoms or with a cyano group;
R₂ represents a dioxolane, pyridine, imidazole, pyrazole, triazole, pyrrole,
furan, oxazole, indole, imidazoline, thiophene, pyrazine, pyrimidine or thiazole group, optionally substituted with one or more hydroxyl, methyl, hydroxymethyl, methoxy, halogen, NH₂, (C₁-C₆)alkyl or oxido groups,
R₁ represents a hydrogen atom or a methyl group;
R₃ and R₄ represent a hydrogen atom;
in the form of the base or of an acid-addition salt.

8. Compounds of formula (I} according to any one of the preceding claims, **characterized in that** they are chosen from:
• 6-(1,3-Dioxolan-2-yl)-*N*-phenylimidazo[1,2-*a*]pyridine-2-carboxamide
• *N*-Phenyl-6-(pyrid-3-yl)imidazo[1,2-*a*]pyridine-2-carboxamide
• *N*-Phenyl-6-(pyrid-2-yl)imidazo[1,2-*a*]pyridine-2-carboxamide
• 6-[5-(Hydroxymethyl)pyrid-3-yl]-*N*-phenylimidazo[1,2-*a*]pyridine-2-carboxamide
• 6-[4-(Hydroxymethyl)pyrid-2-yl]-*N*-phenylimidazo[1,2-*a*]pyridine-2-carboxamide and the hydrochloride (1:1) thereof
• 6-(6-Aminopyrid-2-yl)-*N*-phenylimidazo[1,2-*a*]pyridine-2-carboxamide and the hydrochloride (1:1) thereof
• 6-(1*H*-Imidazol-4-yl)-*N*-phenylimidazo[1,2-*a*]pyridine-2-carbaxamide and the hydrochloride (1:1) thereof
• *N*-Phenyl-6-(1*H*-pyrazol-3-yl)imidazo[1,2-*a*]pyridine-2-carboxamide
• *N*-Phenyl-6-(1*H*-1,2,4-triazol-3-yl)imidazo[1,2-*a*]pyridine-2-carboxamide
• *N*-Phenyl-6-(1*H*-pyrrol-3-yl)imidazo[1,2-*a*]pyridine-2-carboxamide
• 6-(2-Methyl-1*H*-imidazol-4-yl)-*N*-phenylimidazo[1,2-*a*]pyridine-2-carboxamide
• 6-(3-Furyl)-*N*-phenylimidazo[1,2-*a*]pyridine-2-carboxamide
• 6-(1*H*-Imidazol-1-yl)-*N*-phenylimidazo[1,2-*a*]pyridine-2-carboxamide and the trifluoroacetate (1:1) thereof
• 6-(Oxazol-5-yl)-*N*-phenylimidazo[1,2-*a*]pyridine-2-carboxamide
• 6-(2-Aminothiazol-4-yl)-*N*-phenylimidazo[1,2-*a*]pyridine-2-carboxamide
• 6-(2-Methyl-1,3-dioxolan-2-yl)-*N*-phenylimidazo[1,2-*a*]pyridine-2-carboxamide
• 6-(4,5-Dihydro-1*H*-imidazol-2-yl)-*N*-phenylimidazo[1,2-*a*]pyridine-2-carboxamide and the hydrochloride (1:1) thereof
• 6-(6-Methoxypyrid-2-yl)-*N*-phenylimidazo[1,2-*a*]pyridine-2-carboxamide
• 5-Methyl-*N*-phenyl-6-(pyrid-2-yl)imidazo[1,2-*a*]pyridine-2-carboxamide
• 6-(2-Amino-1*H*-imidazol-4-yl)-*N*-phenylimidazo[1,2-*a*]pyridine-2-carboxamide and the trifluoroacetate (1:1) thereof
• 6-(2-Aminothiazol-5-yl)-*N*-phenylimidazo[1,2-*a*]pyridine-2-carboxamide
• 6-(6-Hydroxypyrid-2-yl)-*N*-phenylimidazo[1,2-*a*]pyridine-2-carboxamide
• *N*-Phenyl-6-(1*H*-1,2,3-triazol-4-yl)imidazo[1,2-*a*]pyridine-2-carboxamide
• *N*-(3,5-Difluorophenyl)-6-(furan-3-yl)imidazo[1,2-*a*]pyridine-2-carboxamide
• *N*-(3-Fluorophenyl)-6-(furan-3-yl)imidazo[1,2-*a*]pyridine-2-carboxamide
• 6-(2-Furyl)-*N*-phenylimidzo[1,2-*a*]pyridine-2-carboxamide
• 6-[6-Hydroxymethyl)-pyrid-2-yl]-*N*-phenylimidazo[1,2-*a*]pyridine-2-carboxamide and the hydrochloride (1:1) thereof
• 6-(1-Oxidopyrid-3-yl)-*N*-phenylimidazo[1,2-*a*]pyridine-2-carboxamide and the hydrochloride (1:1) thereof
• *N*-Phenyl-6-(1*H*-pyrazol-4-yl)imidazo[1,2-*a*]pyridine-2-carboxamide
• 6-(1*H*-Imidazol-2-yl)-*N*-phenylimidazo[1,2-*a*]pyridine-2-carboxamide hydrochloride (1:1)
• 6-(1-Methyl-1*H*-imidazol-4-yl)-*N*-phenylimidazo[1,2-*a*]pyridine-2-carboxamide
• 6-(Oxazol-2-yl)-*N*-phenyliddazo[1,2-*a*]pyridine-2-carboxamde
• *N*-(3,5-Difluorophenyl)-6-(pyrid-2-yl)imidazo[1,2-*a*]pyridine-2-carboxamide
• *N*-Phenyl-6-(pyrid-4-yl)imidazo[1,2-*a*]pyridine-2-carboxamide
• 6-(1*H*-Indol-3-yl)-*N*-phenylimidazo[1,2-*a*]pyridine-2-carboxamide
• *N*-Phenyl-6-(thiophen-2-yl)imidazo[1,2-*a*]pyridine-2-carboxamide
• *N*-Phenyl-6-(pyrazin-2-yl)imidazo[1,2-*a*]pyridine-2-carboxamide
• 6-(1-Oxidopyrid-2-yl)-*N*-phenylimidazo[1,2-*a*]pyridine-2-carboxamde
• *N*-Phenyl-6-(pyrimidin-6-yl)imidazo[1,2-*a*]pyridine-2-carboxamide
• *N*-Phenyl-6-(thien-3-yl)imidazo[1,2-*a*]pyridine-2-carboxamide
• 6-(5-Fluoro-2-furyl)-*N*-phenylimidazo[1,2-*c*]pyridine-2-carboxamide
• *N*-(3-Fluorophenyl)-6-(pyrid-2-yl)imidazo[1,2-*a*]pyridine-2-carboxamide
• *N*-(2-Fluorophenyl)-6-(pyrid-2-yl)imidazo[1,2-*a*]pyridine-2-carboxamde
• *N*-(2,5-Difluorophenyl)-6-(pyrid-2-yl)imidazo[1,2-*a*]pyridine-2-carboxamide
• *N*-(2,3-Difluorophenyl)-6-(pyrid-2-yl)imidazo[1,2-*a*]pyridine-2-carboxamide
• 6-(6-Aminopyrid-2-yl)-*N*-(3-fluorophenyl)imidazo[1,2-*a*]pyridine-2-carboxamide
• *N*-(3-Fluorophenyl)-6-(1*H*-pyrrol-3-yl)imidazo[1,2-*a*]pyridine-2-carboxamide
• *N*-(3-Fluorophenyl)-6-(1*H* pyrazol-3-yl)imidazo[1,2-*a*]pyridine-2-carboxamide
• *N*-(3-Fluorophenyl)-6-(furan-2-yl)imidazo[1,2-*a*]pyridine-2-carboxamide
• *N*-(3-Fluorophenyl)-6-(oxazol-2-yl)imidazo[1,2-*a*]pyridine-2-carboxamide
• *N*-Phenyl-6-(pyrimidin-2-yl)imidazo[1,2-*a*]pyridine-2-carboxamide
• *N*-(3-Fluorophenyl)-6-(1*H*-imidazol-4-yl)imidazo[1,2-*a*]pyridine-2-carboxamide
• *N-*(3-Fluorophenyl)-6-(1*H*-1,2,4-triazol-3-yl)imidazo[1,2-*a*]pyridine-2-carboxamide
• 6-(2-Aminothiazol-4-yl)-*N*-(3-fluorophenyl)imidazo[1,2-*a*]pyridine-2-carboxamide
• *N*-(3-Fluorophenyl)-6-(1*H*-1,2,3-triazol-4-yl)imidazo[1,2-*a*]pyridine-2-carboxamide
• *N*-(3-Fluorophenyl)-6-(1*H*-pyrazol-4-yl)imidazo[1,2-*a*]pyridine-2-carboxamide
• *N*-(3-Fluorophenyl)-6-[5-(hydroxymethyl)furan-2-yl]imidazo[1,2-*a*]pyridine-2-carboxamide
• 6-[2-(Hydroxymethyl)-1*H*-imidazol-4-yl]-*N*-phenylimidazo[1,2-*a*]pyridine-2-carboxamide
• *N*-(3,5-Difluorophenyl)-6-(6-methylpyrid-2-yl)imidazo[1,2-*a*]pyridine-2-carboxamide
• *N*-(2-Chlorophenyl)-6-(pyrid-2-yl)imidazo[1,2-*a*]pyridine-2-carboxamide
• *N*-(3,5-Difluorophenyl)-6-(1*H-*pyrrol-3-yl)imidazo[1,2-*a*]pyridine-2-carboxamide
• *N*-(2-Fluorophenyl)-6-(1*H*-pyrrol-3-yl)imidazo[1,2-*a*]pyridine-2-carboxamide
• *N*-(2,5-Difluorophenyl)-6-(1*H*-pyrrol-3-yl)imidazo[1,2-*a*]pyridine-2-carboxamide
• *N*-(2,3-Difluorophenyl)-6(1*H*-pyrrol-3-yl)imidazo[1,2-*a*]pyridine-2-carboxamide
• *N*-(3,5-Difluorophenyl)-6-(1*H*-pyrazol-3-yl)imidazo[1,2-*a*]pyridine-2-carboxamide
• *N*-(2-Fluorophenyl)-6-(furan-2-yl)imidazo[1,2-*a*]pyridine-2-carboxamide
• *N*-(2,5-Difluorophenyl)-6-(furan-2-yl)imidazo[1,2-*a*]pyridine-2-carboxamide
• *N*₋(2,3-Difluorophenyl)-6-(furan-2-yl)imidazo[1,2-*a*]pyridine.2-carboxamide
• *N*-(2-Chlorophenyl)-6-(furan-2-yl)imidazo[1,2-*a*]pyridine-2-carboxamide
• *N*-(2,5-Difluorophenyl)-6-(oxazol-2-yl)imidazo[1,2-*a*]pyridine-2-carboxamide
• *N*-(2-Chlorophenyl)-6-(oxazol-2-yl)imidazo[1,2-*a*]pyridine-2-carboxamide
• *N*-(2-Fluorophenyl)-6-(furan-3-yl)imidazo[1,2-*a*]pyridine-2-carboxamide
• *N*-(2,5-Difluorophenyl)-6-(furan-3-yl)imidazo[1,2-*a*]pyridine-2-carboxamide
• *N*-(2,3-Difluorophenyl)-6-(furan-3-yl)imidazo[1,2-*a*]pyridine-2-carboxamide
• *N*-(2-Chlorophenyl)-6-furan-3-yl)imidazo[1,2-*a*]pyridine-2-carboxamide
• *N*-(2,3-Difluorophenyl)-6-[5-(hydroxymethyl)furan-2-yl]imidazo[1,2-*a*]pyridine-2-carboxamide
• *N*-(2-Fluorophenyl)-6-(1*H*-1,2-,4-triazol-3-yl)imidazo[1,2-*a*]pyridine-2-carboxamide
• *N*-(2-Chlorophenyl)-6-(1*H*-1,2,4-triazol-3-yl)imidazo[1,2-*a*]pyridine-2-carboxamide
• *N*-(2-Cyanophenyl)-6-(pyrid-2-yl)imidazo[1,2-*a*]pyridine-2-carboxamide
• 6-(6-Aminopyrid-2-yl)-*N*-(3,5-difluorophenyl)imidazo[1,2-*a*]pyridine-2-carboxamide and the dihydrochloride thereof
• 6-(6-Aminopyrid-2-yl)-*N*-(2-fluorophenyl)imidazo[1,2-*a*]pyridine-2-carboxamide and the dihydrochloride thereof
• 6-(6-Aminopyrid-2-yl)-*N*-(2,5-difluorophenyl)imidazo[1,2-*a*]pyridine-2-carboxamide and the dihydrochloride thereof
• 6-(6-Aminopyrid-2-yl)-*N*-(2,3-difluorophen.yl)imidazo[1,2,*a*]pyridine-2-carboxamide and the dihydrochloride thereof
• 6-(6-Aminopyrid-2-yl)-*N*-(2-chlorophenyl)imidazo[1,2-*a*]pyridine-2-carboxamide and the dihydrochloride thereof
• *N*-(2-Chlorophenyl)-6-(1*H*-pyrrol-3-yl)imidazo[1,2-*a*]pyridine-2-carboxamide
• *N*-{2-Fluorophenyl)-6-(1*H*-pyrazol-3-yl)imidazo[1,2-*a*]pyridine-2-carboxamide
• *N*-(2-Chlorophenyl)-6-(1*H*-pyrazol-3-yl)imidazo[1,2-*a*]pyridine-2-carboxamide
• *N*-(3,5-Difluorophenyl)-6-(furan-2-yl)imidazo[1,2-*a*]pyridine-2-carboxamide
• *N*-(3,5-Difluorophenyl)-6-(oxazol-2-yl)imidazo[1,2-*a*]pyridine-2-carboxamide
• *N*-(2-Fluorophenyl)-6-(oxazol-2-yl)imidazo[1,2-*a*]pyridine-2-carboxamide
• *N-*(2,3-Difluorophenyl)-6-(oxazol-2-yl)imidazo[1,2-*a*]pyridine-2-carboxamide
• *N*-(3,5-Difluorophenyl)-6-[5-(hydroxymethyl)furan-2-yl]imidazo[1,2-*a*]pyridine-2-carboxamide
• *N*-(2,5-Difluorophenyl)-6-[5-(hydroxymethyl)furan-2-yl]imidazo[1,2-*a*]pyridine-2-carboxamide
• *N*-(2,3-Difluorophenyl)-6-(1*H*-imidazol-4-yl)iddazo[1,2-*a*]pyridine-2-carboxamide
• *N*-(2,5-Difluorophenyl)-6-(1*H*-1,2,4-triazol-3-yl)imidazo[1,2-*a*]pyridine-2-carboxamide
• *N*-(2,3-Difluorophenyl)-6-(1*H*-1,2,4-triazol-3-yl)imidazo[1,2-*a*]pyridine-2-carboxamide
• *N*-(3,5-Difluorophenyl)-6-(1*H*-1,2,3-triazol-4-yl)imidazo[1,2-*a*]pyridine-2-carboxamide
• *N*-(2- Fluorophenyl)-6-(1*H*-1,2,3-triazol-4-yl)pyridine[1,2-*a*]pyridine-2-carboxamide
• *N*-(2,5-Difluorophenyl)-6-(1*H*-1,2,3-triazol-4-yl)imidazo[1,2-*a*]pyridine-2-carboxamide
• *N*-(2-Chlorophenyl)-6-(1*H*-1,2,3-triazol-4-yl)imidazo[1,2-*a*]pyridine-2-carboxamide;
and the acid-addition salts thereof.

9. Medicament, **characterized in that** it comprises a compound of formula (I) according to any one of Claims 1 to 8, or an addition salt of this compound with a pharmaceutically acceptable acid.

10. Pharmaceutical composition, **characterized in that** it comprises a compound of formula (I) according to any one of Claims 1 to 8, or a pharmaceutically acceptable salt of this compound, and also at least one pharmaceutically acceptable excipient.

11. Use of a compound of formula (I) according to any one of Claims 1 to 8, for the preparation of a medicament for treating or preventing neurodegenerative diseases.

12. Use of a compound of formula (I) according to any one of Claims 1 to 8, for the preparation of a medicament for treating or preventing cerebral trauma and epilepsy.

13. Use of a compound of formula (I) according to any one of Claims 1 to 8, for the preparation of a medicament for treating or preventing psychiatric diseases.

14. Use of a compound of formula (I) according to any one of Claims 1 to 8, for the preparation of a medicament for treating or preventing inflammatory diseases.

15. Use of a compound of formula (I) according to any one of Claims 1 to 8, for the preparation of a medicament for treating or preventing osteoporosis.

16. Use of a compound of formula (I) according to any one of Claims 1 to 8, for the preparation of a medicament for treating or preventing cancers.

17. Use of a compound of formula (I) according to any one of Claims 1 to 8, for the preparation of a medicament for treating or preventing Parkinson's disease, Alzheimer's disease, tauopathies and multiple sclerosis.

18. Use of a compound of formula (I) according to any one of Claims 1 to 8, for the preparation of a medicament for treating or preventing schizophrenia, depression, substance dependency and attention-deficit hyperactivity disorder.

19. Compounds:
6-Bromo-N-phenylimidazo[1,2-*a*]pyridine-2-carboxamide
6-Iodo-N-phenylimidazo[1,2,*a*]pyridine-2-carboxamide
N-Phenyl-6-cyanoimidazo[1,2-*a*]pyridine-2-carboxamide
6-Trimethylstanny-N-phenylimidazo[1,2-*a*]pyridine-2-carboxamide
N-Phenyl-6-(4,4,5,5-tetramethyl-1,3,2-dioxaborolan-2-yl)imidazo[1,2-*a*]pyridine-2-carboxamide and the hydrobromide (1:1) thereof
2-Phenylcarbamoylimidazo[1,2-*a*]pyridine-6-boromc acid hydrochloride (1:1)
N-Phenyl-6-vinylimidazo[1,2-*a*]pyridine-2-carboxamide
6-Formyl-N-phenylimidazo[1,2-*a*]pyridine-2-carboxamide
6-(2-Bromoacetyl)-N-phenylimidazo[1,2-*a*]pyridine-2-carboxamide
6-(1-Ethoxyvinyl)-N-phenylimidazo[1,2-*a*]pyridine-2-carboxamide
N-(3,5-Difluorophenyl)-6-iodoimidazo[1,2-*a*]pyridine-2-carboxamide
6-(6-{[(1,1-Dimethylethoxy)carbonyl]amino}pyrid-2-yl)imidazo[1,2-*a*]pyridine-2-carboxylic acid
Ethyl 6-(6-aminopyrid-2-yl)imidazo[1,2-*a*]pyridine-2-carboxylate
Ethyl 6-(6-{[(1,1-dimethylethoxy)carbonyl]amino}pyrid-2-yl)imidazo[1,2-*a*]pyridine-2-carboxylate
Ethyl 6-{6-{bis[(1,1-dimethylethoxy)carbonyl]amino}pyrid-2-yl)imidazo[1,2-*a*]pyridine-2-carboxylate
6-(Pyrid-2-yl)imidazo[1,2-*a*]pyridine-2-carboxylic acid
Ethyl (6-pyrid-2-yl)imidazo[1,2-*a*]pyridine-2-carboxylate
6-(1-Triphenylmethyl-1*H*-imidazol-4-yl)imidazo[1,2-*a*]pyridine-2-carboxylic acid
Ethyl 6-(1-triphenylmethyl-1*H*-imidazol-4-yl)imidazo[1,2-*a*]pyridine-2-carboxylate
6-(2-{[(1,1-Dimethylexhoxy)carbanyl]amino}thiazol-4-yl)imidazo[1,2-*a*]pyridine-2-carboxylic acid
Ethyl 6-(2-{[(1,1-d'imethylethoxy)carbonyl]amino}thiazol-4-yl)imidazo[1,2-*a*]pyridine-2-carboxylate
6-(1*H*-Pyrrol-3-yl)imidazo[1,2-*a*]pyridine-2-carboxylic acid and the hydrochloride (1:1) thereof
Ethyl 6-[1-(triisopropylsilyl)-1*H*-pyrrol-3-yl]imidazo[1,2-*a*]pyridine-2-carboxylate
6-(1*H*-Pyrazol-3-yl)imidazo[1,2-*a*]pyridine-2-carboxylic acid
Ethyl 6-(1*H*-pyrazol-3-yl)imidazo[1,2-*a*]pyridine-2-carboxylate
6-(1*H*-Pyrazol-4-yl)imidazo[1,2-*a*]pyridine-2-carboxylic acid
Ethyl 6-(1*H*-pyrazol-4-yl)imidazo[1,2-*a*]pyridine-2-carboxylate
6-(furan-2-yl)imidazo[1,2-*a*]pyridine-2-carboxylic acid
Ethyl 6-(furan-2-yl)imidazo[1,2-*a*]pyridine-2-carboxylate
6-(Furan-3-yl)imidazo[1,2-*a*]pyridine-2-carboxylic acid
Ethyl 6-(furan-3-yl)imidazo[1,2-*a*]pyridine-2-carboxylate
6-[5-(Hydroxymethyl)furan-2-yl]imdazo[1,2-*a*]pyridine-2-carboxylic acid
Ethyl 6-(5-formylfuran-2-yl)imidazo[1,2-*a*]pyridine-2-carboxylate
Ethyl 6-[5-(hydroxymethyl)furan-2-yl]imidazo[1,2-*a*]pyridine-2-carboxylate
6-(Thiophen-3-yl)imidazo[1,2-*a*]pyridine-2-carboxylic acid
Ethyl 6-(thiophen-3-yl)imidazo[1,2-*a*]pyridine-2-carboxylate
6-(Oxazol-2-yl)imidazo[1,2-*a*]pyridine-2-carboxylic acid
Ethyl 6-(oxazol-2-yl)imidazo[1,2-*a*]pyridine-2-carboxylate
6-(1*H*-1,2,4-Triazol-3-yl)imidazo[1,2-*a*]pyridine-2-carboxylic acid
Ethyl 6-[ethoxy(imino)methyl]imidazo[1,2-*a*]pyridine-2-carboxylate
Ethyl 6-[hydrazino(imino)methyl]imidazo[1,2-*a*]pyridine-2-carboxylate
Ethyl 6-(1*H*-1,2,4-triazol-3-yl)imidazo[1,2-*a*]pyridine-2-carboxylate
6-(1H-1,2,3-Triazol-4-yl)imidazo[1,2-*a*]pyridine-2-carboxylic acid
Ethyl 6-(1*H*-1,2,3-triazol-4-yl)imidaza[1,2-*a*]pyridine-2-carboxylate
6-(1*H*-1,2,3-Triazol-4-yl)imidazo[1,2-*a*]pyridine-2-carboxylic acid
Ethyl 6-(4,4,5,5-tetramethyl-1,3,2-dioxaboxolan-2-yl]imidazo[1,2-*a*]pyridine-2-carboxylate hydrobromide (1:1)
2-Ethoxycarbonylimidazo[1,2-*a*]pyridine-6-boronic acid.

20. Use of the compounds according to Claim 19, for the preparation of compounds of general formula (I) as defined in any one of Claims 1 to 8.
